# EUROPEAN PATENT APPLICATION

(11) **EP 4 664 196 A2**
(43) Date of publication of application: **17.12.2025**
(21) Application number: 25181938.9
(22) Date of filing: 11.06.2025
(51) Int. Cl.: G03F 7/004, G03F 7/038, G03F 7/09

(54) **BENZENESULFONIC ACID SALT COMPOUND FOR FORMING ORGANIC FILM, COMPOSITION FOR FORMING ORGANIC FILM, METHOD FOR FORMING ORGANIC FILM, AND PATTERNING PROCESS**

(30) Priority: 13.06.2024 JP 2024095959
(71) Applicant: SHIN-ETSU CHEMICAL CO., LTD., Tokyo 1000005 (JP)
(72) Inventor: Sawamura, Takashi, Niigata (JP); Mitsui, Ryo, Niigata (JP)
(74) Representative: Schicker, Silvia

(57) **Abstract**

The present invention is a composition for forming an organic film, containing: a resin and/or compound (A) for forming an organic film; a benzenesulfonic acid salt compound (B) represented by the following formula (1), an anion moiety in the formula (1) having a molecular weight of 200 or more, and the compound (B) not containing a perfluoroalkyl group; and a solvent (C), where R₁ represents a linear, cyclic, or branched, alkyl group, alkenyl group, oxoalkyl group, aryl group, or aralkyl group having 1 to 20 carbon atoms and optionally having a substituent not containing a perfluoroalkyl group, represents -P(R)₂, R being an alkyl group or aryl group having 1 to 20 carbon atoms, or represents a halogen atom or a nitro group, "n" represents an integer of 1 to 5, and A⁺ represents an ammonium cation, a pyridinium cation, a sulfonium cation, a phosphonium cation, an imidazolium cation, a piperidinium cation, or a pyrrolidinium cation. This can provide: a composition for forming an organic film that makes it possible to form a film excellent in coating properties, such as coating defects, film-formability, and filling property results, when used as an organic underlayer film for a multilayer resist, that is also useful as a photoresist material, and that contains a benzenesulfonic acid salt that is not a perfluoroalkyl compound (PFAS); a patterning process using the composition; and a compound suitable for the composition.

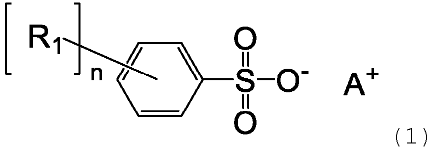

## Description

### TECHNICAL FIELD

The present invention relates to: a benzenesulfonic acid salt compound for forming an organic film; a composition for forming an organic film; a method for forming an organic film; and a patterning process.

### BACKGROUND ART

As LSIs advance toward higher integration and higher processing speed, miniaturization of pattern rule is progressing rapidly. This is because the spread of high-speed communication of 5 G and artificial intelligence (AI) has progressed, and high-performance devices for processing these are needed. As a cutting-edge technology for miniaturization, 5-nm node devices have been mass-produced by extreme ultraviolet ray (EUV) lithography at a wavelength of 13.5 nm. Furthermore, studies are also in progress on employing EUV lithography in next-generation 3-nm node and the following-generation 2-nm node devices.

In a monolayer resist method, which is employed as a typical resist patterning process, as the thinning of resist patterns progresses as described above, pattern formation becomes difficult, and it is known that, as a fine pattern processing method, a multilayer resist method, in which a pattern is formed by laminating films having different dry etching properties for forming a pattern with a high aspect ratio on an uneven substrate, is excellent. There has been developed and put to practical use, a three-layer resist method in which a photoresist layer made of an organic photosensitive polymer used in a monolayer resist method is combined with a middle layer made of a silicon-based polymer or a silicon-based CVD film, and an underlayer made of an organic polymer (Patent Document 1).

In the three-layer resist method, for example, an organic film made of a novolak or the like is formed uniformly as a resist underlayer film on a substrate to be processed; a silicon-containing film is formed thereon as a resist middle layer film; and an ordinary organic photoresist film is formed thereon as a resist upper layer film. In dry etching using a fluorine-based gas plasma, an organic resist upper layer film has favorable etching selectivity to a silicon-containing resist middle layer film, and therefore, a resist pattern is transferred to the silicon-containing resist middle layer film by dry etching using a fluorine-based gas plasma. According to this method, the pattern can be transferred to the silicon-containing film even when using a resist composition that causes difficulties in forming a pattern having a sufficient film thickness for directly processing the substrate to be processed or when using a resist composition that does not have sufficient dry etching resistance for processing the substrate. In addition, by subsequently transferring the pattern by dry etching with an oxygen-based gas plasma, it is possible to obtain a pattern in a novolak film having sufficient dry etching resistance for processing.

Many techniques are already known (e.g. Patent Document 2) regarding organic underlayer films like the organic underlayer film described above. However, in association with recent progress in miniaturization, the need for excellent filling property in addition to dry etching property is rising. There is a demand for an organic underlayer film material that enables uniform film formation even on an underlying substrate to be processed having a complex form or any material, and that has a filling property that allows a required pattern to be filled without gaps.

When a semiconductor substrate or the like is manufactured, the above-described organic underlayer film is formed using a coater/developer that can perform treatments such as spin-coating process, EBR process, and baking process. An EBR (Edge Bead Removal) process is a process of removing, after forming a film on a substrate (wafer) by spin-coating, the film on the edge of the substrate with a remover for the purpose of preventing the contamination of a substrate-conveying arm of the coater/developer. Examples of a remover used in EBR processes include a mixed solution of propylene glycol monomethyl ether acetate and propylene glycol monomethyl ether (30 mass%:70 mass%), and such removers are widely used in EBR processes of resist films, resist middle layer films (silicon-containing middle layer films and organic underlayer films).

Due to the effect of a remover in an EBR process, a state where a peripheral portion of an organic underlayer film has a thick film thickness (a hump) occurs in some cases. In the above-described dry etching step at the time of substrate processing, a hump causes defects, and therefore, an organic underlayer film in which a hump is suppressed is desired.

As well as organic underlayer films, a resist material used in photolithography using the organic photosensitive polymer described above is applied by a method such as spin-coating with a solution in the same manner as the organic underlayer film, and the solvent is evaporated by baking to form a film. In the same manner as the organic underlayer film, the film thickness after baking is required to be uniform and flat, and demands for the uniformity and flatness are becoming stricter year by year.

In recent years, there are demands for thick resist films for use in 3D-NAND memory, and even higher flatness is required. As the film thickness becomes thicker, it becomes more difficult to achieve flatness within the film. Meanwhile, along with progress in miniaturization, the thinning of films is progressing, and in this case, the risk of pinhole defects and so forth occurring is increasing.

Examples of film materials using organic matter used as semiconductor-processing materials are described above, but obtaining a material that forms a film with in-plane uniformity of film thickness and without pinholes is also industrially greatly advantageous in film-formation materials that do not use organic matter.

Here, in recent years, the effect of perfluoroalkyl compounds (PFAS) on health has been pointed out, and there is a movement in the European REACH to restrict the production and sale of PFAS compounds. There are many uses for perfluoroalkyl compounds, and because of properties, such as water and oil repellency, resistance to heat, resistance to chemicals, and not absorbing light, originating from the structure, perfluoroalkyl compounds are used for a wide range of uses, for example, water repellents, surface treatment agents, emulsifiers, fire extinguishers, and coating agents. Therefore, a demand for the development of an alternative material not having a PFAS structure has become urgent.

As examples of materials containing a perfluoroalkyl compound described above, thermal acid generators and photo-acid generators containing a perfluoroalkyl sulfonic acid have high acidity, and are widely used for resist materials, underlayer films, and antireflective films (Patent Documents 3 and 4).

From the viewpoint of stricter restriction in the future, it is necessary to use materials that do not apply to the PFAS restriction. For example, as the above-described thermal acid generators, thermal acid generators having 5-sulfosalicylic acid or p-phenolsulfonic acid and usage thereof are proposed (Patent Document 4).

### CITATION LIST

### PATENT LITERATURE

Patent Document 1: JP 4355943 B2
Patent Document 2: JP 2004-205685 A
Patent Document 3: JP 2018-173521 A
Patent Document 4: JP 2024-1185 A

### SUMMARY OF INVENTION

### TECHNICAL PROBLEM

The present invention has been made in view of the above-described circumstances, and provides a material for forming a film, containing a benzenesulfonic acid salt that is not a perfluoroalkyl compound (PFAS). By using an organic film material composition containing the benzenesulfonic acid salt, it is possible to form a film excellent in coating property such as coating defects, such as pinholes on a substrate (wafer), film-formability (in-plane uniformity), and filling property results. Furthermore, when the composition for forming an organic film is used as an organic underlayer film material, it is possible to provide an organic film excellent in process margin when used as an organic underlayer film for a multilayer resist, and the present invention provides a method for forming an organic film, using the composition for forming an organic film, and patterning processes using the composition for forming an organic film. Furthermore, this composition for forming an organic film is also useful as a photoresist material, and is a resist material which is excellent in the flatness of a film after application and in which few defects are generated not only after application but also after development. The present invention further provides a patterning process using the composition. An object of the above-described present invention is to provide a material for forming a film that does not come under PFAS and provide a material for forming a film with little environmental load.

### SOLUTION TO PROBLEM

To achieve the object, the present invention provides the following.

The present invention provides a composition for forming an organic film, comprising: a resin and/or compound (A) for forming an organic film; a benzenesulfonic acid salt compound (B) represented by the following formula (1), an anion moiety in the formula (1) having a molecular weight of 200 or more, and the compound (B) not containing a perfluoroalkyl group; and a solvent (C), 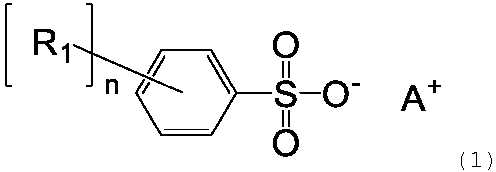 wherein R₁ represents a linear, cyclic, or branched, alkyl group, alkenyl group, oxoalkyl group, aryl group, or aralkyl group having 1 to 20 carbon atoms and optionally having a substituent not containing a perfluoroalkyl group, represents -P(R)₂, R being an alkyl group or aryl group having 1 to 20 carbon atoms, or represents a halogen atom or a nitro group, "n" represents an integer of 1 to 5, and A⁺ represents an ammonium cation, a pyridinium cation, a sulfonium cation, a phosphonium cation, an imidazolium cation, a piperidinium cation, or a pyrrolidinium cation.

Such a composition for forming an organic film can impart photosensitivity, and can be applied as a resist film used for photolithography etc.

Furthermore, a composition for forming an organic film containing a benzenesulfonic acid salt compound having such a molecular weight can work sufficiently as a thermal acid generator or a photo-acid generator, and by virtue of the specification of the molecular weight, prevents sublimation at the time of heat treatment and has excellent in-plane uniformity and sufficient curability in edge portions. Therefore, the film formation material of the present invention can provide a film formation material that can be applied not only to various film formation materials containing only a polymer, but also to film formation materials containing an organic polymer, a compound, or inorganic matter such as silicon, titanium, and zirconium.

The benzenesulfonic acid salt compound (B) is preferably represented by the following general formula (2-1), (2-2), or (2-3), 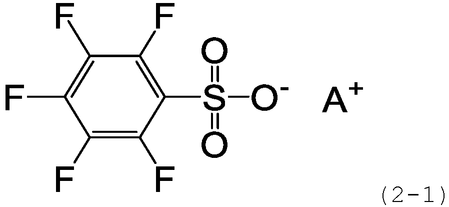
wherein A⁺ is identical to the A⁺ in the formula (1), 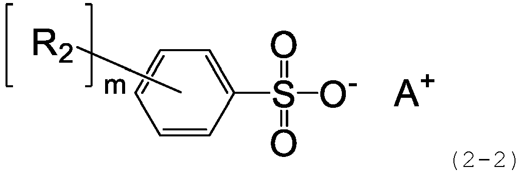
wherein R₂ represents a chlorine atom or an iodine atom; "m" represents an integer of 1 to 5; and A⁺ is identical to the A⁺ in the formula (1), 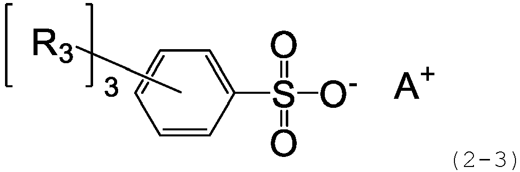
wherein R₃ represents an alkyl group having 1 to 20 carbon atoms and optionally having a substituent not containing a perfluoroalkyl group; and A⁺ is identical to the A⁺ in the formula (1).

A benzenesulfonic acid salt having a substituent shown above functions as an acid generator, also prevents sublimation at the time of heat treatment, and can provide an excellent film formation material.

The anion moiety in the general formula (2-1), (2-2), or (2-3) of the benzenesulfonic acid salt compound (B) preferably has a molecular weight of 245 or more.

Such a composition for forming an organic film can prevent sublimation at the time of heat treatment with certainty, and can also achieve better in-plane uniformity in the formation of an organic film.

It is further preferable that the benzenesulfonic acid salt compound (B) is represented by the general formula (2-3), the R₃ in the formula does not contain a perfluoroalkyl group and represents a branched or cyclic alkyl group having 3 to 20 carbon atoms, and A⁺ represents a triethylammonium cation or a tributylammonium cation.

Such a composition for forming an organic film can be used more favorably from the viewpoint of easily producing materials and the viewpoint of the range of combinations with polymers and compounds for forming an organic film.

The resin or compound (A) for forming an organic film preferably has any of a methylol group, an epoxy group, or a phenolic hydroxy group.

When such a resin or compound (A) for forming an organic film is used, curability is enhanced, and an even better composition for forming an organic film can be provided.

The present invention can also provide a method for forming an organic film to be used in a manufacturing process of a semiconductor device, the method comprising:
spin-coating a substrate to be processed with the above-described composition for forming an organic film; and
forming a cured film by heating the substrate coated with the composition for forming an organic film at a temperature of 100°C or higher and 600°C or lower for 10 to 600 seconds.

Such a method for forming an organic film makes it possible to fill a pattern having a complicated shape on a substrate to be processed by spin-coating, form an organic film having excellent in-plane uniformity, and remove the organic film on an edge portion while suppressing humps in an EBR process.

The present invention also provides a patterning process comprising:
forming an organic film on a body to be processed by using the above-described composition for forming an organic film;
forming a resist middle layer film on the organic film by using a resist middle layer film material containing a silicon atom;
forming a resist upper layer film on the resist middle layer film by using a resist upper layer film material comprising a photoresist composition;
forming a circuit pattern in the resist upper layer film;
transferring the pattern to the resist middle layer film by etching while using the resist upper layer film having the formed circuit pattern as a mask;
transferring the pattern to the organic film by etching while using the resist middle layer film having the transferred pattern as a mask; and
further transferring the pattern to the body to be processed by etching while using the organic film having the transferred pattern as a mask.

The inventive composition for forming an organic film can be used suitably in various patterning processes such as a three-layer resist process using a silicon-containing resist middle layer film or an inorganic hard mask and a four-layer resist process additionally using an organic antireflective film. According to such patterning processes of the present invention, it is possible to transfer and form a circuit pattern of a resist upper layer film in a body to be processed with high accuracy.

The present invention also provides a patterning process comprising:
forming an organic film on a body to be processed by using the above-described composition for forming an organic film;
forming a resist middle layer film on the organic film by using a resist middle layer film material containing a silicon atom;
forming an organic antireflective film or an adhesive film on the resist middle layer film;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the resist middle layer film by etching while using the resist upper layer film having the formed circuit pattern as a mask;
transferring the pattern to the organic film by etching while using the resist middle layer film having the transferred pattern as a mask; and
further transferring the pattern to the body to be processed by etching while using the organic film having the transferred pattern as a mask.

In such a patterning process, the organic antireflective film and the adhesive film can be formed with a known organic antireflective film material by spin-coating, and therefore, such a patterning process is suitably achieved in the same manner as the above-described three-layer resist process using a silicon-containing resist middle layer film, except that an organic antireflective film (BARC) or an adhesive film is formed between the silicon-containing resist middle layer film and the resist upper layer film.

The present invention also provides a patterning process comprising:
forming an organic film on a body to be processed by using the above-described composition for forming an organic film;
forming an inorganic hard mask selected from the group consisting of a silicon oxide film, a silicon nitride film, and a silicon oxynitride film on the organic film;
forming a resist upper layer film on the inorganic hard mask by using a resist upper layer film material comprising a photoresist composition;
forming a circuit pattern in the resist upper layer film;
transferring the pattern to the inorganic hard mask by etching while using the resist upper layer film having the formed circuit pattern as a mask;
transferring the pattern to the organic film by etching while using the inorganic hard mask having the transferred pattern as a mask; and
further transferring the pattern to the body to be processed by etching while using the organic film having the transferred pattern as a mask.

Such a patterning process is suitably achieved in the same manner as the above-described three-layer resist process using a silicon-containing resist middle layer film, except that an inorganic hard mask middle layer film is formed instead of the silicon-containing resist middle layer film on the organic film.

The present invention also provides a patterning process comprising:
forming an organic film on a body to be processed by using the above-described composition for forming an organic film;
forming an inorganic hard mask selected from the group consisting of a silicon oxide film, a silicon nitride film, and a silicon oxynitride film on the organic film;
forming an organic antireflective film or an adhesive film on the inorganic hard mask;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the inorganic hard mask by etching while using the resist upper layer film having the formed circuit pattern as a mask;
transferring the pattern to the organic film by etching while using the inorganic hard mask having the transferred pattern as a mask; and
further transferring the pattern to the body to be processed by etching while using the organic film having the transferred pattern as a mask.

Such a patterning process is suitably achieved in the same manner as the above-described three-layer resist process using an inorganic hard mask middle layer film, except that an organic antireflective film (BARC) or an adhesive film is formed between the inorganic hard mask middle layer film and the resist upper layer film.

In the inventive patterning process, the inorganic hard mask is preferably formed by a CVD method or an ALD method.

According to a patterning process in which the inorganic hard mask is formed by a CVD method or an ALD method as described, the pattern can be formed suitably.

The circuit pattern is preferably formed by a lithography using light having a wavelength of 10 nm or more and 300 nm or less, a direct writing with electron beam, nanoimprinting, or a combination thereof.

According to such a means for forming a circuit pattern, a fine pattern can be formed more suitably.

The circuit pattern is preferably developed with an alkaline development or an organic solvent.

According to such a patterning process, the circuit pattern can be formed more suitably.

In the patterning process, the body to be processed is preferably a semiconductor device substrate or the semiconductor device substrate coated with any of a metal film, a metal carbide film, a metal oxide film, a metal nitride film, a metal oxycarbide film, and a metal oxynitride film.

When the body to be processed is as described, the pattern can be formed suitably.

In the patterning process, the metal constituting the body to be processed is preferably silicon, titanium, tungsten, hafnium, zirconium, chromium, germanium, copper, silver, gold, aluminum, indium, gallium, arsenic, palladium, iron, tantalum, iridium, molybdenum, or an alloy thereof.

When the body to be processed is as described, the pattern can be formed suitably.

The present invention also provides a benzenesulfonic acid salt compound for forming an organic film, being represented by the following formula (1), an anion moiety in the formula (1) having a molecular weight of 200 or more, and the compound not containing a perfluoroalkyl group, 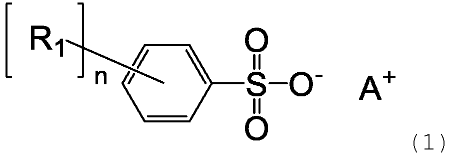 wherein R₁ represents a linear, cyclic, or branched, alkyl group, alkenyl group, oxoalkyl group, aryl group, or aralkyl group having 1 to 20 carbon atoms and optionally having a substituent not containing a perfluoroalkyl group, represents -P(R)₂, R being an alkyl group or aryl group having 1 to 20 carbon atoms, or represents a halogen atom or a nitro group, "n" represents an integer of 1 to 5, and A⁺ represents an ammonium cation, a pyridinium cation, a sulfonium cation, a phosphonium cation, an imidazolium cation, a piperidinium cation, or a pyrrolidinium cation.

Such a benzenesulfonic acid salt compound for forming an organic film can be used suitably in the inventive composition for forming an organic film, the inventive method for forming an organic film, and the inventive patterning processes.

The benzenesulfonic acid salt compound for forming an organic film is preferably represented by the following general formula (2-1), (2-2), or (2-3), 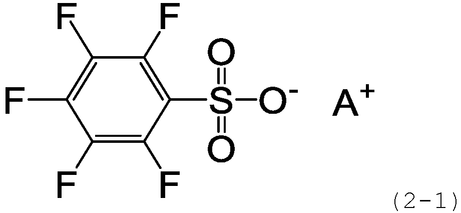
wherein A⁺ is identical to the A⁺ in the formula (1), 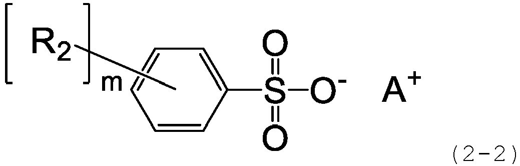
wherein R₂ represents a chlorine atom or an iodine atom; "m" represents an integer of 1 to 5; and A⁺ is identical to the A⁺ in the formula (1), 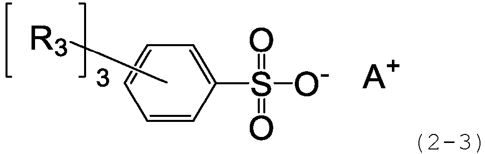
wherein R₃ represents an alkyl group having 1 to 20 carbon atoms and optionally having a substituent not containing a perfluoroalkyl group; and A⁺ is identical to the A⁺ in the formula (1).

Such a benzenesulfonic acid salt compound for forming an organic film is applied more suitably for forming an organic film.

In the benzenesulfonic acid salt compound for forming an organic film, the anion moiety in the general formula (2-1), (2-2), or (2-3) preferably has a molecular weight of 245 or more.

Such a benzenesulfonic acid salt compound for forming an organic film can prevent sublimation at the time of heating with certainty, and in addition, can be applied further preferably for forming an organic film.

It is preferable that the benzenesulfonic acid salt compound for forming an organic film is represented by the general formula (2-3), the R₃ in the formula not containing a perfluoroalkyl group, the compound being a branched or cyclic alkyl group having 3 to 20 carbon atoms, and A⁺ representing a triethylammonium cation or a tributylammonium cation.

Such a benzenesulfonic acid salt compound for forming an organic film is applied particularly favorably for forming an organic film.

The present invention also provides a patterning process comprising:
forming a resist film on a body to be processed by using a resist material comprising the above-described composition for forming an organic film;
exposing the resist film to a high-energy beam; and
developing the exposed resist film by using a developer.

According to such a patterning process, it is possible to form a fine pattern by using a high-energy beam, and it is possible to process a fine pattern having excellent in-plane uniformity and having few defects.

### ADVANTAGEOUS EFFECTS OF INVENTION

As described above, in the present invention, it is possible to apply a film formation material excellent in film-formability (in-plane uniformity) on a substrate (wafer) and filling property, and when an organic film formation material having a combination including a resin and/or compound for forming an organic film is used for an organic underlayer film, it is possible to provide a composition for forming an organic film excellent in film-formability on an organic film.

In addition, when the composition for forming an organic film contains a combination of a photo-acid generator etc. and is used as a resist material, the resist material can be used for photolithography excellent in in-plane uniformity, and a fine pattern can be processed.

The inventive composition for forming an organic film is extremely useful as: an organic film material used in multilayer resist processes, such as a two-layer resist process, a three-layer resist process using a silicon-containing resist middle layer film or an inorganic hard mask, or a four-layer resist process using a silicon-containing resist middle layer film or inorganic hard mask and an organic antireflective film; or as a film formation material, such as a photoresist material, for manufacturing a semiconductor device.

An organic film obtained using the inventive composition for forming an organic film can achieve excellent etching resistance during etching of the body to be processed.

### BRIEF DESCRIPTION OF DRAWINGS

FIG. 1 is an explanatory diagram of an example of the inventive patterning process according to a three-layer resist process.

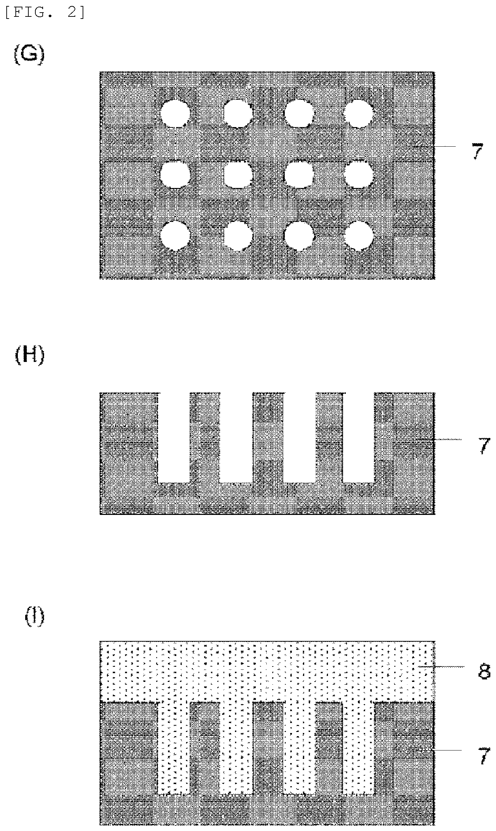

FIG. 2 is an explanatory diagram of a filling property evaluation method in the Examples.

### DESCRIPTION OF EMBODIMENTS

As described above, there has been required a film formation material that is excellent in film-formability, can realize high in-plane uniformity and filling property, and can withstand environmental restriction.

For example, when an organic film is to be formed, a resin for forming an organic film, a thermal acid generator, etc. are dissolved in an organic solvent to form a composition, the composition is applied with a coater and a developer onto a substrate on which a structure, a circuit, etc. is formed, the composition is spread by the substrate rotating, the composition on the edge is removed in an EBR process, and then the composition is baked to form an organic film.

In the process of baking the composition, if the thermal acid generator has a small molecular weight, the acid generator sublimes during baking. Thus, the curing of the film mainly in peripheral portions of the wafer becomes insufficient, and uniformity over the entire film becomes degraded.

The present inventors have further studied earnestly and found out that when a benzenesulfonic acid salt compound having a particular molecular weight and having a particular substituent is contained, a composition for forming an organic film has excellent film-formability, high filling property, and excellent flatness. Thus, the present invention has been completed.

In addition, the present inventors have studied earnestly to achieve a film formation material which has excellent film-formability, for example, high flatness after application, favorable coating property in the outermost edge portion of a substrate (wafer), etc., recently desired, and in which few defects are generated after application, after development, and furthermore, after etching, and found out that a benzenesulfonic acid salt compound in which the anion moiety has a molecular weight of 200 or more is a promising material. Thus, the present inventors have arrived at the present invention.

Furthermore, from the viewpoint of recently tightened environmental restriction, there are demands for the development of a material that does not come under PFAS. Compounds that do not contain a perfluoroalkyl group are not classified as PFAS in OECD, and therefore, are promising as film formation materials.

That is, the present invention is a composition for forming a film, containing a benzenesulfonic acid salt compound which does not contain a perfluoroalkyl group and in which the anion moiety has a molecular weight of 200 or more.

Hereinafter, the present invention will be described in detail, but the present invention is not limited thereto.

### [Composition for Forming Organic Film]

The present invention is a composition for forming an organic film, containing: a resin and/or compound (A) for forming an organic film; a benzenesulfonic acid salt compound (B) represented by the following formula (1), an anion moiety in the formula (1) having a molecular weight of 200 or more, and the compound (B) not containing a perfluoroalkyl group; and a solvent (C), 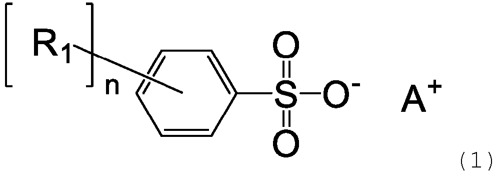 where R₁ represents a linear, cyclic, or branched, alkyl group, alkenyl group, oxoalkyl group, aryl group, or aralkyl group having 1 to 20 carbon atoms and optionally having a substituent not containing a perfluoroalkyl group, represents -P(R)₂, R being an alkyl group or aryl group having 1 to 20 carbon atoms, or represents a halogen atom or a nitro group, "n" represents an integer of 1 to 5, and A⁺ represents an ammonium cation, a pyridinium cation, a sulfonium cation, a phosphonium cation, an imidazolium cation, a piperidinium cation, or a pyrrolidinium cation.

The benzenesulfonic acid salt compound (B) is preferably represented by the following general formula (2-1), (2-2), or (2-3), 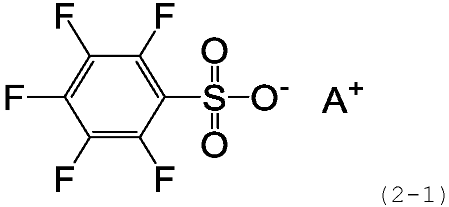
where A⁺ is identical to the A⁺ in the formula (1), 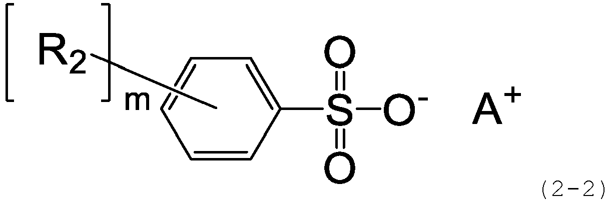
where R₂ represents a chlorine atom or an iodine atom; "m" represents an integer of 1 to 5; and A⁺ is identical to the A⁺ in the formula (1), 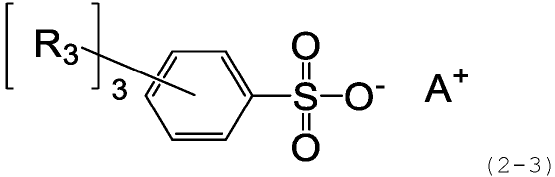
where R₃ represents an alkyl group having 1 to 20 carbon atoms and optionally having a substituent not containing a perfluoroalkyl group; and A⁺ is identical to the A⁺ in the formula (1).

The anion moiety in the general formula (2-1), (2-2), or (2-3) of the benzenesulfonic acid salt compound (B) preferably has a molecular weight of 245 or more.

It is preferable that the benzenesulfonic acid salt compound (B) is represented by the general formula (2-3), the R₃ in the formula does not contain a perfluoroalkyl group and represents a branched or cyclic alkyl group having 3 to 20 carbon atoms, and A⁺ represents a triethylammonium cation or a tributylammonium cation.

In the general formula (1), R₁ represents a linear, cyclic, or branched, alkyl group, alkenyl group, oxoalkyl group, aryl group, or aralkyl group having 1 to 20 carbon atoms and optionally having a substituent not containing a perfluoroalkyl group, represents -P(R)₂, R being an alkyl group or aryl group having 1 to 20 carbon atoms, or represents a halogen atom or a nitro group, "n" represents an integer of 1 to 5.

In the general formula (1), A⁺ represents an ammonium cation (excluding a tetramethylbenzylammonium cation), a pyridinium cation, a sulfonium cation, a phosphonium cation, an imidazolium cation, a piperidinium cation, or a pyrrolidinium cation.

As specific examples, examples of ammonium cations include trimethylammonium cation, triethylammonium cation, tripropylammonium cation, triisopropylammonium cation, tributylammonium cation, triisobutylammonium cation, tripentylammonium cation, trihexylammonium cation, trioctylammonium cation, tetramethylammonium cation, tetraethylammonium cation, tetrapropylammonium cation, tetraisopropylammonium cation, tetrabutylammonium cation, tetraisobutylammonium cation, tetrapentylammonium cation, tetrahexylammonium cation, and tetraoctylammonium cation; examples of pyridinium cations include pyridinium cation, methylpyridinium cation, ethylpyridinium cation, propylpyridinium cation, isopropylpyridinium cation, butylpyridinium cation, fluoropyridinium cation, chloropyridinium cation, bromopyridinium cation, iodopyridinium cation, dimethylaminopyridinium cation, and trimethylpyridinium cation; examples of sulfonium cations include triphenylsulfonium cation, diphenyl[4-(phenylthio)phenyl]sulfonium cation, tris(4-methylphenyl)sulfonium cation, tris(4-t-butylphenyl)sulfonium cation, 5-phenyldibenzothiophenium cation, and 10-phenylphenoxathiinium cation; examples of phosphonium cations include triethylphosphonium cation, tributylphosphonium cation, triphenylphosphonium cation, tetraethylphosphonium cation, tetrabutylphosphonium cation, and ethyltriphenylphosphonium cation; examples of imidazolium cations include 1-allyl-3-methylimidazolium cation, 1-butyl-3-methylimidazolium cation, 1-benzyl-3-methylimidazolium cation, 1,3-dimethylimidazolium cation, and 2,3-dimethyl-1-propylimidazolium cation; examples of piperidinium cations include 1-butyl-1-methylpiperidinium cation and 1-methyl-1-propylpiperidinium cation; and examples of pyrrolidinium cations include 1-allyl-1-methylpyrrolidinium cation, 1-butyl-1-methylpyrrolidinium cation, 1-ethyl-1-methylpyrrolidinium cation, 1-methyl-1-propylpyrrolidinium cation, and 1-(2-methoxymethyl)-1-methylpyrrolidinium cation. From simplicity and convenience on manufacturing, a tetraethylammonium cation or a tetrabutylammonium cation is preferable.

It is preferable that the benzenesulfonic acid salt compound (B) is represented by the general formula (2-3), the R₃ in the formula does not contain a perfluoroalkyl group and represents a branched or cyclic alkyl group having 3 to 20 carbon atoms, and A⁺ represents a triethylammonium cation or a tributylammonium cation.

The R₂ in the general formula (2-2) represents a chlorine atom or an iodine atom. "m" represents an integer of 1 to 5, and in the case of a chlorine atom, "m" is preferably 2 or 3 and in the case of an iodine atom, "m" is preferably 1 or 2.

The R₃ in the general formula (2-3) represents an alkyl group having 1 to 20 carbon atoms and optionally having a substituent not containing a perfluoroalkyl group. Specific examples thereof include a methyl group, an ethyl group, a propyl group, an isopropyl group, a butyl group, an isobutyl group, a pentyl group, a hexyl group, an octyl group, a dodecyl group, a cyclopentyl group, a cyclohexyl group, a norbornyl group, and an adamantyl group. Preferable are branched or cyclic alkyl groups having 3 to 20 carbon atoms, and examples include an isopropyl group, an isobutyl group, a cyclopentyl group, and a cyclohexyl group.

Examples of the anion component of the benzenesulfonic acid salt compound include the following structures, but are not limited thereto.

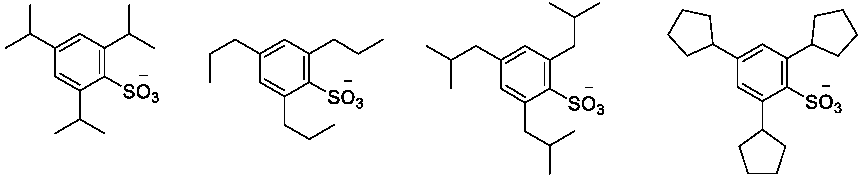

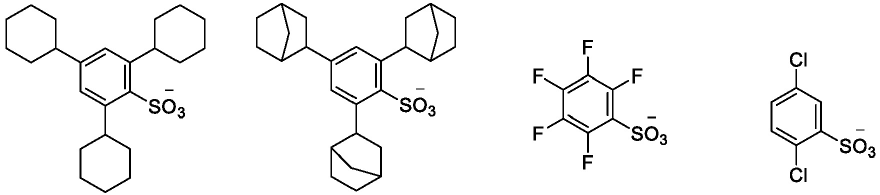

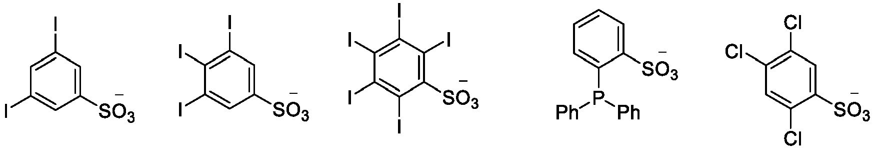

As the cation component of the benzenesulfonic acid salt compound, shown are an ammonium cation (excluding a tetramethylbenzylammonium cation), a pyridinium cation, a sulfonium cation, a phosphonium cation, an imidazolium cation, a piperidinium cation, and a pyrrolidinium cation. As specific examples, examples of ammonium cations include trimethylammonium cation, triethylammonium cation, tripropylammonium cation, triisopropylammonium cation, tributylammonium cation, triisobutylammonium cation, tripentylammonium cation, trihexylammonium cation, trioctylammonium cation, tetramethylammonium cation, tetraethylammonium cation, tetrapropylammonium cation, tetraisopropylammonium cation, tetrabutylammonium cation, tetraisobutylammonium cation, tetrapentylammonium cation, tetrahexylammonium cation, and tetraoctylammonium cation; examples of pyridinium cations include pyridinium cation, methylpyridinium cation, ethylpyridinium cation, propylpyridinium cation, isopropylpyridinium cation, butylpyridinium cation, fluoropyridinium cation, chloropyridinium cation, bromopyridinium cation, iodopyridinium cation, dimethylaminopyridinium cation, and trimethylpyridinium cation; examples of sulfonium cations include triphenylsulfonium cation, diphenyl[4-(phenylthio)phenyl]sulfonium cation, tris(4-methylphenyl)sulfonium cation, tris(4-t-butylphenyl)sulfonium cation, 5-phenyldibenzothiophenium cation, and 10-phenylphenoxathiinium cation; examples of phosphonium cations include triethylphosphonium cation, tributylphosphonium cation, triphenylphosphonium cation, tetraethylphosphonium cation, tetrabutylphosphonium cation, and ethyltriphenylphosphonium cation; examples of imidazolium cations include 1-allyl-3-methylimidazolium cation, 1-butyl-3-methylimidazolium cation, 1-benzyl-3-methylimidazolium cation, 1,3-dimethylimidazolium cation, and 2,3-dimethyl-1-propylimidazolium cation; examples of piperidinium cations include 1-butyl-1-methylpiperidinium cation and 1-methyl-1-propylpiperidinium cation; and examples of pyrrolidinium cations include 1-allyl-1-methylpyrrolidinium cation, 1-butyl-1-methylpyrrolidinium cation, 1-ethyl-1-methylpyrrolidinium cation, 1-methyl-1-propylpyrrolidinium cation, and 1-(2-methoxymethyl)-1-methylpyrrolidinium cation. From simplicity and convenience on manufacturing, a tetraethylammonium cation or a tetrabutylammonium cation is preferable.

A common method for synthesizing the inventive benzenesulfonic acid salt compound represented by the formula (1) will be described.

1 mol of benzenesulfonic acid is dissolved in water to obtain an approximately 10% aqueous solution, and 1 mol of amine is added dropwise thereto at room temperature. This solution is aged at room temperature for about 24 hours, and then the solvent is distilled off under reduced pressure. By drying the product under reduced pressure at 50°C, a benzenesulfonic acid salt can be obtained.

In materials, including the benzenesulfonic acid salt compound (B), used in photolithography of semiconductors, metal impurities need to be reduced. This is because, if a metal impurity is present, the metal impurity adversely affects the operation of a device manufactured using the material. To reduce metal, washing with pure water, an ion-exchanged resin, etc. are used. The amount of metal impurities is preferably 100 ppm or less, more preferably 100 ppb or less, in the case of a resist material.

The film formation material of the present invention preferably contains 0.0001 to 10 mass% of the benzenesulfonic acid salt compound (B). The above-described polymer is preferably contained in an amount of 0.1 to 7 mass%, more preferably 2 to 5 mass%. Here, the benzenesulfonic acid salt compound (B) contributes as an acid generator for imparting film curability. By combining the benzenesulfonic acid salt compound (B) as an acid generator with an organic compound or the like, high film-formability can be imparted.

The organic film formation material can be used as a component of photosensitive or non-photosensitive resist materials, materials for forming a top coat to be formed on a resist film, materials for forming a resist middle layer film, etc. As an underlayer film material, the material is applicable not only to organic underlayer films containing an organic compound or resin, but also to film formation materials containing a metallic element, such as silicon, titanium, zirconium, tin, and hafnium. For example, the resist material may be a chemically-amplified resist or a non-chemically-amplified resist, and may be a hydrocarbon-based resist or a metal-based resist containing silicon, titanium, zirconium, hafnium, selenium, germanium, zinc, iron, cobalt, nickel, copper, tin, antimony, molybdenum, tungsten, indium, etc. Examples other than organic compounds or resins include silicon-containing resist middle layer film materials of JP 2007-302873 A, JP 2008-19423 A, etc., coating-type compositions for forming a BPSG film of JP 2016-074774 A, etc., titanium-containing resist middle layer film materials of JP 2014-199429 A, JP 2014-178602 A, etc., and metal oxide film formation materials of JP 2014-134581 A, etc. Besides these, the organic film formation material can be expected to be applied to various materials as an acid generator at the time of film formation.

By combining the above-described benzenesulfonic acid salt compound (B) with a resin and/or compound (A) for forming an organic film and a solvent, the mixture can be used as a composition for forming an organic film.

When the amount of the resin and/or compound (A) for forming an organic film is regarded as 100 parts by mass, a composition for forming an organic film in which the benzenesulfonic acid salt compound (B) is contained in an amount of 0.01 parts by mass to 5 parts by mass is provided.

When the benzenesulfonic acid salt compound (B) is contained in the above-described range, the film-formability of the composition for forming an organic film can be enhanced. Therefore, although there are various resins and compounds, such as resins having various repeating units, compounds having a rigid structure and having high crystallinity, and compounds having a substituent having high polarity, the inventive benzenesulfonic acid salt compound (B) can provide a composition for forming an organic film excellent in film-formability even when such resins and compounds having different properties are used.

It is possible to obtain a composition for forming an organic film containing the benzenesulfonic acid salt compound (B) in which the cation moiety is photodegradable.

When such a composition for forming an organic film is obtained, photosensitivity and so forth can be imparted to the composition for forming an organic film, and the organic film formation material can be applied as a resist material used in photolithography and so forth.

Note that, in the inventive composition for forming an organic film, one kind of each of the benzenesulfonic acid salt compound (B), the resin and/or compound (A) for forming an organic film, and the solvent (C) may be used, or two or more kinds of each may be used in combination. The inventive benzenesulfonic acid salt compound (B) is excellent in heat resistance, and functions as an acid generator that imparts excellent film-formability and high leveling property. Uses of the compound are not limited to organic underlayer films, and the compound can be used for coating materials for photolithography in general. Examples of uses include photosensitive resist materials and materials for forming a top coat to be formed on a resist film. Furthermore, the compound is applicable not only to organic underlayer film formation materials but also to silicon-containing resist middle layer films, and is usable as an acid generator suitable for achieving highly versatile film-formability applicable to various film formation materials.

Note that, when the inventive benzenesulfonic acid salt compound (B) described above is used as an acid generator, one kind of the compound may be used, or two or more kinds thereof may be used in combination. These benzenesulfonic acid salt compounds (B) are preferably contained in an amount of 0.01 parts by mass to 10 parts by mass, further preferably 0.01 parts by mass to 5 parts by mass based on 100 parts by mass of the resin and/or compound (A) for forming an organic film.

### [Resin and/or Compound (A) for Forming Organic Film]

The resin and/or compound (A) for forming an organic film contained in the inventive composition for forming an organic film is not particularly limited as long as the material is a resin or compound that has sufficient film-formability in spin-coating and curability. When the composition is used as an organic underlayer film material, a resin or compound including an aromatic skeleton is preferable from the viewpoints of etching resistance, optical characteristics, heat resistance, etc.

Examples of the aromatic skeleton include benzene, naphthalene, anthracene, pyrene, indene, fluorene, furan, pyrrole, thiophene, phosphole, pyrazole, oxazole, isoxazole, thiazole, pyridine, pyrazine, pyrimidine, pyridazine, triazine, carbazole, etc. Among these, benzene, naphthalene, fluorene, and carbazole are particularly preferable.

A composition for forming an organic film in which the resin or compound (A) for forming an organic film has any of a methylol group, an epoxy group, or a phenolic hydroxy group is preferable. When such a resin or compound (A) for forming an organic film is contained, curability is enhanced, and an even better composition for forming an organic film can be provided.

Examples of the resin and/or compound (A) for forming an organic film used in the present invention include resins containing the following structures, disclosed in JP 2012-001687 A and JP 2012-077295 A.

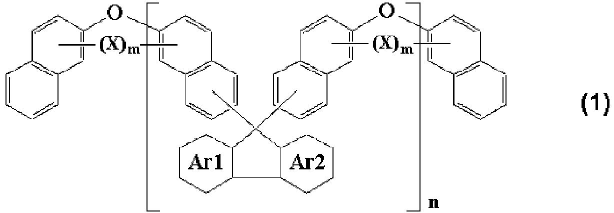

In the formula (1), the cyclic structures Ar1 and Ar2 each represent a benzene ring or a naphthalene ring. X represents a single bond or an alkylene group having 1 to 20 carbon atoms. "m" represents 0 or 1. "n" represents any natural number that provides a molecular weight of 100,000 or less. Note that the symbols in the formula apply only in this formula.

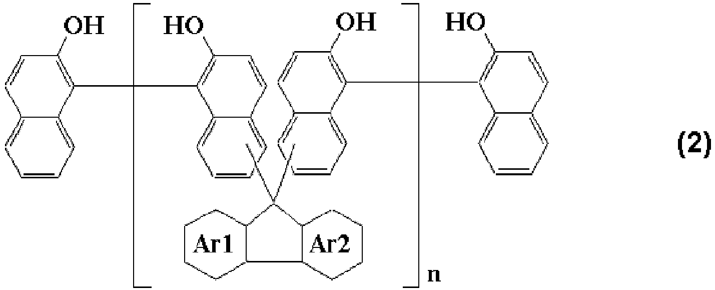

In the formula (2), the cyclic structures Ar1 and Ar2 each represent a benzene ring or a naphthalene ring. "n" represents any natural number that provides a weight-average molecular weight of 100,000 or less as measured by gel permeation chromatography in terms of polystyrene. Note that the symbols in the formula apply only in this formula.

Further examples of the resin and/or compound (A) for forming an organic film used in the present invention include resins containing the following structures, disclosed in JP 2004-264710 A, JP 2005-043471 A, JP 2005-250434 A, JP 2007-293294 A, and JP 2008-065303 A.

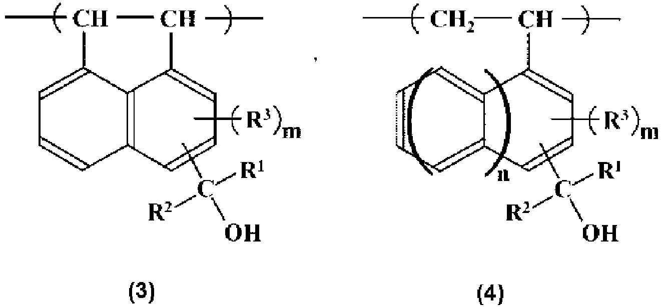

In the formula (3) and the formula (4), R¹ and R² each represent a hydrogen atom, an alkyl group having 1 to 3 carbon atoms, or an aryl group; R³ represents an alkyl group having 1 to 3 carbon atoms, a vinyl group, an allyl group, or an aryl group that is optionally substituted; "n" represents 0 or 1; and "m" represents 0, 1, or 2. Note that the symbols in the formulae apply only in these formulae.

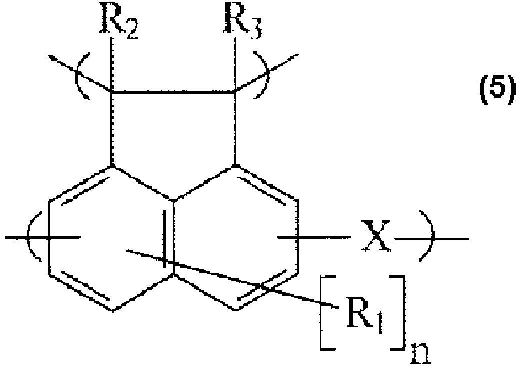

In the formula (5), R₁ represents a monovalent atom other than a hydrogen atom or a monovalent group; and "n" represents an integer of 0 to 4. Here, when "n" is 2 to 4, the multiple R₁'s may be identical to or different from each other. R₂ and R₃ each independently represent a monovalent atom or group. X represents a divalent group. Note that the symbols in the formula apply only in this formula. 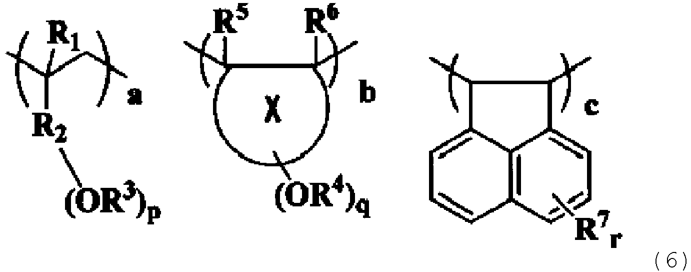 In the formula (6), R₁ represents a hydrogen atom or a methyl group. R₂ represents a single bond, a linear, branched, or cyclic alkylene group having 1 to 20 carbon atoms, or an arylene group having 6 to 10 carbon atoms, and may have any of ether, ester, lactone, and amide. R³ and R⁴ each represent a hydrogen atom or a glycidyl group. X represents any polymer of any of a hydrocarbon including an indene skeleton, a cycloolefin having 3 to 10 carbon atoms, and maleimide, and may have any of ether, ester, lactone, and carboxylic anhydride. R⁵ and R⁶ each represent a hydrogen atom, a fluorine atom, a methyl group, or a trifluoromethyl group. R⁷ represents a hydrogen atom, a linear, branched, or cyclic alkyl group having 1 to 6 carbon atoms, a hydroxy group, or an alkoxycarbonyl group. "p" and "q" each represent an integer of 1 to 4. "r" represents an integer of 0 to 4. "a", "b", and "c" each satisfy 0.5 ≤ a+b+c ≤ 1, 0 ≤ a ≤ 0.8, 0 ≤ b ≤ 0.8, 0.1 ≤ a+b ≤ 0.8, and 0.1 ≤ c ≤ 0.8. Note that the symbols in the formula apply only in this formula.

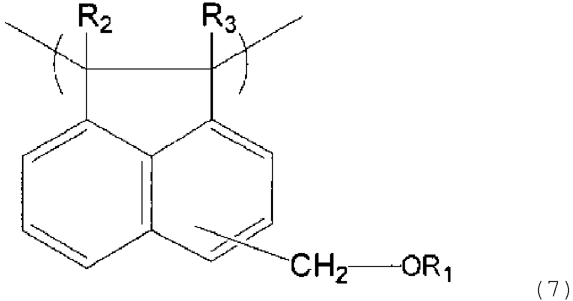

In the formula (7), R₁ represents a hydrogen atom or a monovalent organic group; and R₂ and R₃ each independently represent a monovalent atom or a monovalent organic group. Note that the symbols in the formula apply only in this formula.

Specific examples of the resin and/or compound (A) for forming an organic film used in the present invention include resins containing the following structures, disclosed in JP 2004-205685 A, JP 2007-171895 A, and JP 2009-014816 A.

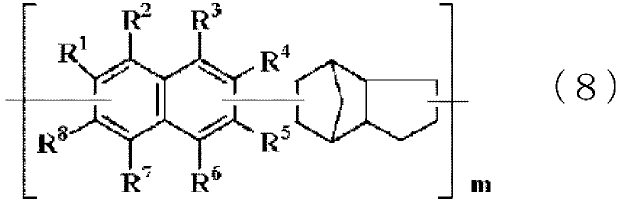

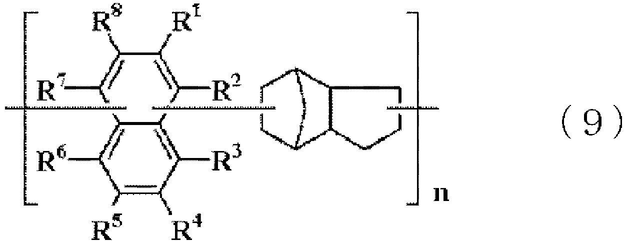

In the formula (8) and the formula (9), R¹ to R⁸ each independently represent a hydrogen atom, a hydroxy group, an alkyl group having 1 to 6 carbon atoms and optionally being substituted, an alkoxy group having 1 to 6 carbon atoms and optionally being substituted, an alkoxycarboxy group having 2 to 6 carbon atoms and optionally being substituted, an aryl group having 6 to 10 carbon atoms and optionally being substituted, a hydroxyalkyl group having 1 to 6 carbon atoms, an isocyanate group, or a glycidyl group. "m" and "n" each represent a positive integer. Note that the symbols in the formulae apply only in these formulae.

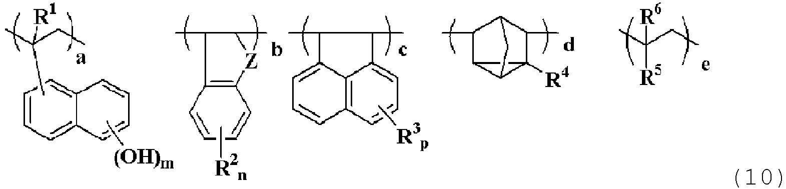

In the formula (10), R¹ and R⁶ each represent a hydrogen atom or a methyl group. R², R³, and R⁴ each represent a hydrogen atom, an alkyl group having 1 to 4 carbon atoms, an alkoxy group, a hydroxy group, an acetoxy group, an alkoxycarbonyl group, or an aryl group having 6 to 10 carbon atoms; R⁵ represents a condensed polycyclic hydrocarbon group having 13 to 30 carbon atoms, -O-R⁷, -C(=O)-O-R⁷, -O-C(=O)-R⁷, or -C(=O)-NR⁸-R⁷; "m" represents 1 or 2; "n" represents an integer of 0 to 4; and "p" represents an integer of 0 to 6. R⁷ represents an organic group having 7 to 30 carbon atoms; R⁸ represents a hydrogen atom or a hydrocarbon group having 1 to 6 carbon atoms. Z represents a methylene group, -O-, -S-, or -NH-. "a", "b", "c", "d", and "e" each satisfy 0 < a < 1.0, 0 ≤ b ≤ 0.8, 0 ≤ c ≤ 0.8, 0 ≤ d ≤ 0.8, 0 ≤ e ≤ 0.8, and 0 < b+c+d+e < 1.0. Note that the symbols in the formula apply only in this formula.

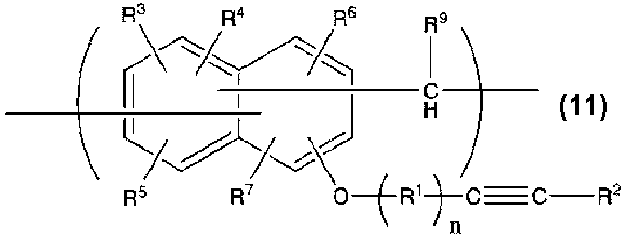

In the formula (11), "n" represents 0 or 1. R¹ represents a methylene group that is optionally substituted, an alkylene group having 2 to 20 carbon atoms and optionally being substituted, or an arylene group having 6 to 20 carbon atoms and optionally being substituted. R² represents a hydrogen atom, an alkyl group having 1 to 20 carbon atoms and optionally being substituted, or an aryl group having 6 to 20 carbon atoms and optionally being substituted. R³ to R⁷ each represent a hydroxy group, an alkyl group having 1 to 6 carbon atoms and optionally being substituted, an alkoxy group having 1 to 6 carbon atoms and optionally being substituted, an alkoxycarbonyl group having 2 to 10 carbon atoms and optionally being substituted, an aryl group having 6 to 14 carbon atoms and optionally being substituted, or a glycidyl ether group having 2 to 6 carbon atoms and optionally being substituted. R⁹ represents a hydrogen atom, a linear, branched, or cyclic alkyl group having 1 to 10 carbon atoms, a linear, branched, or cyclic alkyl ether group having 1 to 10 carbon atoms, or an aryl group having 6 to 10 carbon atoms. Note that the symbols in the formula apply only in this formula.

Examples of the formula (11) include the following resins.

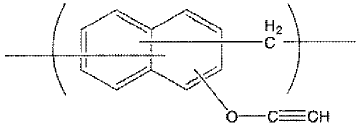

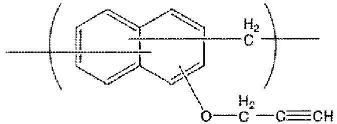

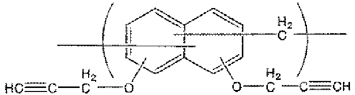

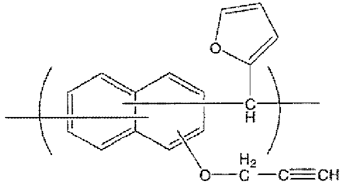

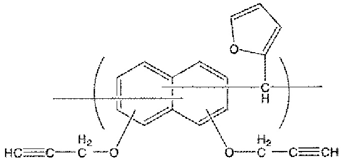

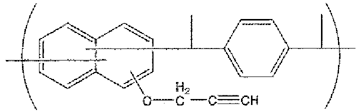

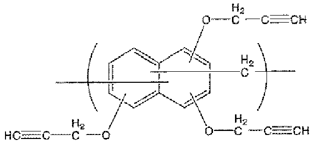

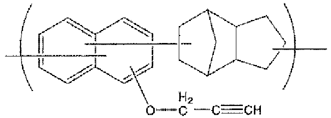

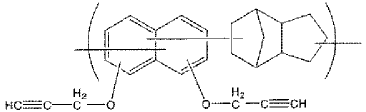

Further examples of the resin and/or compound (A) for forming an organic film used in the present invention include resins containing the following structures, disclosed in JP 2007-199653 A, JP 2008-274250 A, and JP 2010-122656 A.

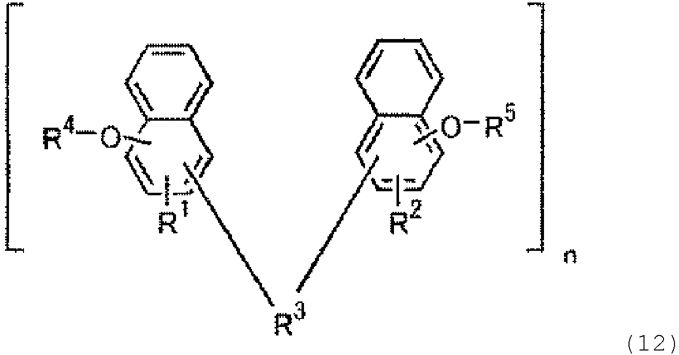

In the formula (12), R¹ and R² are identical to or different from each other and each independently represent a hydrogen atom, a linear, branched, or cyclic alkyl group having 1 to 10 carbon atoms, an aryl group having 6 to 10 carbon atoms, or an alkenyl group having 2 to 10 carbon atoms; R³ represents a single bond or a linear, branched, or cyclic alkylene group having 1 to 30 carbon atoms, and optionally has a bridged cyclic hydrocarbon group, a double bond, a heteroatom, or an aromatic group having 6 to 30 carbon atoms; R⁴ and R⁵ each independently represent a hydrogen atom or a glycidyl group; and "n" represents an integer of 1 to 4. Note that the symbols in the formula apply only in this formula.

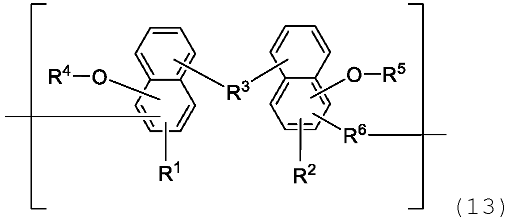

In the formula (13), R¹ and R² are identical to or different from each other and each independently represent a hydrogen atom, a linear, branched, or cyclic alkyl group having 1 to 10 carbon atoms, an aryl group having 6 to 10 carbon atoms, or an alkenyl group having 2 to 10 carbon atoms; R³ represents a single bond or a linear, branched, or cyclic alkylene group having 1 to 30 carbon atoms, and optionally has a bridged cyclic hydrocarbon group, a double bond, a heteroatom, or an aromatic group having 6 to 30 carbon atoms; R⁴ and R⁵ each independently represent a hydrogen atom or a glycidyl group; and R⁶ represents a single bond or a linear or branched alkylene group having 1 to 10 carbon atoms. Note that the symbols in the formula apply only in this formula.

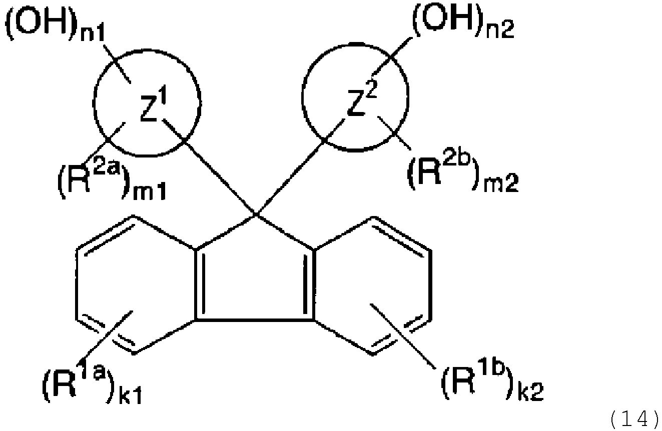

In the formula (14), the ring Z¹ and the ring Z² each represent a condensed polycyclic aromatic hydrocarbon ring; and R^{1a}, R^{1b}, R^{2a}, and R^{2b} are identical to or different from each other and each represent a substituent. "k1" and "k2" may be identical to or different from each other and each represent 0 or an integer of 1 to 4; "m1" and "m2" each represent 0 or an integer of 1 or more; and "n1" and "n2" each represent 0 or an integer of 1 or more. Here, n1+n2 ≥ 1. Note that the symbols in the formula apply only in this formula.

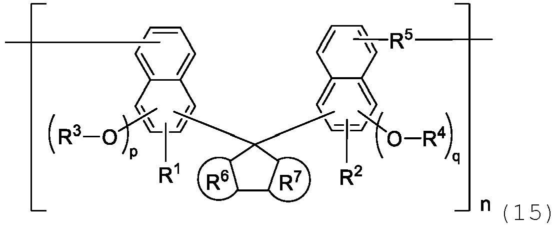

In the formula (15), R₁ and R₂ are identical to or different from each other, and each represent a hydrogen atom, a linear, branched, or cyclic alkyl group having 1 to 10 carbon atoms, an aryl group having 6 to 10 carbon atoms, or an alkenyl group having 2 to 10 carbon atoms. R³ and R⁴ each represent a hydrogen atom or a glycidyl group; R⁵ represents a single bond or a linear or branched alkylene group having 1 to 10 carbon atoms; and R⁶ and R⁷ each represent a benzene ring or a naphthalene ring. "p" and "q" each represent 1 or 2. "n" satisfies 0 < n ≤ 1. Note that the symbols in the formula apply only in this formula.

Examples of the formula (15) include the following resins.

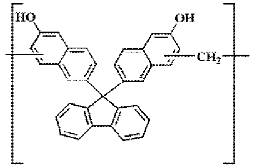

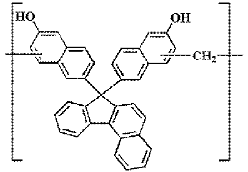

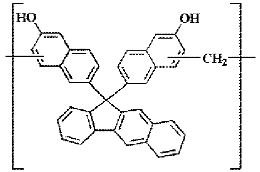

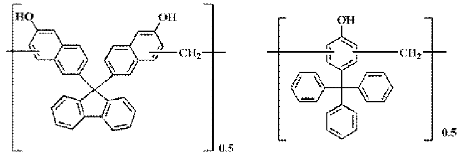

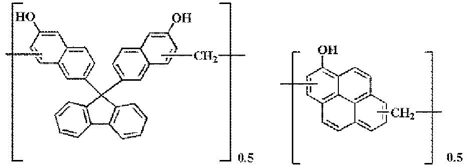

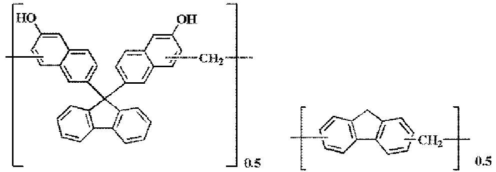

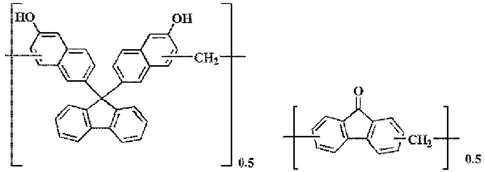

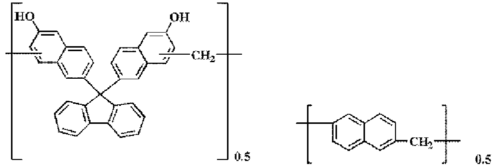

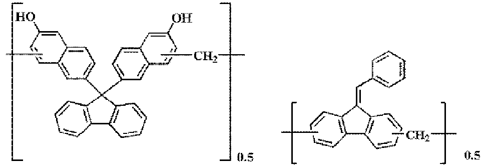

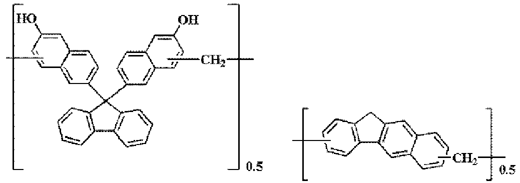

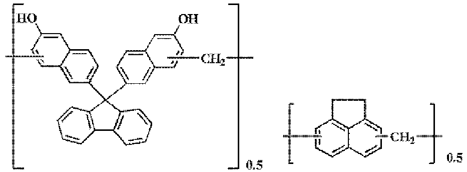

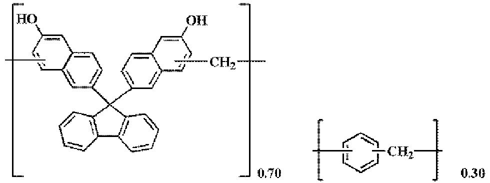

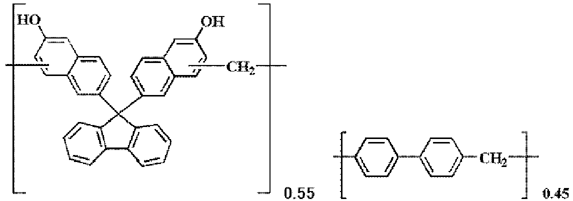

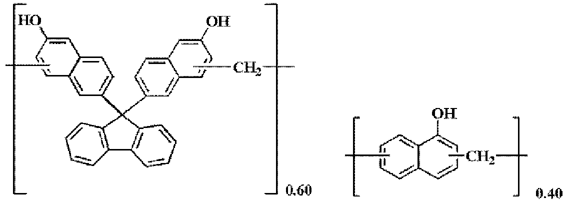

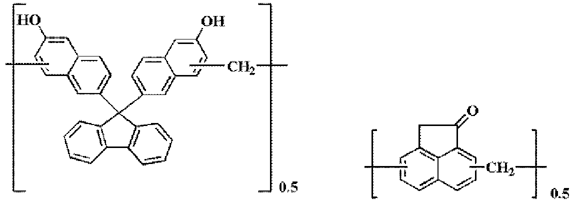

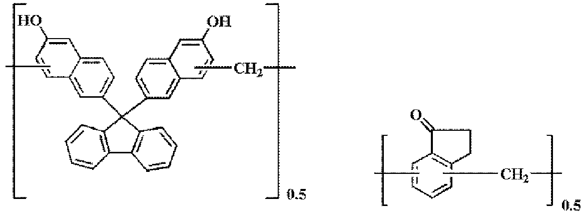

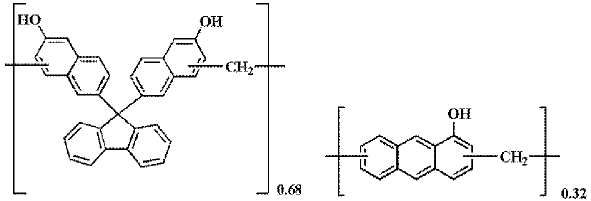

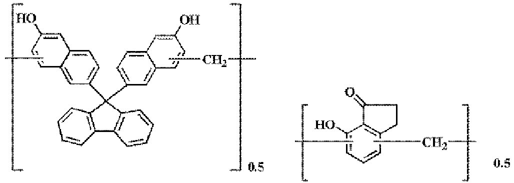

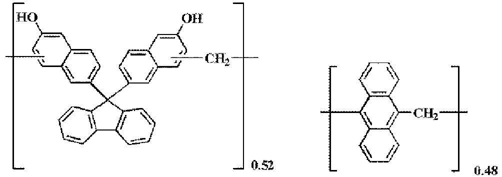

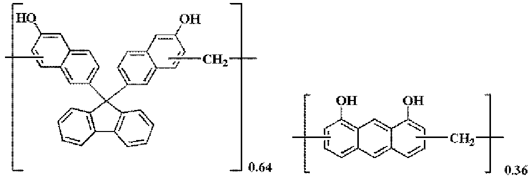

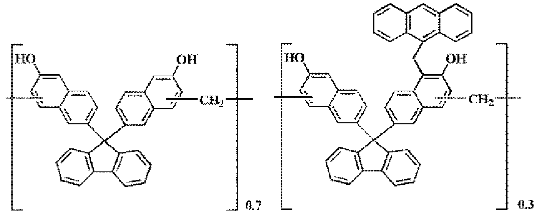

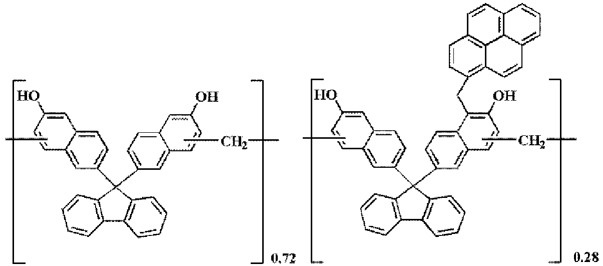

Examples of the resin and/or compound (A) for forming an organic film used in the present invention include resins containing the following structure, disclosed in JP 2012-214720 A.

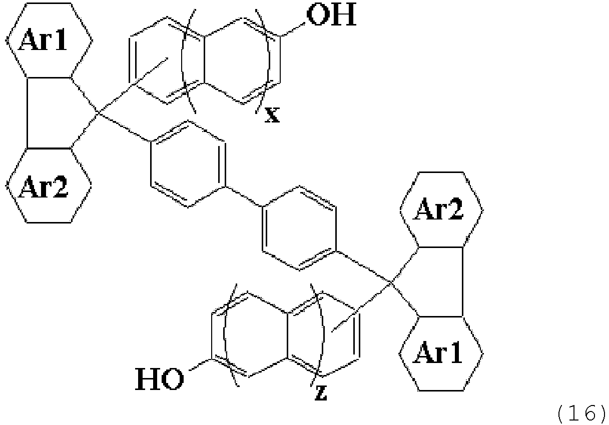

In the formula (16), the cyclic structures Ar1 and Ar2 each represent a benzene ring or a naphthalene ring. "x" and "z" each independently represent 0 or 1. Note that the symbols in the formula apply only in this formula.

Examples of the resin and/or compound (A) for forming an organic film used in the present invention include resins disclosed in JP 2014-029435 A.

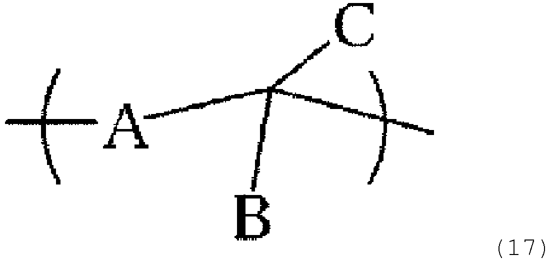

In the formula (17), A represents a structure having carbazole, B represents a structure having an aromatic ring, and C represents a hydrogen atom, an alkyl group, or a structure having an aromatic ring, B and C optionally constituting a ring with each other. One to four carboxy groups, a salt thereof, or a carboxylic acid ester group is contained in the combined structure of A, B, and C. Note that the symbols in the formula apply only in this formula.

Examples of the resin and/or compound (A) for forming an organic film used in the present invention include polymers disclosed in WO 2012/077640 A1, the polymers containing a unit structure represented by the following formula (18) and a unit structure represented by the following formula (19), where the ratio of the unit structure represented by the formula (18) to the unit structure represented by the formula (19) is 3 to 97 : 97 to 3 by molar ratio.

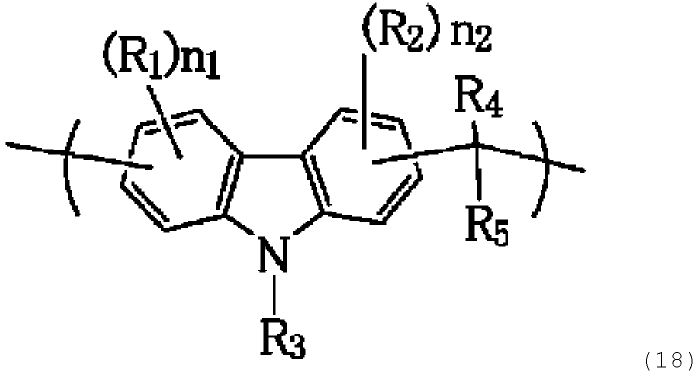

In the formula (18), R₁ and R₂ each independently represent a hydrogen atom, a halogen atom, a nitro group, an amino group, a hydroxy group, an alkyl group having 1 to 10 carbon atoms, an alkenyl group having 2 to 10 carbon atoms, an aryl group having 6 to 40 carbon atoms, or a combination of the groups, optionally including an ether bond, a ketone bond, or an ester bond. R₃ represents a hydrogen atom, an alkyl group having 1 to 10 carbon atoms, an alkenyl group having 2 to 10 carbon atoms, an aryl group having 6 to 40 carbon atoms, or a combination of the groups, optionally including an ether bond, a ketone bond, or an ester bond. R₄ represents a hydrogen atom, or an aryl group or heterocyclic group having 6 to 40 carbon atoms and optionally being substituted with a halogen atom, a nitro group, an amino group, or a hydroxy group; R₅ represents a hydrogen atom, or an alkyl group having 1 to 10 carbon atoms optionally substituted with a halogen atom, a nitro group, an amino group or a hydroxy group, aryl group having 6 to 40 carbon atoms optionally substituted with a halogen atom, a nitro group, an amino group or a hydroxy group, or heterocyclic group optionally substituted with a halogen atom, a nitro group, an amino group or a hydroxy group; and R₄ and R₅ may form a ring with each other. "n1" and "n2" each represent an integer of 1 to 3. Note that the symbols in the formula apply only in this formula.

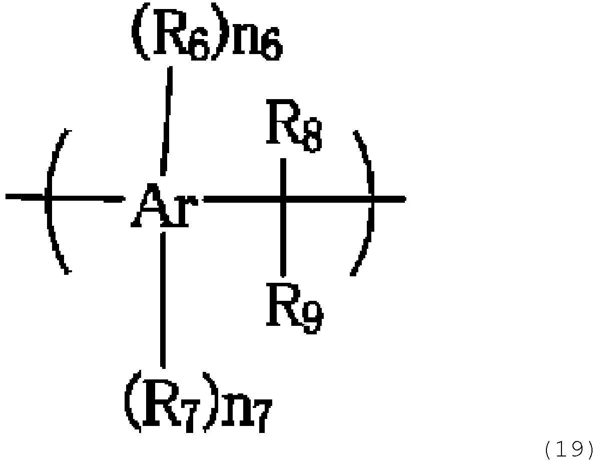

In the formula (19), Ar represents an aromatic ring group having 6 to 20 carbon atoms; R₆ represents a hydroxy group; and R₇ represents a hydrogen atom, a halogen atom, a nitro group, an amino group, an alkyl group having 1 to 10 carbon atoms, an alkenyl group having 2 to 10 carbon atoms, an aryl group having 6 to 40 carbon atoms, or a combination of the groups, optionally including an ether bond, a ketone bond, or an ester bond. R₈ represents a hydrogen atom, or an aryl group or heterocyclic group having 6 to 40 carbon atoms and optionally substituted with a halogen atom, a nitro group, an amino group, or a hydroxy group; R₉ represents a hydrogen atom, or an alkyl group having 1 to 10 carbon atoms optionally substituted with a halogen atom, a nitro group, an amino group, or a hydroxy group, an aryl group having 6 to 40 carbon atoms optionally substituted with a halogen atom, a nitro group, an amino group, or a hydroxy group, or a heterocyclic group optionally substituted with a halogen atom, a nitro group, an amino group, or a hydroxy group; and R₈ and R₉ may form a ring with each other. "n6" represents an integer of 1 to "p", "n7" represents an integer of p-n6. Here, "p" is the maximum number of substituents with which the aromatic ring group Ar can be substituted. Note that the symbols in the formula apply only in this formula.

Examples of the resin and/or compound (A) for forming an organic film used in the present invention include polymers containing a unit structure represented by the following formula (20), disclosed in WO 2010/147155 A1.

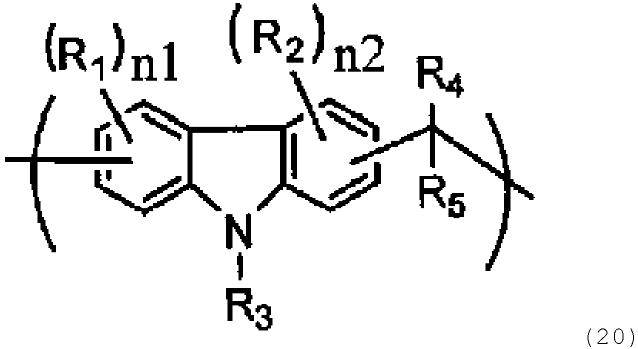

In the formula (20), R₁ and R₂ are each selected from the group consisting of a hydrogen atom, a halogen group, a nitro group, an amino group, a hydroxy group, alkyl groups having 1 to 10 carbon atoms, alkenyl groups having 2 to 10 carbon atoms, aryl groups having 6 to 40 carbon atoms, and combinations thereof, the alkyl groups, the alkenyl groups, or the aryl groups optionally containing an ether bond, a ketone bond, or an ester bond; R₃ is selected from the group consisting of a hydrogen atom, alkyl groups having 1 to 10 carbon atoms, alkenyl groups having 2 to 10 carbon atoms, aryl groups having 6 to 40 carbon atoms, and combinations thereof, the alkyl groups, the alkenyl groups, or the aryl groups optionally containing an ether bond, a ketone bond, or an ester bond; R₄ represents an aryl group or heterocyclic group having 6 to 40 carbon atoms, the aryl group or the heterocyclic group optionally substituted with a halogen group, a nitro group, an amino group, or a hydroxy group; R₅ represents a hydrogen atom or an alkyl group having 1 to 10 carbon atoms or an aryl group or heterocyclic group having 6 to 40 carbon atoms, the alkyl group, the aryl group or the heterocyclic group optionally substituted with a halogen group, a nitro group, an amino group, or a hydroxy group; R₄ and R₅ optionally form a ring together with the carbon atom bonded to R₄ and R₅; and "n1" and "n2" each represent an integer of 1 to 3. Note that the symbols in the formula apply only in this formula.

Examples of the resin and/or compound (A) for forming an organic film used in the present invention include: novolak resins obtained by a reaction between one or more phenols, such as phenol, cresol, xylenol, catechol, resorcinol, hydroquinone, pyrogallol, hydroxyquinol, and phloroglucinol, and one or more aldehyde sources, such as formaldehyde, paraformaldehyde, and trioxane, in the presence of an acid catalyst; and resins containing a repeating unit structure represented by the following formula (21), disclosed in WO 2012/176767 A1.

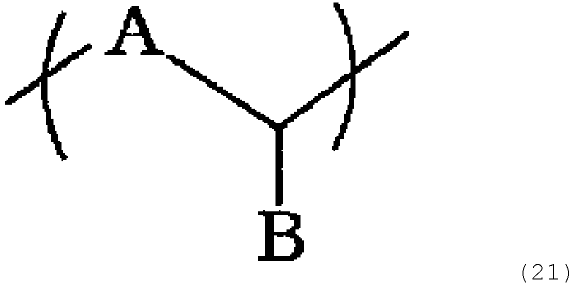

In the formula (21), A represents a hydroxy group-substituted phenylene group derived from polyhydroxybenzene; and B represents a monovalent condensed aromatic hydrocarbon ring group in which two to six benzene rings are condensed. Note that the symbols in the formula apply only in this formula.

Examples of the resin and/or compound (A) for forming an organic film used in the present invention include novolak resins having a fluorene or tetrahydrospirobiindene structure, disclosed in JP 2005-128509 A, JP 2006-259249 A, JP 2006-259482 A, JP 2006-293298 A, and JP 2007-316282 A, the novolak resins containing a repeating unit structure represented by the following formula (22-1) or (22-2).

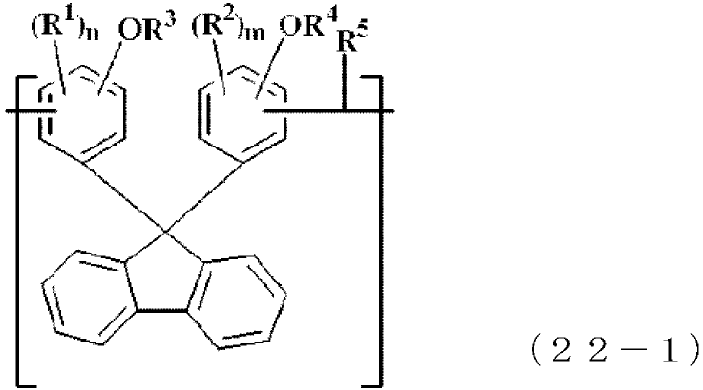

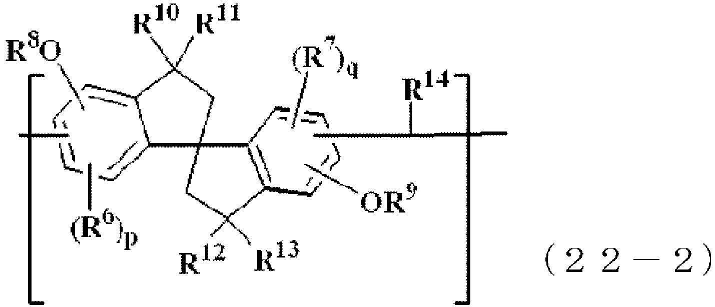

In the formula (22-1) and the formula (22-2), R¹, R², R⁶, and R⁷ each independently represent a hydrogen atom, a linear, branched, or cyclic alkyl group having 1 to 10 carbon atoms, an aryl group having 6 to 10 carbon atoms, an allyl group, or a halogen atom; R³, R⁴, R⁸, and R⁹ each independently represent a hydrogen atom, a linear, branched, or cyclic alkyl group having 1 to 6 carbon atoms, a linear, branched, or cyclic alkenyl group having 2 to 6 carbon atoms, an aryl group having 6 to 10 carbon atoms, or a glycidyl group; and R⁵ and R¹⁴ each independently represent a hydrogen atom, a linear, branched, or cyclic alkyl group having 1 to 10 carbon atoms, or an aryl group having 6 to 10 carbon atoms. "n", "m", "p", and "q" each represent an integer of 1 to 3. R¹⁰ to R¹³ each independently represent a hydrogen atom, a halogen atom, a hydroxy group, a linear, branched, or cyclic alkyl group having 1 to 6 carbon atoms, or a linear, branched, or cyclic alkoxy group having 1 to 6 carbon atoms. Note that the symbols in the formulae apply only in these formulae.

Examples of the resin and/or compound (A) for forming an organic film used in the present invention include reaction products obtained by a method disclosed in JP 2012-145897 A. More specific examples include polymers obtained by condensing one or more compounds represented by the following general formula (23-1) and/or (23-2) and one or more compounds represented by the following general formula (24-1) and/or (24-2) and/or an equivalent thereof.

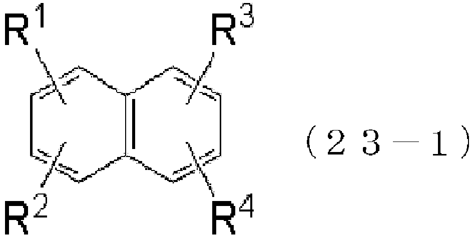

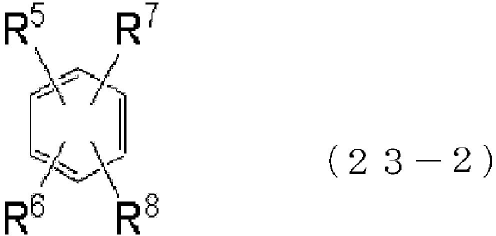

In the general formula (23-1) and the general formula (23-2), R¹ to R⁸ each independently represent a hydrogen atom, a halogen atom, a hydroxy group, an isocyanato group, a glycidyloxy group, a carboxy group, an amino group, an alkoxy group having 1 to 30 carbon atoms, an alkoxycarbonyl group having 1 to 30 carbon atoms, an alkanoyloxy group having 1 to 30 carbon atoms, or a saturated or unsaturated organic group having 1 to 30 carbon atoms and optionally being substituted. Furthermore, any two of the above-described substituents selected from each of R¹ to R⁴ or R⁵ to R⁸ may be bonded to each other within a molecule to form a cyclic substituent. Note that the symbols in the formulae apply only in these formulae.

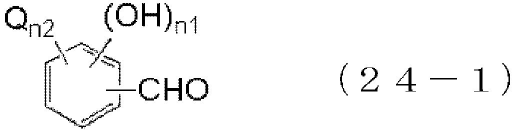

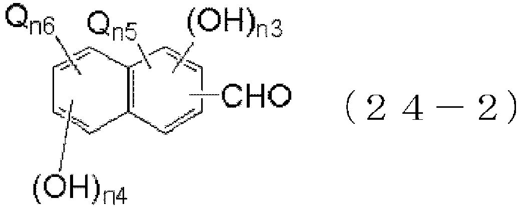

In the general formula (24-1) and the general formula (24-2), Q represents an organic group having 1 to 30 carbon atoms and optionally being substituted, and furthermore, any two Q's selected therefrom are optionally bonded with each other within a molecule to form a cyclic substituent. "n1" to "n6" each represent the number of each substituent, "n1" to "n6" are each independently 0, 1, or 2, and in the formula (24-1), hydroxybenzaldehyde is excluded. Meanwhile, in the formula (24-2), the relationships 0 ≤ n3+n5 ≤ 3, 0 ≤ n4+n6 ≤ 4, and 1 ≤ n3+n4 ≤ 4 are satisfied. Note that the symbols in the formulae apply only in these formulae.

Further examples also include polymers obtained by condensing one or more compounds represented by the general formula (23-1) and/or (23-2), one or more compounds represented by the general formula (24-1) and/or (24-2) and/or an equivalent thereof, and one or more compounds represented by the following general formula (25) and/or an equivalent thereof.

Y-CHO (25)

In the formula (25), Y represents a hydrogen atom or a monovalent organic group having 30 or fewer carbon atoms and optionally having a substituent, and the formula (25) is different from the general formula (24-1) and the general formula (24-2). Note that the symbol in the formula applies only in this formula.

Examples of the resin and/or compound (A) for forming an organic film used in the present invention include compounds containing the following structure, disclosed in JP 2017-119671 A.

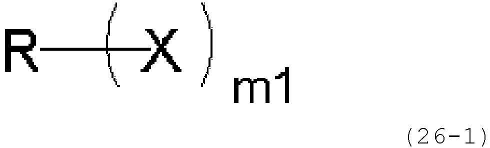

In the formula (26-1), R represents a single bond or an organic group having 1 to 50 carbon atoms; X represents a group represented by any of the following general formulae (26-2); and "m1" represents an integer that satisfies 2 ≤ m1 ≤ 10. Note that the symbols in the formula apply only in this formula.

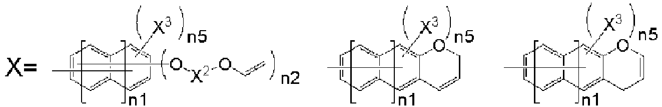

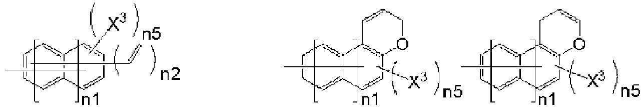

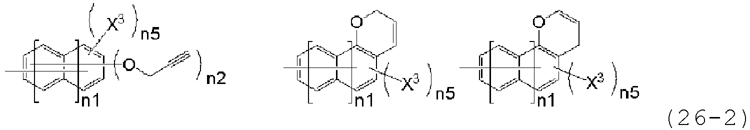

In the formulae, X² represents a divalent organic group having 1 to 10 carbon atoms; "n1" represents 0 or 1; "n2" represents 1 or 2; X³ represents a group represented by the following general formula (26-3); and "n5" represents 0, 1, or 2. Note that the symbols in the formulae apply only in these formulae.

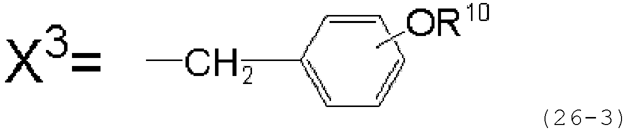

In the formula, R¹⁰ represents a hydrogen atom or a saturated or unsaturated hydrocarbon group having 1 to 10 carbon atoms, and a hydrogen atom on the benzene ring in the formula may be substituted with a methyl group or a methoxy group. Note that the symbol in the formula applies only in this formula.

Examples of the compounds containing the above-described structures include the following compounds.

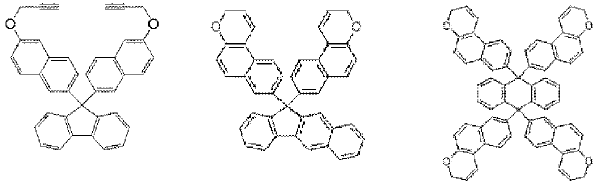

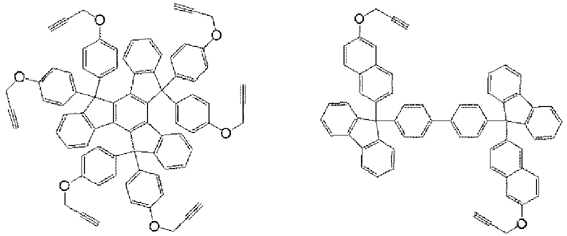

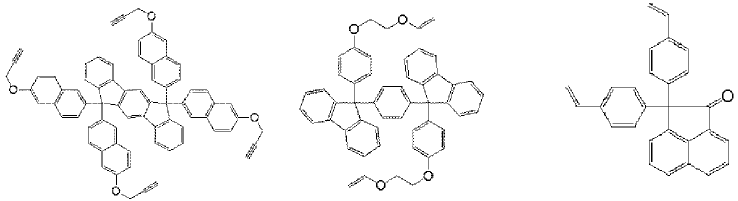

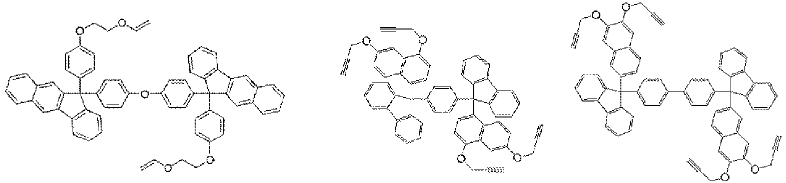

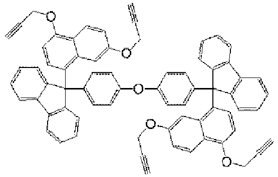

Examples of the resin and/or compound (A) for forming an organic film used in the present invention include polymers having a repeating unit represented by the following general formula (27-1), disclosed in JP 2019-044022 A.

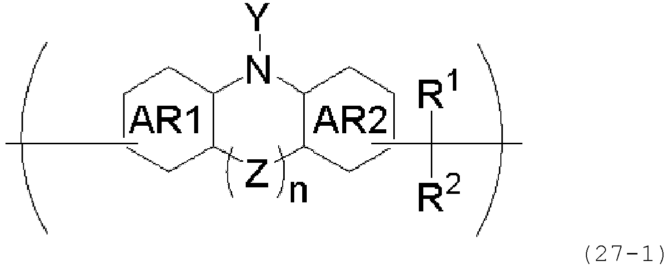

In the formula (27-1), AR1 and AR2 each represent a benzene ring or naphthalene ring optionally having a substituent. R¹ and R² each independently represent a hydrogen atom or an organic group having 1 to 30 carbon atoms, and when R¹ and R² each represent an organic group, R¹ and R² are optionally bonded to each other within a molecule to form a cyclic organic group. "n" represents 0 or 1; when n = 0, AR1 and AR2 do not form a bridged structure between the aromatic rings of AR1 and AR2 via Z; when n = 1, AR1 and AR2 form a bridged structure between the aromatic rings of AR1 and AR2 via Z; and Z represents a single bond or one of the following formulae (27-2). Y represents a group represented by the following formula (27-3). Note that the symbols in the formula apply only in this formula.

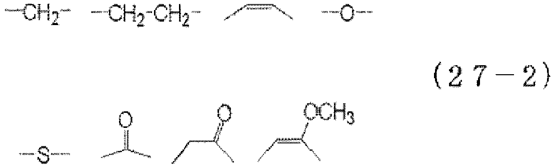

----R³-C≡C-R⁴ (27-3)

In the formula, R³ represents a single bond or a divalent organic group having 1 to 20 carbon atoms; R⁴ represents a hydrogen atom or a monovalent organic group having 1 to 20 carbon atoms; and a broken line represents an attachment point. Note that the symbols in the formula apply only in this formula.

Examples of the polymers having a repeating unit represented by the general formula (27-1) include the following polymers.

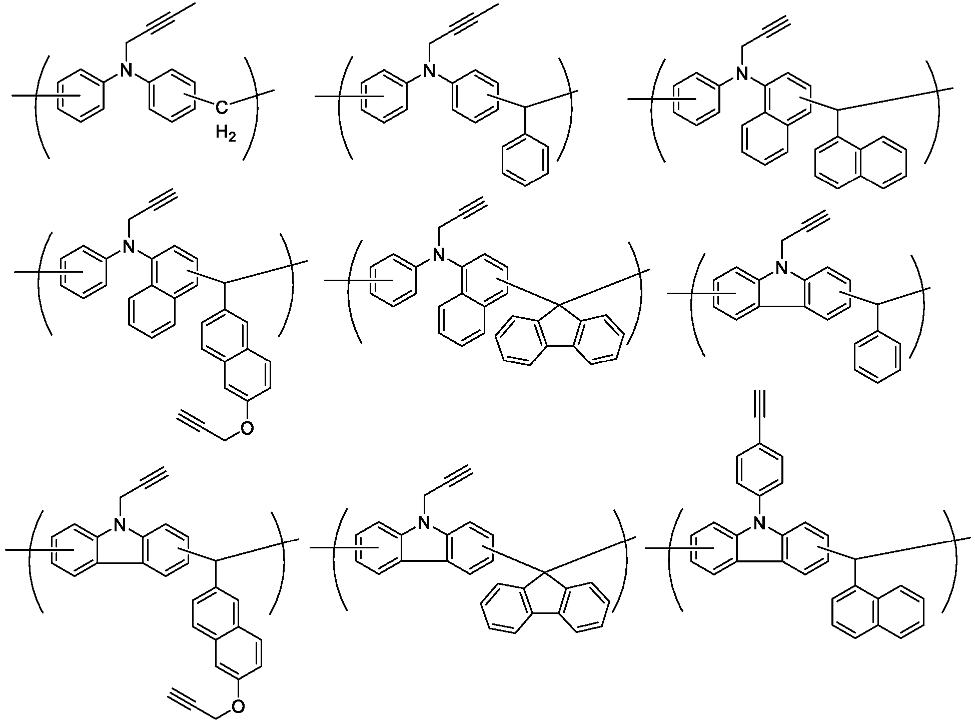

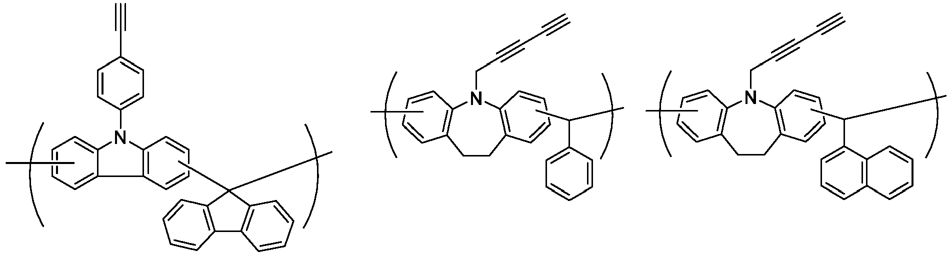

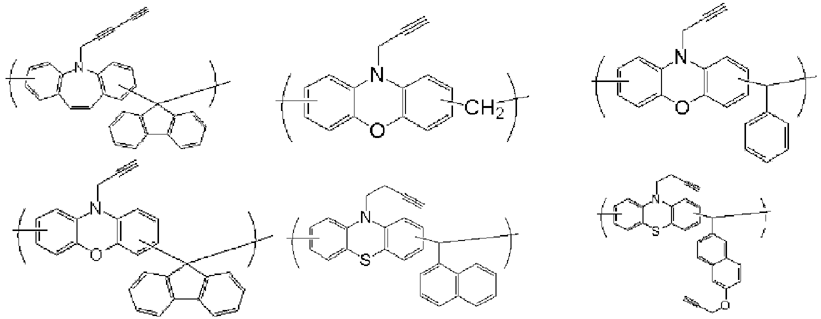

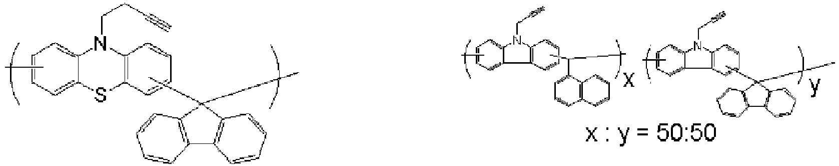

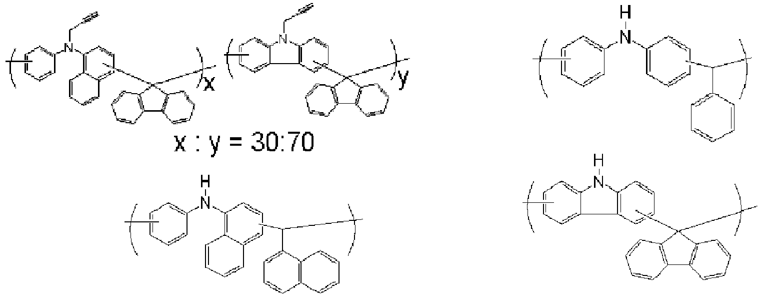

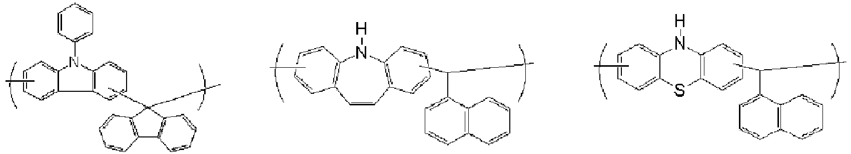

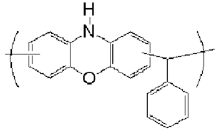

The resin and/or compound (A) for forming an organic film may be synthesized by a known method, and a commercially available product may be used.

The amount of the resin and/or compound (A) for forming an organic film to be contained is not particularly limited as long as sufficient film-formability can be achieved on spin-coating with the composition for forming an organic film, but the resin and/or compound (A) for forming an organic film is preferably contained in an amount of 10 to 40 parts by mass, more preferably 10 to 30 parts by mass, and further preferably 10 to 25 parts by mass, based on 100 parts by mass of the composition for forming an organic film. For example, when a hole or trench, having a very high aspect ratio, of a 3D NAND memory architecture is to be filled with the composition for forming an organic film, the contained amount of the resin for forming an organic film needs to be large. However, on the other hand, such a composition for forming an organic film is highly viscous, and in-plane uniformity after spin-coating and filling property are degraded. Even when the resin (A) for forming an organic film is contained in such a proportion, the inventive composition for forming an organic film makes it possible to form an organic film having excellent in-plane uniformity and excellent filling property, and can therefore be applied suitably.

Meanwhile, the compound (B) is preferably contained in an amount of 0.01 parts by mass to 10 parts by mass based on 100 parts by mass of the resin and/or compound (A) for forming an organic film. A composition for forming an organic film containing the compound in such an amount makes it possible to form an organic film with better in-plane uniformity.

### [Solvent (C)]

The solvent (C) usable for the inventive composition for forming an organic film is not particularly limited as long as the solvent can dissolve the resin and/or compound (A) for forming an organic film and the compound (B), and the solvent can preferably also dissolve the crosslinking agent, surfactant, etc. described later. Specifically, it is possible to use a solvent having a boiling point lower than 180°C, such as the solvents disclosed in paragraphs [0091] and [0092] of JP 2007-199653 A. In particular, preferable solvents are propylene glycol monomethyl ether acetate, propylene glycol monomethyl ether, 2-heptanone, cyclopentanone, cyclohexanone, and mixtures of two or more thereof.

The solvent (C) is preferably contained in an amount of 200 to 10,000 parts by mass, more preferably 300 to 5,000 parts based on 100 parts by mass of the resin and/or compound (A) for forming an organic film. When the amount is in such a range, the concentration is adjustable in accordance with the desired film thickness.

Furthermore, the inventive composition for forming an organic film may also contain, as an organic solvent, a high-boiling-point solvent, having a boiling point of 180°C or higher, in addition to the above-described solvent having a boiling point of lower than 180°C (a mixture of a solvent having a boiling point of lower than 180°C and a solvent having a boiling point of 180°C or higher). The high-boiling-point organic solvent is not particularly limited to hydrocarbons, alcohols, ketones, esters, ethers, or chlorine-based solvents as long as the solvent is capable of dissolving the compound for forming an organic film. Specific examples include 1-octanol, 2-ethylhexanol, 1-nonanol, 1-decanol, 1-undecanol, ethylene glycol, 1,2-propylene glycol, 1,3-butylene glycol, 2,4-pentanediol, 2-methyl-2,4-pentanediol, 2,5-hexanediol, 2,4-heptanediol, 2-ethyl-1,3-hexanediol, diethylene glycol, dipropylene glycol, triethylene glycol, tripropylene glycol, glycerin, n-nonyl acetate, ethylene glycol monohexyl ether, ethylene glycol mono-2-ethylhexyl ether, ethylene glycol monophenyl ether, ethylene glycol monobenzyl ether, diethylene glycol monoethyl ether, diethylene glycol monoisopropyl ether, diethylene glycol mono-n-butyl ether, diethylene glycol monoisobutyl ether, diethylene glycol monohexyl ether, diethylene glycol monophenyl ether, diethylene glycol monobenzyl ether, diethylene glycol diethyl ether, diethylene glycol dibutyl ether, diethylene glycol butyl methyl ether, triethylene glycol dimethyl ether, triethylene glycol monomethyl ether, triethylene glycol-n-butyl ether, triethylene glycol butyl methyl ether, triethylene glycol diacetate, tetraethylene glycol dimethyl ether, dipropylene glycol monomethyl ether, dipropylene glycol mono-n-propyl ether, dipropylene glycol mono-n-butyl ether, tripropylene glycol dimethyl ether, tripropylene glycol monomethyl ether, tripropylene glycol mono-n-propyl ether, tripropylene glycol mono-n-butyl ether, ethylene glycol monoethyl ether acetate, ethylene glycol monobutyl ether acetate, diethylene glycol monomethyl ether acetate, diethylene glycol monoethyl ether acetate, diethylene glycol monobutyl ether acetate, triacetin, propylene glycol diacetate, dipropylene glycol monomethyl ether acetate, dipropylene glycol methyl-n-propyl ether, dipropylene glycol methyl ether acetate, 1,4-butanediol diacetate, 1,3-butylene glycol diacetate, 1,6-hexanediol diacetate, γ-butyrolactone, dihexyl malonate, diethyl succinate, dipropyl succinate, dibutyl succinate, dihexyl succinate, dimethyl adipate, diethyl adipate, dibutyl adipate, and the like. One of the solvents may be used or a mixture of two or more kinds may be used.

The boiling point of the high-boiling-point solvent can be selected appropriately in accordance with the temperature at which the composition for forming an organic film is heated, and the boiling point of the high-boiling-point solvent to be contained is preferably 180°C to 300°C, further preferably 200°C to 300°C. When the boiling point is as described, sufficient thermal flowability can be achieved, since there is no risk of excessive evaporation rate at the baking (heating) due to the boiling point being too low. Moreover, a solvent having such a boiling point does not remain in the film without evaporating even after the baking due to the boiling point being too high. Therefore, there is no risk of the solvent adversely affecting the physical properties, such as etching resistance, of the film.

Furthermore, when the high-boiling-point solvent is used, the amount to be blended is preferably 1 to 30 parts by mass per 100 parts by mass of the solvent having a boiling point of lower than 180°C. When the contained amount is as described, and there is no risk that sufficient thermal flowability cannot be imparted at the time of baking due to the contained amount being too small, or that the solvent remains in the film and leads to degradation of physical properties, such as etching resistance, of the film due to the contained amount being too large.

The above-described composition for forming an organic film is provided with both high filling property and high planarizing property by thermal flowability being imparted to the resin and/or compound for forming an organic film by virtue of the high-boiling-point solvent being contained.

### [Other Components]

In addition, the inventive composition for forming an organic film can contain a crosslinking agent for further promoting the crosslinking reaction.

Specific examples of the crosslinking agent include those disclosed in paragraphs [0055] to [0060] of JP 2007-199653 A. One kind of the crosslinking agent may be used, or two or more kinds thereof may be used in combination. The crosslinking agent is preferably contained in an amount of 1 to 100 parts by mass, more preferably 5 to 50 parts by mass based on 100 parts by mass of the resin and/or compound (A) for forming an organic film. When the amount is as described, it is possible to enhance curability and further suppress intermixing with an upper layer film.

The inventive composition for forming an organic film can also contain a surfactant in order to further improve in-plane uniformity in spin-coating. Specific examples of the surfactant include those disclosed in paragraphs [0142] to [0147] of JP 2009-269953 A. One kind of the surfactant may be used, or two or more kinds thereof may be used in combination. When the surfactant is contained, the contained amount is preferably 0.01 to 10 parts by mass, more preferably 0.05 to 5 parts by mass based on 100 parts by mass of the resin and/or compound for forming an organic film. When the amount is as described, it is possible to form an organic film excellent in in-plane uniformity.

The inventive composition for forming an organic film can further contain a basic compound for enhancing storage stability. The basic compound serves as a quencher to an acid to prevent a very small amount of acid generated by the acid generator from promoting the crosslinking reaction. Specific examples of such a basic compound include those disclosed in paragraphs [0086] to [0090] of JP 2007-199653 A. One kind of the basic compound may be used, or two or more kinds thereof may be used in combination. When the basic compound is contained, the contained amount is preferably 0.05 to 50 parts by mass, more preferably 0.1 to 10 parts by mass based on 100 parts by mass of the resin and/or compound (A) for forming an organic film. When the amount is as described, it is possible to improve the storage stability of the composition for forming an organic film.

As described above, the inventive composition for forming an organic film is excellent in hump suppression at the time of an EBR process. Accordingly, the inventive composition for forming an organic film is extremely useful as a resist underlayer film material (organic film material) for multilayer resist processes such as two-layer resist processes, three-layer resist processes using a silicon-containing resist middle layer film or a silicon-containing inorganic hard mask middle layer film, and four-layer resist processes using a silicon-containing resist middle layer film or silicon-containing inorganic hard mask middle layer film and an organic antireflective film or adhesive film.

### [Method for Forming Organic Film]

The inventive method for forming an organic film is a method for forming an organic film to be used in a manufacturing process of a semiconductor device, the method including:
spin-coating a substrate to be processed with the inventive composition for forming an organic film described above; and
forming an organic film by heating the substrate coated with the composition for forming an organic film at a temperature of 100°C or higher and 600°C or lower for 10 to 600 seconds to cure the composition.

In this method for forming an organic film, firstly, the inventive composition for forming an organic film described above is applied onto a substrate to be processed by spin-coating. An excellent filling property can be obtained by using a spin-coating method. After removing a film on an edge portion in an EBR process, baking (heating) is performed in order to promote the crosslinking reaction. Incidentally, the solvent in the composition can be evaporated by this baking, and therefore, mixing can be prevented even when a resist upper layer film or a silicon-containing resist middle layer film is formed on the organic film.

The baking is performed at a temperature of 100°C or higher and 600°C or lower for 10 to 600 seconds, preferably at a temperature of 200°C or higher and 500°C or lower for 10 to 300 seconds. Considering influence on device damage and wafer deformation, the upper limit of the heating temperature in a wafer process of lithography is preferably 600°C or lower, more preferably 500°C or lower. By performing the heat treatment under such conditions, the crosslinking reaction can be promoted, and it is possible to form an organic film that does not mix with a film formed thereon.

### [Patterning Process]

In the following, patterning processes using the inventive composition for forming an organic film will be described.

The present invention provides a patterning process including the steps of:
forming a resist film on a substrate by using a composition for forming an organic film;
exposing the resist film to a high-energy beam; and
developing the exposed resist film by using a developer.

When the inventive composition for forming an organic film is used, the above-described exposure can be performed to form a pattern.

The high-energy beam is preferably a g-line, an i-line, a KrF excimer laser, an ArF excimer laser, ultraviolet ray, an electron beam, or extreme ultraviolet ray having a wavelength of 3 to 15 nm.

When a high-energy beam given above is used, a fine pattern can be formed.

### [Three-Layer Resist Process Using Silicon-Containing Resist Middle Layer Film]

The present invention provides a patterning process including:
forming an organic film on a body to be processed by using the above-described composition for forming an organic film;
forming a resist middle layer film on the organic film by using a resist middle layer film material containing a silicon atom;
forming a resist upper layer film on the resist middle layer film by using a resist upper layer film material containing a photoresist composition;
forming a circuit pattern in the resist upper layer film;
transferring the pattern to the resist middle layer film by etching while using the resist upper layer film having the formed circuit pattern as a mask;
transferring the pattern to the organic film by etching while using the resist middle layer film having the transferred pattern as a mask; and
further transferring the pattern to the body to be processed by etching while using the organic film having the transferred pattern as a mask.

The body to be processed is preferably a semiconductor device substrate or the semiconductor device substrate coated with any of a metal film, a metal carbide film, a metal oxide film, a metal nitride film, a metal oxycarbide film, and a metal oxynitride film. The body to be processed is not particularly limited, but specifically, may be a substrate made of Si, α-Si, p-Si, SiO₂, SiN, SiON, W, TiN, Al, etc.; the substrate coated with the above-mentioned metal film or the like as a layer to be processed; etc.

Examples of the layer to be processed include various Low-k films made of Si, SiO₂, SiON, SiN, p-Si, α-Si, W, W-Si, Al, Cu, Al-Si, or the like; and stopper films thereof. Generally, the layer can be formed to have a thickness of 50 to 10,000 nm, particularly preferably 100 to 5,000 nm. Note that when the layer to be processed is formed, the substrate and the layer to be processed are formed from different materials.

Incidentally, the metal constituting the body to be processed preferably contains silicon, titanium, tungsten, hafnium, zirconium, chromium, germanium, copper, silver, gold, aluminum, indium, gallium, arsenic, palladium, iron, tantalum, iridium, molybdenum, or an alloy thereof.

An organic film is formed on the body to be processed by using the inventive composition for forming an organic film. In this case, the inventive method for forming an organic film described above can be adopted.

Subsequently, a resist middle layer film (silicon-containing resist middle layer film) is formed on the organic film by using a resist middle layer film material containing a silicon atom. As the resist middle layer film material containing a silicon atom, a polysiloxane-based middle layer film material is preferable. By imparting an antireflective effect to the silicon-containing resist middle layer film, reflection can be suppressed. Particularly, for 193-nm light exposure, when a material containing many aromatic groups and having high etching selectivity relative to the substrate is used as a composition for forming an organic film, the k-value and thus the substrate reflection are increased; in contrast, the reflection can be suppressed by imparting absorption to the silicon-containing resist middle layer film so as to have an appropriate k-value, and the substrate reflection can be reduced to 0.5% or less. As the silicon-containing resist middle layer film having an antireflective effect, a polysiloxane is preferably used which has anthracene for 248-nm or 157-nm light exposure, or a phenyl group or a light-absorbing group having a silicon-silicon bond for 193-nm light exposure in a pendant structure, and which is to be crosslinked by an acid or heat.

Subsequently, a resist upper layer film is formed on the silicon-containing resist middle layer film by using a resist upper layer film material containing a photoresist composition. The resist upper layer film material may be a positive type or a negative type, and a photoresist composition that is normally used can be used. After the spin-coating with the resist upper layer film material, pre-baking is preferably performed at 60 to 180°C for 10 to 300 seconds. Then, light exposure, post-exposure baking (PEB), and development are performed according to conventional methods to obtain a resist upper layer film pattern. Note that the thickness of the resist upper layer film is not particularly limited, but is preferably 30 to 500 nm, particularly preferably 50 to 400 nm.

Subsequently, a circuit pattern (resist upper layer film pattern) is formed in the resist upper layer film. The circuit pattern is preferably formed by a lithography using light having a wavelength of 10 nm or more and 300 nm or less, a direct writing with electron beam, nanoimprinting, or a combination thereof.

Examples of exposure light include high-energy beams having a wavelength of 300 nm or less, specifically, deep ultraviolet ray, KrF excimer laser beam (248 nm), ArF excimer laser beam (193 nm), F₂ laser beam (157 nm), Kr₂ laser beam (146 nm), Ar₂ laser beam (126 nm), soft X-ray (EUV) of 3 to 20 nm, electron beam (EB), ion beam, X-ray, etc.

Furthermore, the circuit pattern is preferably developed with an alkaline development or an organic solvent.

Subsequently, the pattern is transferred to the silicon-containing resist middle layer film by etching while using the resist upper layer film having the formed circuit pattern as a mask. The etching of the silicon-containing resist middle layer film performed while using the resist upper layer film pattern as a mask is preferably performed by using a fluorocarbon-based gas. Thus, a silicon-containing resist middle layer film pattern is formed.

Subsequently, the pattern is transferred to the organic film by etching while using the silicon-containing resist middle layer film having the transferred pattern as a mask. The silicon-containing resist middle layer film exhibits etching resistance to oxygen gas or hydrogen gas, and therefore, the etching of the organic film performed while using the silicon-containing resist middle layer film pattern as a mask is preferably performed by using an etching gas mainly containing oxygen gas or hydrogen gas. Thus, an organic film pattern is formed.

Subsequently, the pattern is transferred to the body to be processed by etching while using the organic film having the transferred pattern as a mask. This etching of the body to be processed (layer to be processed) can be performed by a conventional method. For example, a body to be processed made of SiO₂, SiN, or silica low-dielectric insulating film is etched mainly with a fluorocarbon-based gas; and a body to be processed made of p-Si, Al, or W is etched mainly with a chlorine- or bromine-based gas. When the substrate is processed by etching with a fluorocarbon-based gas, the silicon-containing resist middle layer film pattern is removed when the substrate is processed. Meanwhile, when the substrate is processed by etching with a chlorine- or bromine-based gas, the silicon-containing resist middle layer film pattern needs to be removed by additional dry etching with a fluorocarbon-based gas after the processing of the substrate.

The organic film obtained by using the inventive composition for forming an organic film can be provided with excellent etching resistance when the body to be processed is etched as described above.

### [Four-Layer Resist Process Using Silicon-Containing Resist Middle Layer Film and Organic Antireflective Film or Adhesive Film]

The present invention also provides a patterning process including:
forming an organic film on a body to be processed by using the above-described composition for forming an organic film;
forming a resist middle layer film on the organic film by using a resist middle layer film material containing a silicon atom;
forming an organic antireflective film or an adhesive film on the resist middle layer film;
forming a resist upper layer film on the organic antireflective film or the adhesive film by using a resist upper layer film material containing a photoresist composition;
forming a circuit pattern in the resist upper layer film;
transferring the pattern to the organic antireflective film or the adhesive film and to the resist middle layer film by etching while using the resist upper layer film having the formed circuit pattern as a mask;
transferring the pattern to the organic film by etching while using the resist middle layer film having the transferred pattern as a mask; and
further transferring the pattern to the body to be processed by etching while using the organic film having the transferred pattern as a mask.

This method can be performed in the same manner as the three-layer resist process using the silicon-containing resist middle layer film, except that the organic antireflective film (BARC) or the adhesive film is formed between the silicon-containing resist middle layer film and the resist upper layer film.

The organic antireflective film and the adhesive film can be formed by spin-coating by using a known organic antireflective film material.

### [Three-Layer Resist Process Using Inorganic Hard Mask Middle Layer Film]

The present invention also provides a patterning process including:
forming an organic film on a body to be processed by using the above-described composition for forming an organic film;
forming an inorganic hard mask selected from the group consisting of a silicon oxide film, a silicon nitride film, and a silicon oxynitride film on the organic film;
forming a resist upper layer film on the inorganic hard mask by using a resist upper layer film material containing a photoresist composition;
forming a circuit pattern in the resist upper layer film;
transferring the pattern to the inorganic hard mask by etching while using the resist upper layer film having the formed circuit pattern as a mask;
transferring the pattern to the organic film by etching while using the inorganic hard mask having the transferred pattern as a mask; and
further transferring the pattern to the body to be processed by etching while using the organic film having the transferred pattern as a mask.

This method can be performed in the same manner as the three-layer resist process using the silicon-containing resist middle layer film, except that the inorganic hard mask middle layer film is formed instead of the silicon-containing resist middle layer film on the organic film.

The inorganic hard mask middle layer film selected from the group consisting of a silicon oxide film, a silicon nitride film, and a silicon oxynitride film (SiON film) can be formed by a CVD method, an ALD method, etc. Examples of methods for forming the silicon nitride film are disclosed in JP 2002-334869 A, WO 2004/066377 A1, etc. The inorganic hard mask middle layer film preferably has a thickness of 5 to 200 nm, more preferably 10 to 100 nm. As the inorganic hard mask middle layer film, a SiON film, which has a high function as an antireflective film, is the most preferably used.

### [Four-Layer Resist Process Using Inorganic Hard Mask Middle Layer Film and Organic Antireflective Film or Adhesive Film]

The present invention also provides a patterning process including:
forming an organic film on a body to be processed by using the above-described composition for forming an organic film;
forming an inorganic hard mask middle layer film selected from the group consisting of a silicon oxide film, a silicon nitride film, and a silicon oxynitride film on the organic film;
forming an organic antireflective film or an adhesive film on the inorganic hard mask middle layer film;
forming a resist upper layer film on the organic antireflective film or the adhesive film by using a resist upper layer film material containing a photoresist composition;
forming a circuit pattern in the resist upper layer film;
transferring the pattern to the organic antireflective film or the adhesive film and to the inorganic hard mask middle layer film by etching while using the resist upper layer film having the formed circuit pattern as a mask;
transferring the pattern to the organic film by etching while using the inorganic hard mask middle layer film having the transferred pattern as a mask; and
further transferring the pattern to the body to be processed by etching while using the organic film having the transferred pattern as a mask.

This method can be performed in the same manner as the three-layer resist process using the inorganic hard mask middle layer film, except that the organic antireflective film (BARC) or the adhesive film is formed between the inorganic hard mask middle layer film and the resist upper layer film.

Particularly, when a SiON film is used as the inorganic hard mask middle layer film, two antireflective films including the SiON film and the BARC make it possible to suppress the reflection even in liquid immersion exposure at a high NA exceeding 1.0. Another merit of forming the BARC is having an effect of reducing trailing of the resist upper layer film pattern immediately above the SiON film.

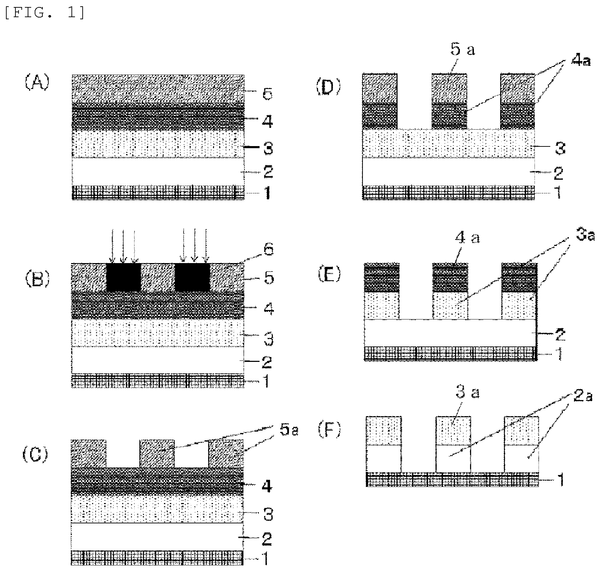

Here, an example of the inventive patterning processes according to a three-layer resist process is shown in FIG. 1 (A) to (F). In the case of a three-layer resist process, as shown in FIG. 1 (A), an organic film 3 is formed by using the inventive composition for forming an organic film on a layer 2 to be processed formed on a substrate 1; then, a silicon-containing resist middle layer film 4 is formed; and a resist upper layer film 5 is formed thereon. Next, as shown in FIG. 1 (B), an exposure portion 6 of the resist upper layer film 5 is exposed, and then PEB (post-exposure baking) is performed. Next, as shown in FIG. 1 (C), development is performed to form a resist upper layer film pattern 5a. Next, as shown in FIG. 1 (D), the silicon-containing resist middle layer film 4 is dry-etched by using a fluorocarbon-based gas while using the resist upper layer film pattern 5a as a mask, and thus, a silicon-containing resist middle layer film pattern 4a is formed. Next, as shown in FIG. 1 (E), the resist upper layer film pattern 5a is removed, then, the organic film 3 is etched with oxygen plasma while using the silicon-containing resist middle layer film pattern 4a as a mask, and thus, an organic film pattern 3a is formed. Furthermore, as shown in FIG. 1 (F), the silicon-containing resist middle layer film pattern 4a is removed, then the layer 2 to be processed is etched while using the organic film pattern 3a as a mask, and thus, a pattern 2a is formed. By humps being suppressed when the organic film is formed, during the dry etching of the drawings (D), (E), and (F), it is possible to reduce defects that are generated due to humps of the organic film.

In a case where an inorganic hard mask middle layer film is formed, the silicon-containing resist middle layer film 4 can be changed to an inorganic hard mask middle layer film, and in a case where a BARC or an adhesive film is formed, the BARC or the adhesive film can be formed between the silicon-containing resist middle layer film 4 and the resist upper layer film 5. The etching of the BARC or the adhesive film may be performed continuously before the etching of the silicon-containing resist middle layer film 4; alternatively, after the BARC or the adhesive film alone is etched, the etching apparatus may be changed and so forth, and then the etching of the silicon-containing resist middle layer film 4 may be performed.

As described above, the inventive patterning processes make it possible to form a fine pattern in a body to be processed with high precision according to a multilayer resist process, and to enhance the flatness of an organic film, thus reducing defects derived from the organic film.

### [Composition for Forming Organic Film (Resist Material)]

The inventive composition for forming an organic film can be used as a resist material. When the inventive composition for forming an organic film contains the inventive benzenesulfonic acid salt compound (B) as an acid generator, the composition may contain a surfactant. Examples of the surfactant include ones disclosed in paragraphs [0165] and [0166] of JP 2008-111103 A. When the inventive composition for forming an organic film contains a surfactant, the contained amount is not particularly limited.

### [Base Polymer]

The inventive composition for forming an organic film may further contain a base polymer. The base polymer is not particularly limited as long as it is a base polymer used in a resist material.

### (Repeating Units-a1 and -a2)

Specifically, the base polymer contains a repeating unit containing an acid-labile group in the case of a positive resist material of a chemically-amplified resist. As a repeating unit containing an acid-labile group, a repeating unit represented by the following formula (a1) (hereinafter, also referred to as repeating unit-a1) or a repeating unit represented by the following formula (a2) (hereinafter, also referred to as repeating unit-a2) is preferable.

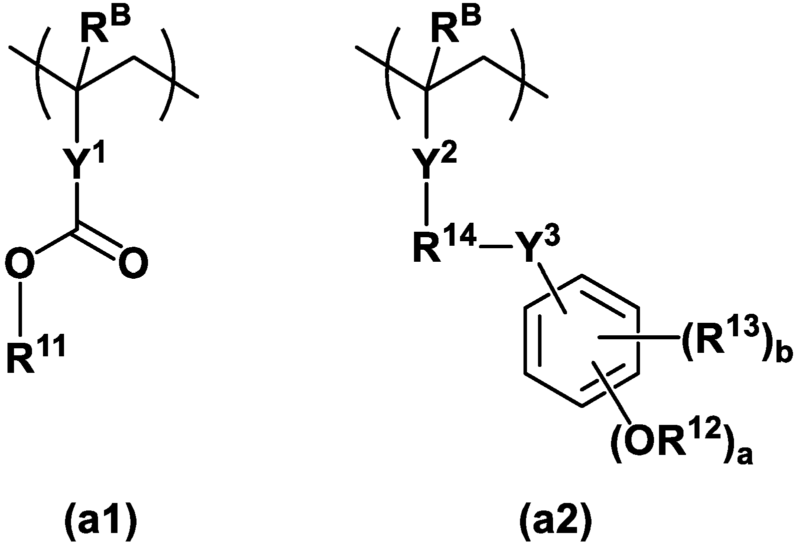

In the formulae (a1) and (a2), each R^{B} independently represents a hydrogen atom or a methyl group. Y¹ represents a single bond, a phenylene group, a naphthylene group, or a linking group having 1 to 12 carbon atoms and containing at least one selected from an ester bond, an ether bond, or a lactone ring. Y² represents a single bond or an ester bond. Y³ represents a single bond, an ether bond, or an ester bond. R¹¹ and R¹² each independently represent an acid-labile group. Incidentally, when the base polymer contains both the repeating unit-a1 and the repeating unit-a2, R¹¹ and R¹² may be identical to or different from each other. R¹³ represents a fluorine atom, a trifluoromethyl group, a cyano group, or a saturated hydrocarbyl group having 1 to 6 carbon atoms. R¹⁴ represents a single bond or an alkanediyl group having 1 to 6 carbon atoms, part of the -CH₂- of the alkanediyl group optionally being substituted with an ether bond or an ester bond. "a" represents 1 or 2 and "b" represents an integer of 0 to 4, provided that 1 ≤ a+b ≤ 5.

Specific examples of a monomer to give the repeating unit-a1 include ones shown below, but are not limited thereto. Note that, in the following formulae, R^{B} and R¹¹ are as defined above.

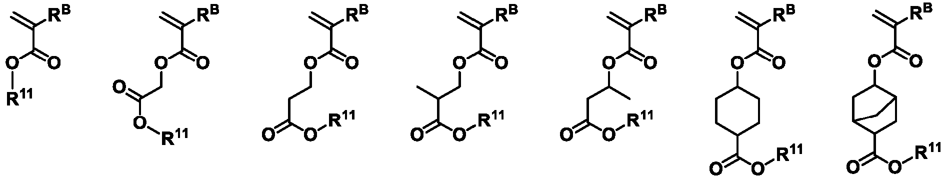

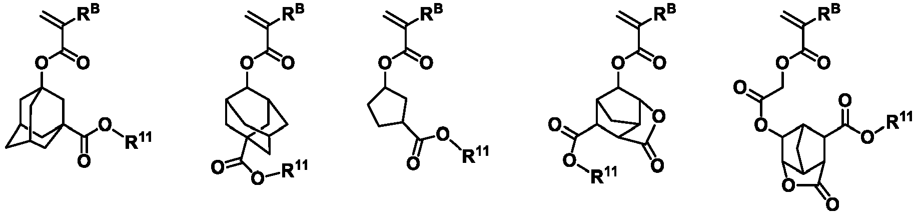

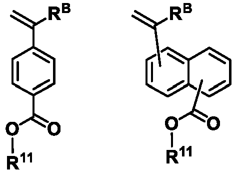

Specific examples of a monomer to give the repeating unit-a2 include ones shown below, but are not limited thereto. Note that, in the following formulae, R^{B} and R¹² are as defined above.

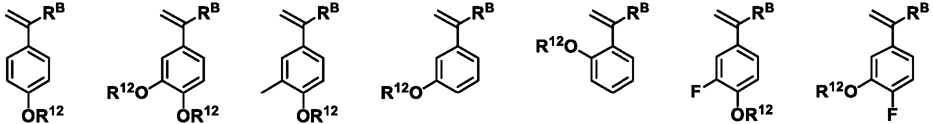

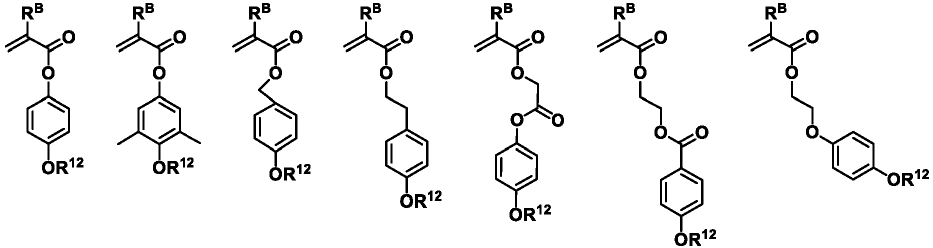

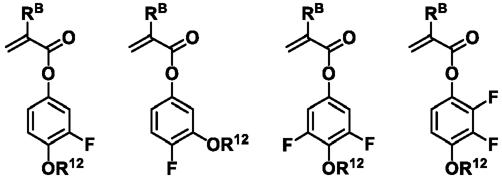

Examples of the acid-labile groups represented by R¹¹ and R¹² include those disclosed in JP 2013-80033 A and JP 2013-83821 A.

Specific examples of the acid-labile groups include those represented by any of the following formulae (AL-1) to (AL-3).

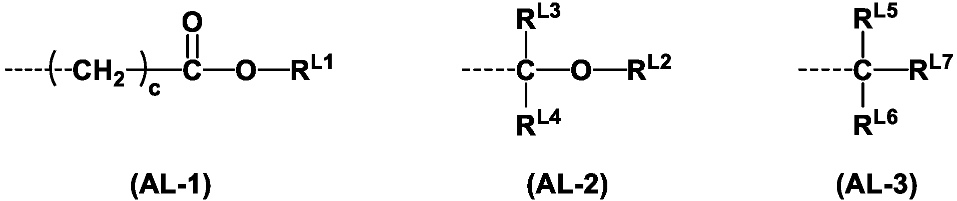

In the formulae, a broken line represents an attachment point.

In the formulae (AL-1) and (AL-2), R^{L1} and R^{L2} each independently represent a hydrocarbyl group having 1 to 40 carbon atoms, and optionally contain a heteroatom, such as an oxygen atom, a sulfur atom, a nitrogen atom, and a fluorine atom. The hydrocarbyl group may be saturated or unsaturated, and may be linear, branched, or cyclic. As the hydrocarbyl group, saturated hydrocarbyl groups having 1 to 40 carbon atoms are preferable, and saturated hydrocarbyl groups having 1 to 20 carbon atoms are more preferable.

In the formula (AL-1), "c" represents an integer of 0 to 10, preferably an integer of 1 to 5.

In the formula (AL-2), R^{L3} and R^{L4} each independently represent a hydrogen atom or a hydrocarbyl group having 1 to 20 carbon atoms, and optionally contain a heteroatom, such as an oxygen atom, a sulfur atom, a nitrogen atom, and a fluorine atom. The hydrocarbyl group may be saturated or unsaturated, and may be linear, branched, or cyclic. As the hydrocarbyl group, saturated hydrocarbyl groups having 1 to 20 carbon atoms are preferable. In addition, any two of R^{L2}, R^{L3}, and R^{L4} may be bonded to each other to form a ring having 3 to 20 carbon atoms together with the carbon atom or the carbon atom and the oxygen atom bonded thereto. The ring is preferably a ring having 4 to 16 carbon atoms, particularly preferably an aliphatic ring.

In the formula (AL-3), R^{L5}, R^{L6}, and R^{L7} each independently represent a hydrocarbyl group having 1 to 20 carbon atoms, and optionally contain a heteroatom, such as an oxygen atom, a sulfur atom, a nitrogen atom, and a fluorine atom. The hydrocarbyl group may be saturated or unsaturated, and may be linear, branched, or cyclic. As the hydrocarbyl group, saturated hydrocarbyl groups having 1 to 20 carbon atoms are preferable. In addition, any two of R^{L5}, R^{L6}, and R^{L7} may be bonded to each other to form a ring having 3 to 20 carbon atoms together with the carbon atom bonded thereto. The ring is preferably a ring having 4 to 16 carbon atoms, particularly preferably an aliphatic ring.

### (Repeating Unit-b)

The base polymer may contain a repeating unit-b containing a phenolic hydroxy group as an adhesive group. Specific examples of a monomer to give the repeating unit-b include ones shown below, but are not limited thereto. Note that, in the following formulae, R^{B} is as defined above.

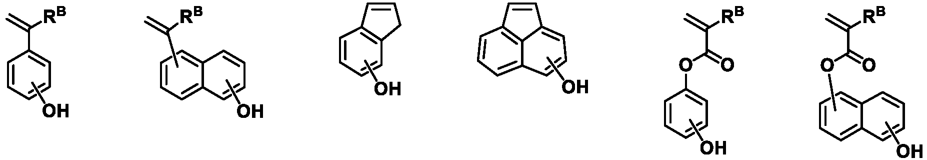

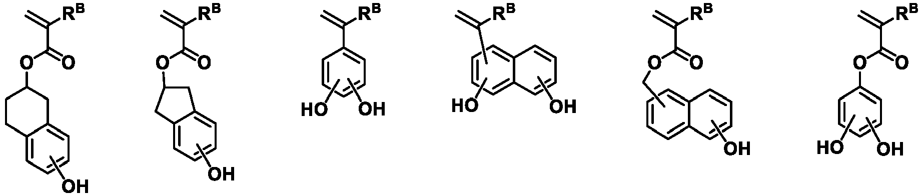

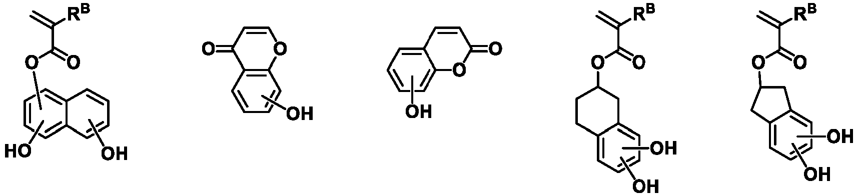

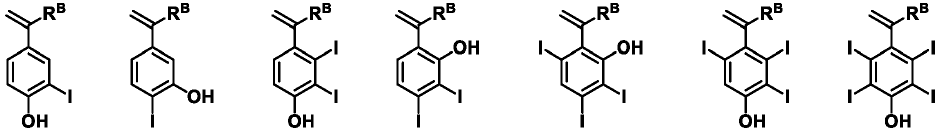

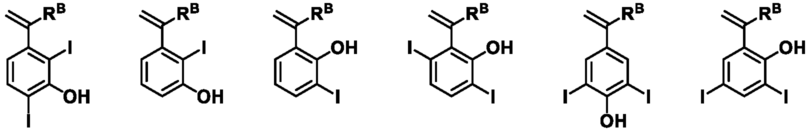

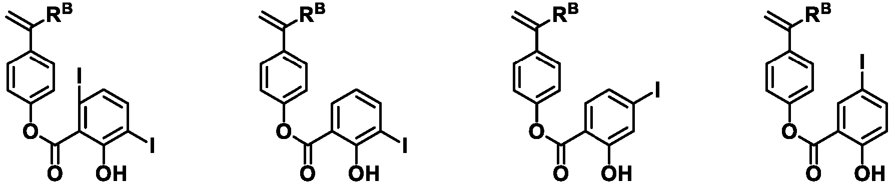

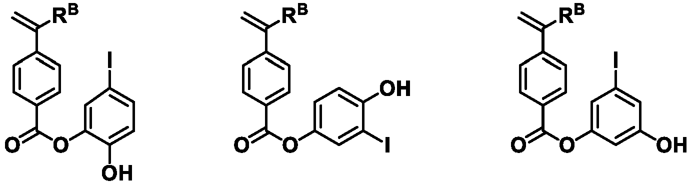

### (Repeating Unit-c)

The base polymer may contain a repeating unit-c containing, as a different adhesive group, a hydroxy group other than a phenolic hydroxy group, a lactone ring, a sultone ring, an ether bond, an ester bond, a sulfonic acid ester bond, a carbonyl group, a sulfonyl group, a cyano group, a carboxy group, or a hemiacetal structure. Specific examples of a monomer to give the repeating unit-c include ones shown below, but are not limited thereto. Note that, in the following formulae, R^{B} is as defined above.

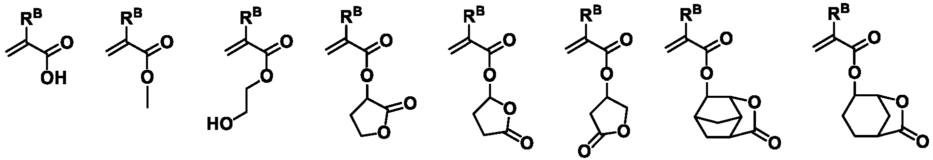

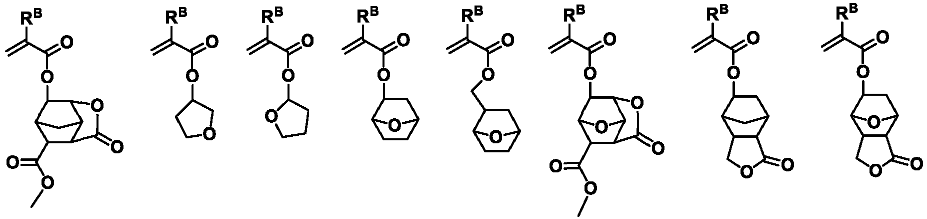

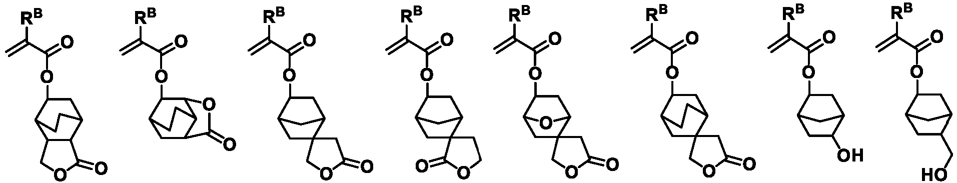

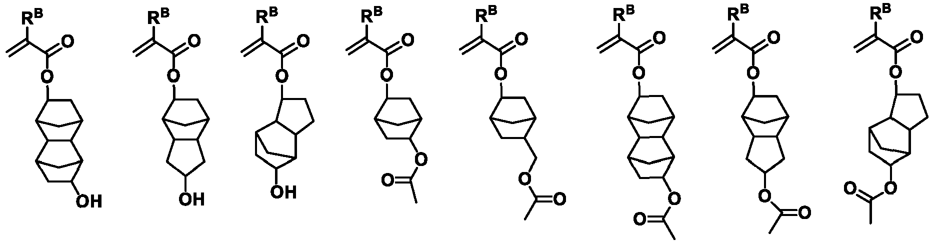

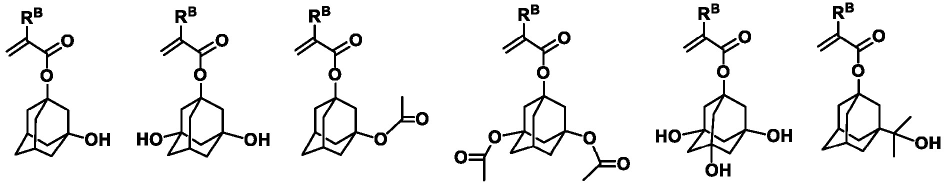

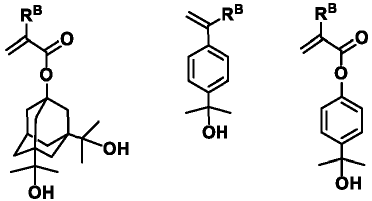

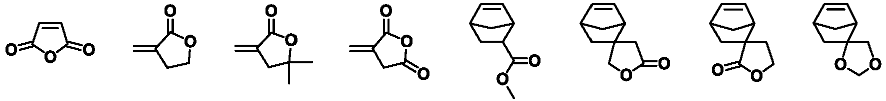

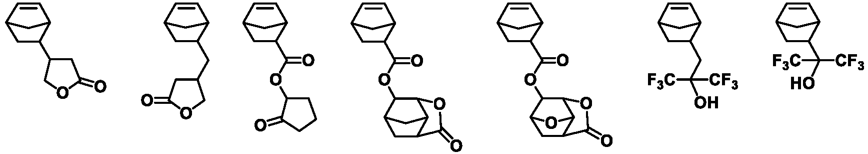

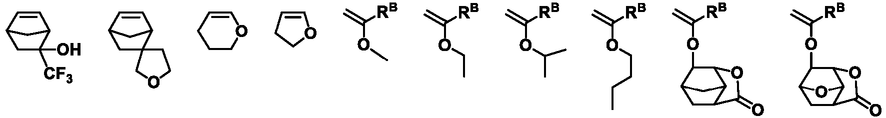

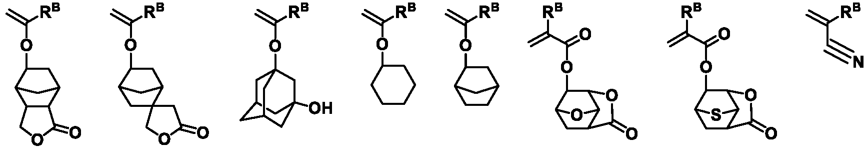

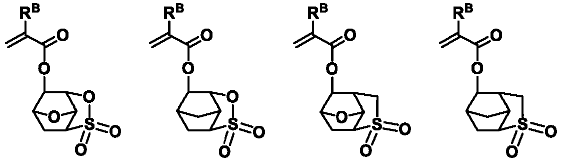

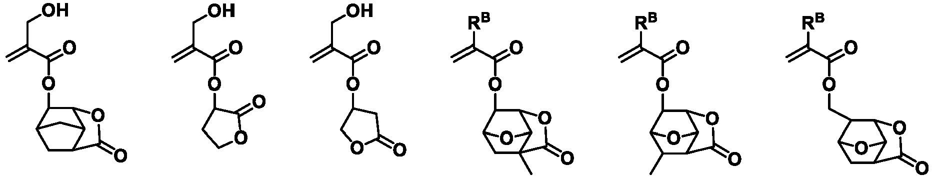

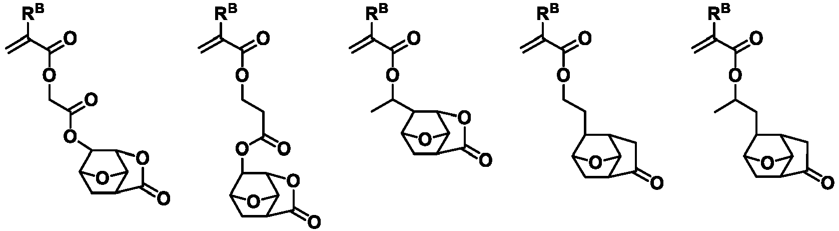

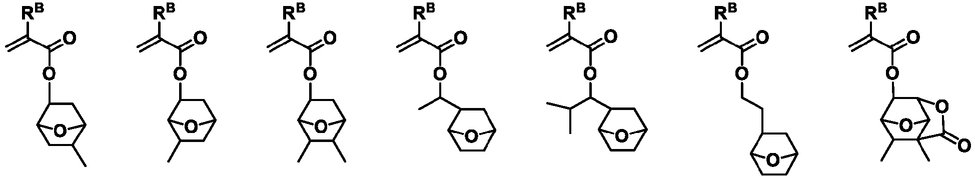

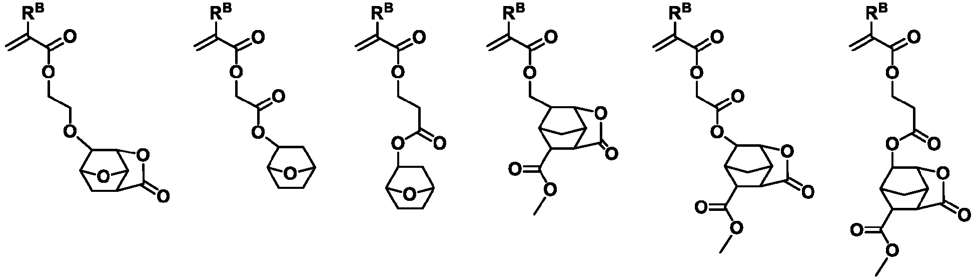

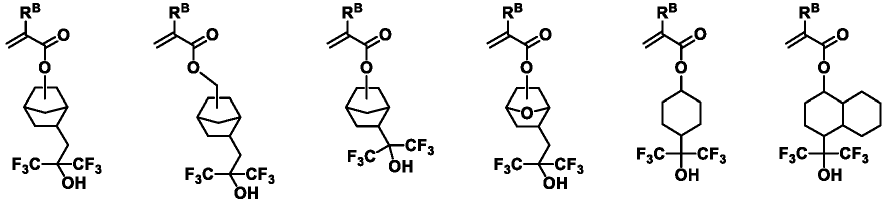

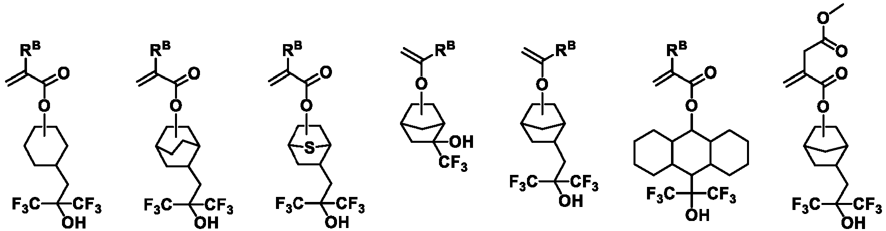

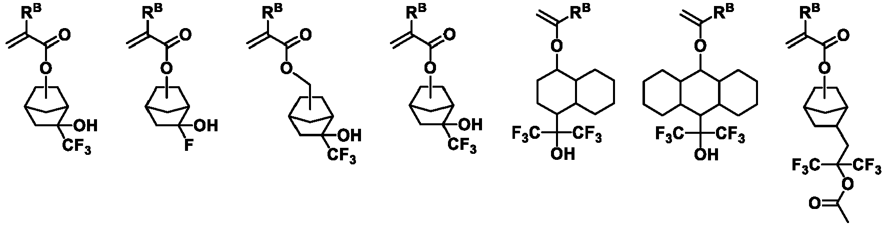

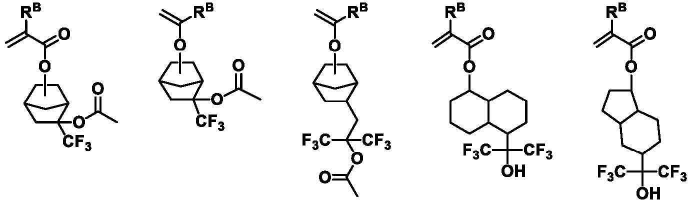

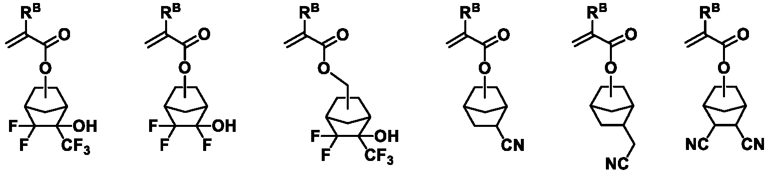

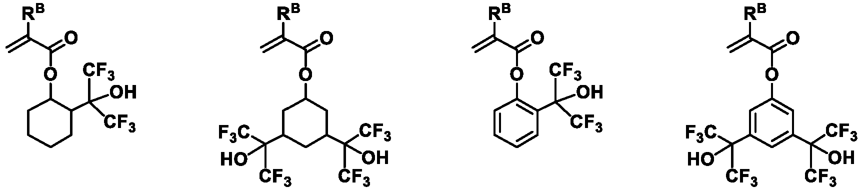

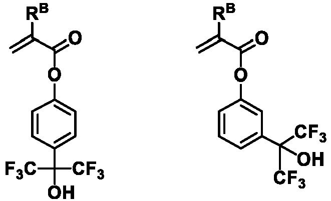

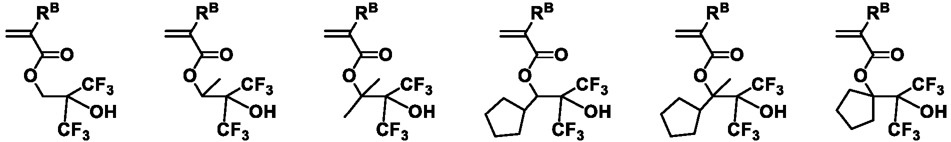

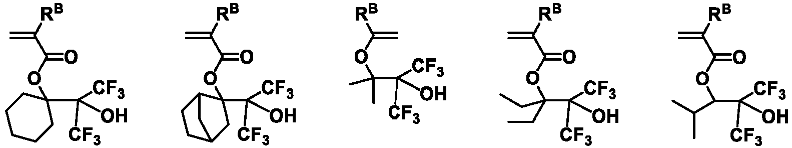

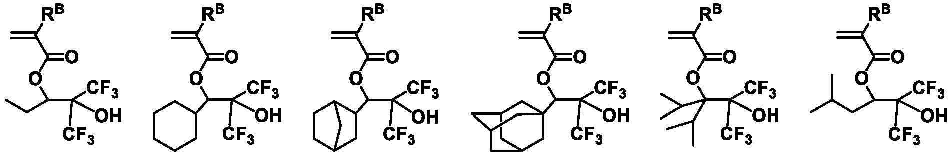

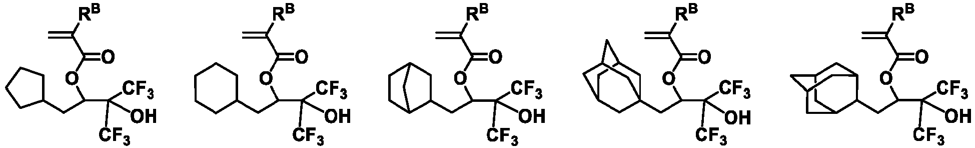

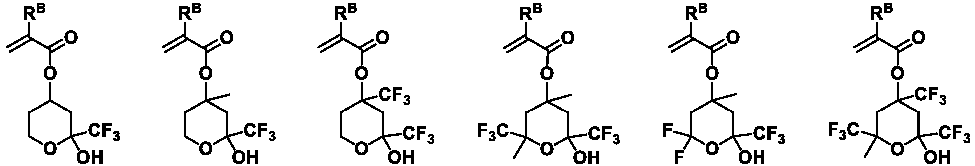

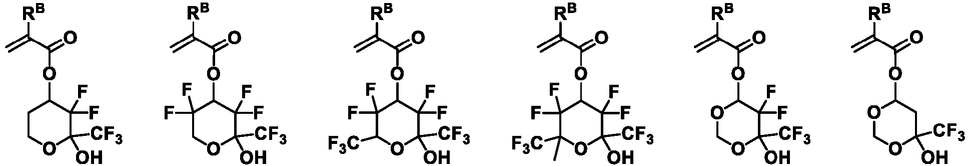

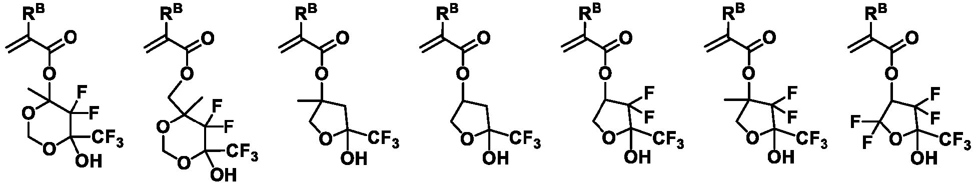

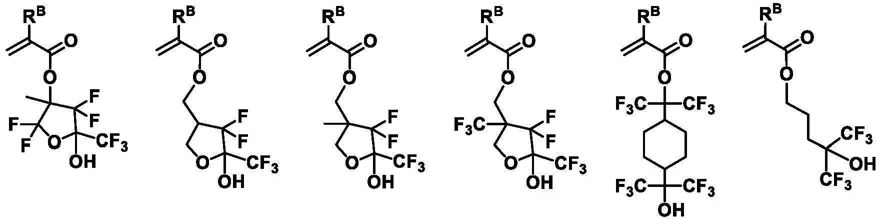

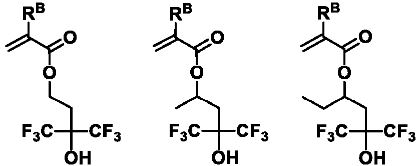

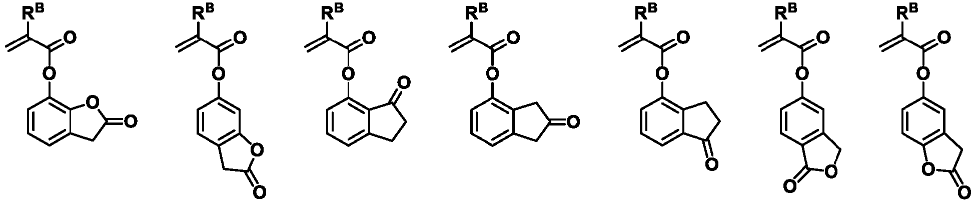

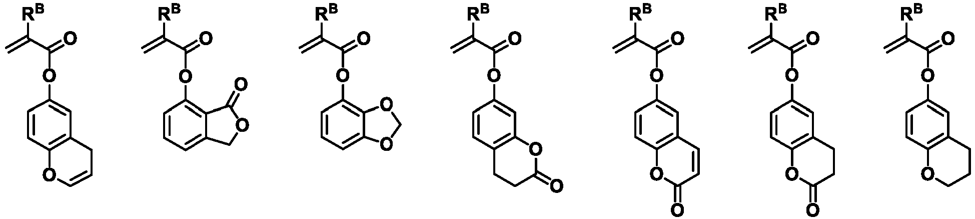

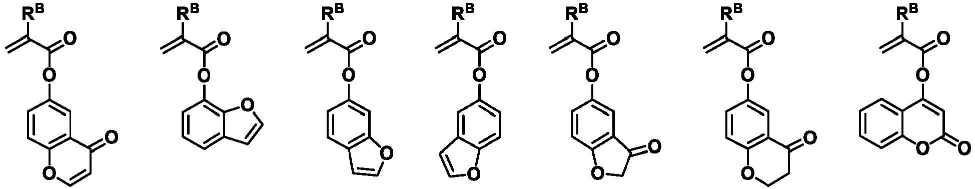

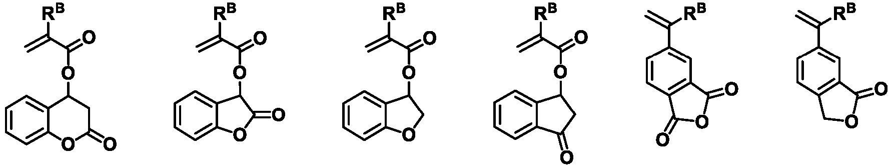

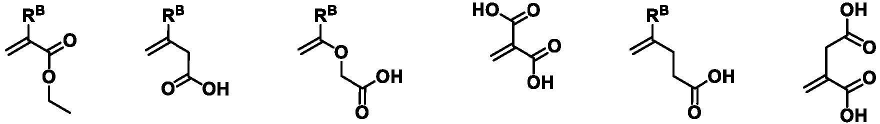

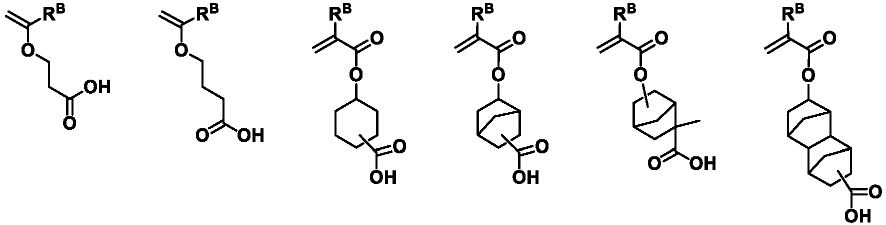

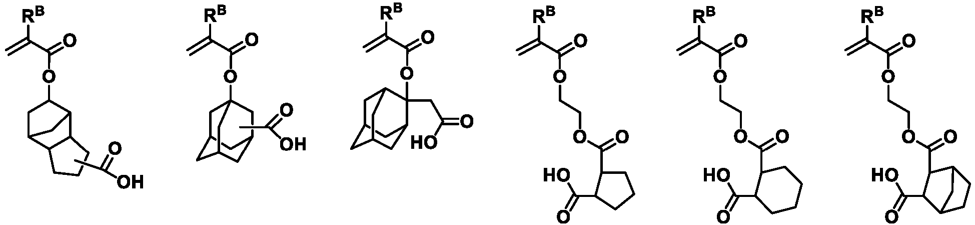

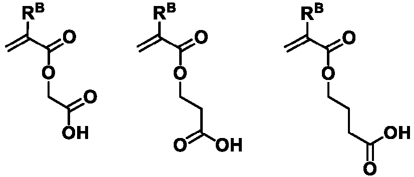

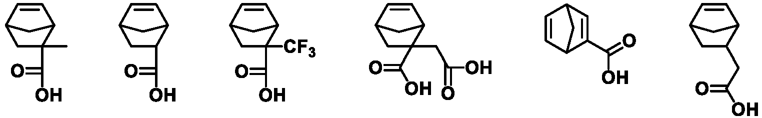

### (Repeating Unit-d)

The base polymer may contain a repeating unit-d derived from indene, benzofuran, benzothiophene, acenaphthylene, chromone, coumarin, norbornadiene, or a derivative thereof. Specific examples of a monomer to give the repeating unit-d include ones shown below, but are not limited thereto.

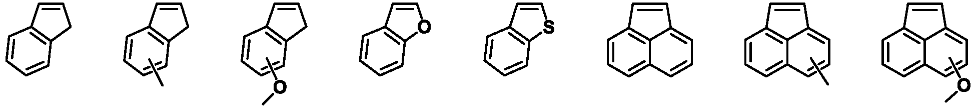

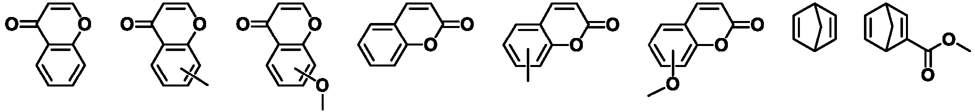

### (Repeating Unit-e)

The base polymer may contain a repeating unit-e derived from styrene, vinylnaphthalene, vinylanthracene, vinylpyrene, methyleneindan, vinylpyridine, or vinylcarbazole.

### (Repeating Unit-f)

The base polymer may contain a repeating unit-f derived from an onium salt having a polymerizable unsaturated bond. Specific examples of preferable repeating units-f include a repeating unit represented by the following formula (f1) (hereinafter, also referred to as repeating unit-f1), a repeating unit represented by the following formula (f2) (hereinafter, also referred to as repeating unit-f2), and a repeating unit represented by the following formula (f3) (hereinafter, also referred to as repeating unit-f3). Note that one kind of the repeating units-f1 to -f3 may be used, or two or more kinds thereof may be used in combination.

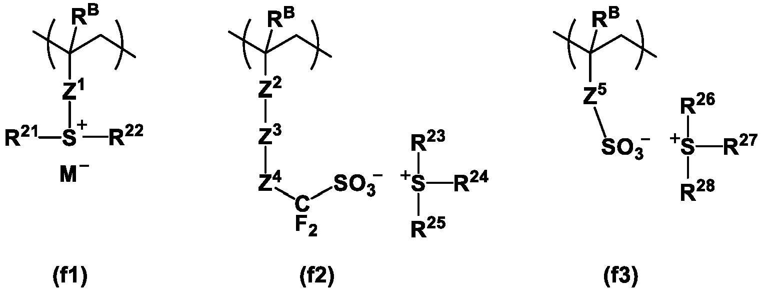

In the formulae (f1) to (f3), each R^{B} independently represents a hydrogen atom or a methyl group. Z¹ represents a single bond, an aliphatic hydrocarbylene group having 1 to 6 carbon atoms, a phenylene group, a naphthylene group, a group having 7 to 18 carbon atoms derived from a combination of these groups, -O-Z¹¹-, -C(=O)-O-Z¹¹-, or -C(=O)-NH-Z¹¹-. Z¹¹ represents an aliphatic hydrocarbylene group having 1 to 6 carbon atoms, a phenylene group, a naphthylene group, or a group having 7 to 18 carbon atoms derived from a combination of these groups, Z¹¹ optionally containing a carbonyl group, an ester bond, an ether bond, or a hydroxy group. Z² represents a single bond or an ester bond. Z³ represents a single bond, -Z³¹-C(=O)-O-, -Z³¹-O-, or -Z³¹-O-C(=O)-. Z³¹ represents an aliphatic hydrocarbylene group having 1 to 12 carbon atoms, a phenylene group, or a group having 7 to 18 carbon atoms derived from a combination of these groups, Z³¹ optionally containing a carbonyl group, an ester bond, an ether bond, an iodine atom, or a bromine atom. Z⁴ represents a methylene group, a 2,2,2-trifluoro-1,1-ethanediyl group, or a carbonyl group. Z⁵ represents a single bond, a methylene group, an ethylene group, a phenylene group, a fluorinated phenylene group, a phenylene group substituted with a trifluoromethyl group, -O-Z⁵¹-, -C(=O)-O-Z⁵¹-, or -C(=O)-NH-Z⁵¹-. Z⁵¹ represents an aliphatic hydrocarbylene group having 1 to 6 carbon atoms, a phenylene group, a fluorinated phenylene group, or a phenylene group substituted with a trifluoromethyl group, Z⁵¹ optionally containing a carbonyl group, an ester bond, an ether bond, a halogen atom, or a hydroxy group.

In the formulae (f1) to (f3), R²¹ to R²⁸ each independently represent a halogen atom or a hydrocarbyl group having 1 to 20 carbon atoms and optionally containing a heteroatom.

Specific examples of the halogen atoms represented by R²¹ to R²⁸ include a fluorine atom, a chlorine atom, a bromine atom, and an iodine atom.

The hydrocarbyl groups represented by R²¹ to R²⁸ may be saturated or unsaturated, and may be linear, branched, or cyclic. Specific examples thereof include alkyl groups having 1 to 20 carbon atoms, such as a methyl group, an ethyl group, an n-propyl group, an isopropyl group, an n-butyl group, an isobutyl group, a sec-butyl group, a tert-butyl group, an n-pentyl group, an n-hexyl group, an n-octyl group, an n-nonyl group, an n-decyl group, an undecyl group, a dodecyl group, a tridecyl group, a tetradecyl group, a pentadecyl group, a heptadecyl group, an octadecyl group, a nonadecyl group, and an icosyl group; cyclic saturated hydrocarbyl groups having 3 to 20 carbon atoms, such as a cyclopropyl group, a cyclopentyl group, a cyclohexyl group, a cyclopropylmethyl group, a 4-methylcyclohexyl group, a cyclohexylmethyl group, a norbornyl group, and an adamantyl group; alkenyl groups having 2 to 20 carbon atoms, such as a vinyl group, a propenyl group, a butenyl group, and a hexenyl group; alkynyl groups having 2 to 20 carbon atoms, such as an ethynyl group, a propynyl group, and a butynyl group; cyclic unsaturated aliphatic hydrocarbyl groups having 3 to 20 carbon atoms, such as a cyclohexenyl group and a norbornenyl group; aryl groups having 6 to 20 carbon atoms, such as a phenyl group, a methylphenyl group, an ethylphenyl group, an n-propylphenyl group, an isopropylphenyl group, an n-butylphenyl group, an isobutylphenyl group, a sec-butylphenyl group, a tert-butylphenyl group, a naphthyl group, a methylnaphthyl group, an ethylnaphthyl group, an n-propylnaphthyl group, an isopropylnaphthyl group, an n-butylnaphthyl group, an isobutylnaphthyl group, a sec-butylnaphthyl group, and a tert-butylnaphthyl group; aralkyl groups having 7 to 20 carbon atoms, such as a benzyl group and a phenethyl group; groups which are combinations of these groups; etc.

Part or all of the hydrogen atoms of the hydrocarbyl groups may be substituted with a group containing a heteroatom, such as an oxygen atom, a sulfur atom, a nitrogen atom, and a halogen atom. Part of the -CH₂- of the hydrocarbyl groups may be substituted with a group containing a heteroatom, such as an oxygen atom, a sulfur atom, and a nitrogen atom, and the resulting hydrocarbyl groups may contain a hydroxy group, a fluorine atom, a chlorine atom, a bromine atom, an iodine atom, a cyano group, a nitro group, a carbonyl group, an ether bond, an ester bond, a sulfonic acid ester bond, a carbonate group, a lactone ring, a sultone ring, a carboxylic acid anhydride (-C(=O)-O-C(=O)-), a haloalkyl group, etc.

Furthermore, R²³ and R²⁴, or R²⁶ and R²⁷ may be bonded to each other to form a ring together with the sulfur atom bonded thereto. In this case, the ring preferably has a structure shown below.

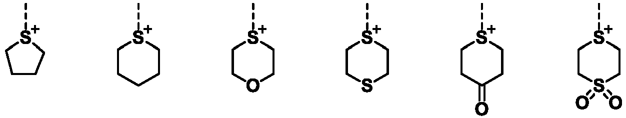

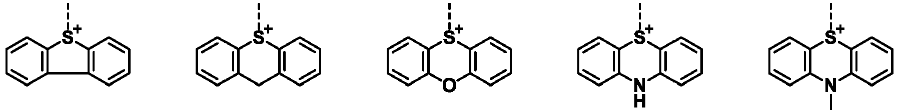

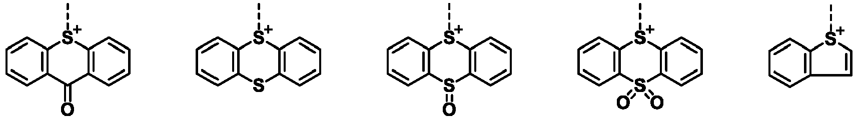

In the formulae, a broken line represents an attachment point.

In the formula (f1), M⁻ represents a non-nucleophilic counter ion. Specific examples of the non-nucleophilic counter ion include: halide ions, such as chloride and bromide ions; fluoroalkylsulfonate ions, such as triflate, 1,1,1-trifluoroethanesulfonate, and nonafluorobutanesulfonate ions; arylsulfonate ions, such as tosylate, benzenesulfonate, 4-fluorobenzenesulfonate, and 1,2,3,4,5-pentafluorobenzenesulfonate ions; alkylsulfonate ions, such as mesylate and butanesulfonate ions; imide ions, such as bis(trifluoromethylsulfonyl)imide, bis(perfluoroethylsulfonyl)imide and bis(perfluorobutylsulfonyl)imide ions; and methide ions such as tris(trifluoromethylsulfonyl)methide and tris(perfluoroethylsulfonyl)methide ions.

Specific examples of the non-nucleophilic counter ion further include: sulfonate ions represented by the following formula (f1-1), having a substituting fluorine atom at α position; sulfonate ions represented by the following formula (f1-2), having a substituting fluorine atom at α position and having a substituting trifluoromethyl group at β position; etc.

R³¹-CF₂-SO₃⁻ (f1-1)

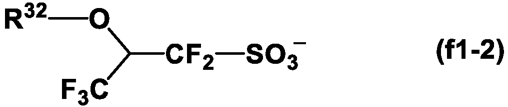

In the formula (f1-1), R³¹ represents a hydrogen atom or a hydrocarbyl group having 1 to 20 carbon atoms, the hydrocarbyl group optionally containing at least one selected from an ether bond, an ester bond, a carbonyl group, a lactone ring, and a fluorine atom. The hydrocarbyl group may be saturated or unsaturated, and may be linear, branched, or cyclic.

In the formula (f1-2), R³² represents a hydrogen atom, a hydrocarbyl group having 1 to 30 carbon atoms, or a hydrocarbylcarbonyl group having 6 to 20 carbon atoms, the hydrocarbyl group and the hydrocarbylcarbonyl group optionally containing an ether bond, an ester bond, a carbonyl group, or a lactone ring. The hydrocarbyl group and the hydrocarbyl moiety of the hydrocarbylcarbonyl group may be saturated or unsaturated, and may be linear, branched, or cyclic.

Specific examples of a cation of a monomer to give the repeating unit-f1 include ones shown below, but are not limited thereto. Note that, in the following formulae, R^{B} is as defined above.

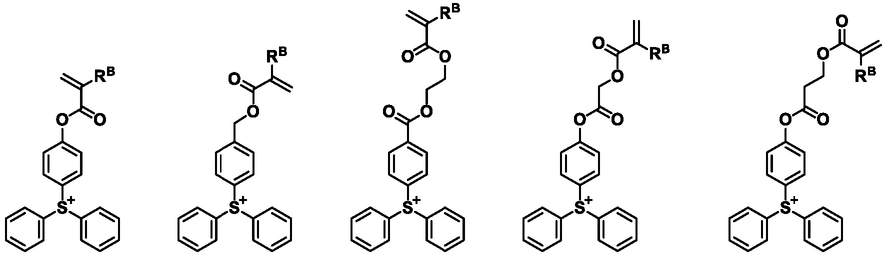

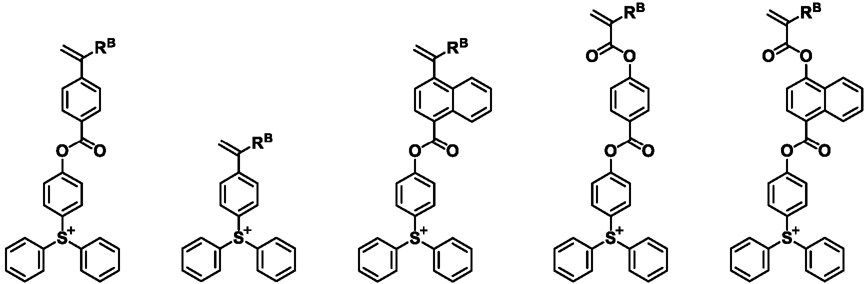

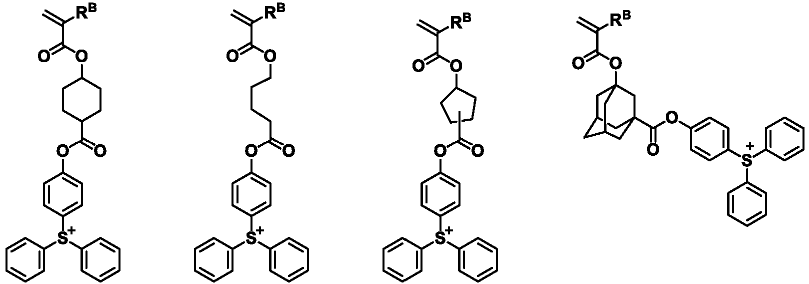

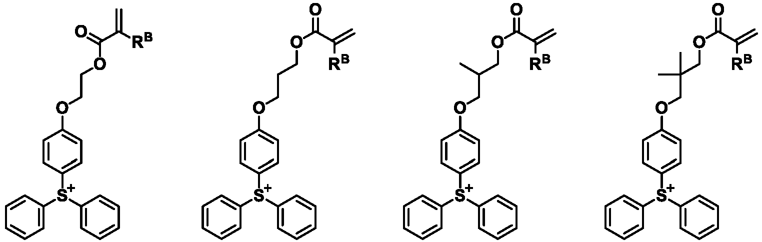

Examples of an anion of a monomer to give the repeating unit-f2 include ones shown below, but are not limited thereto. Note that, in the following formulae, R^{B} is as defined above.

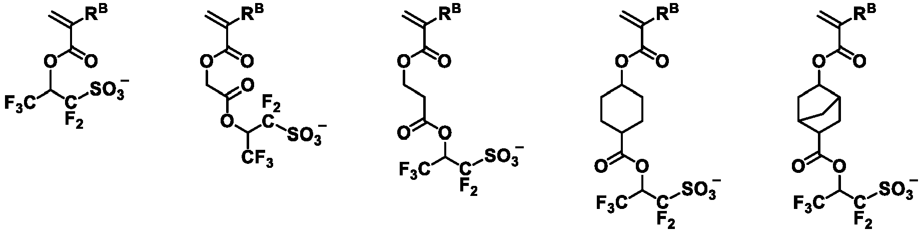

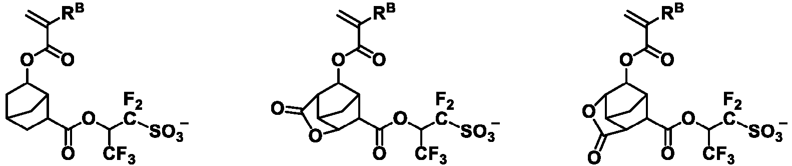

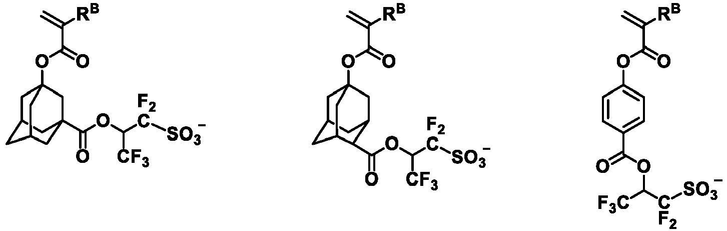

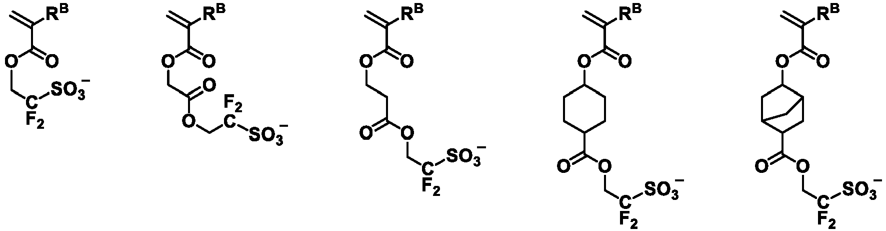

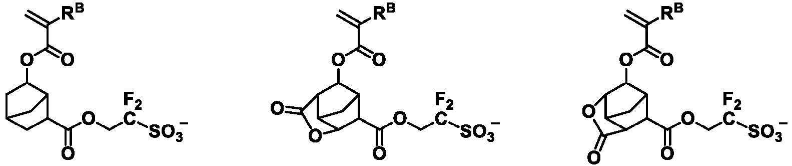

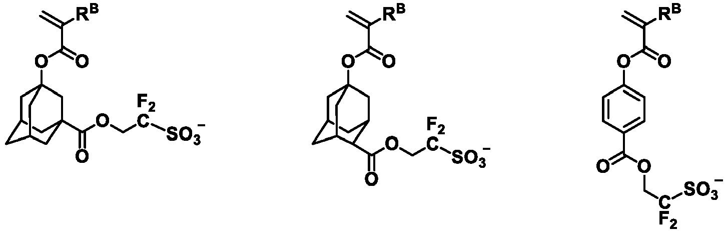

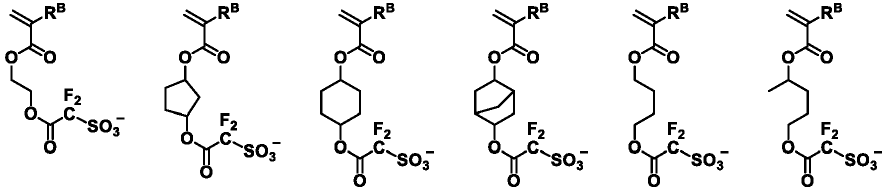

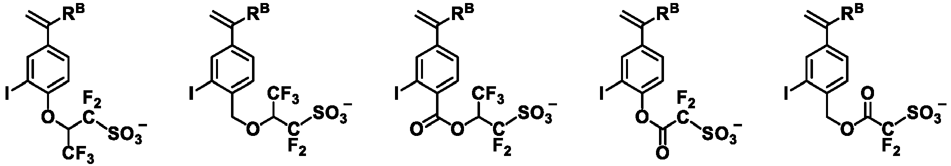

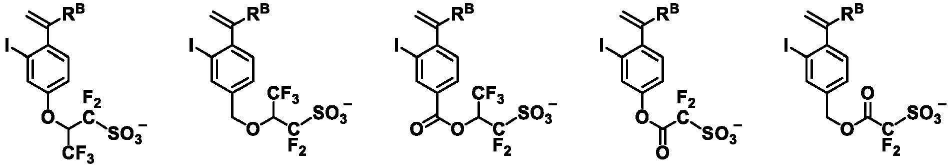

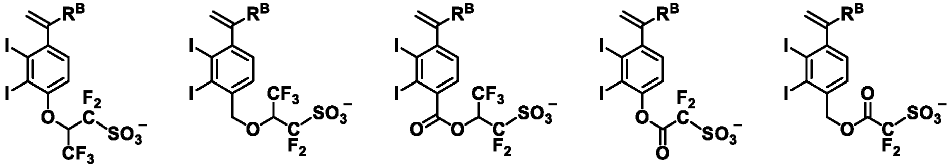

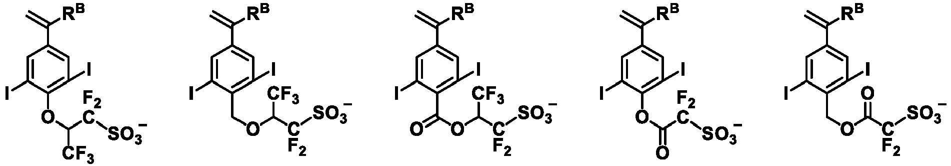

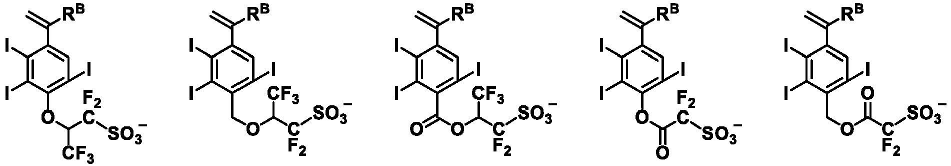

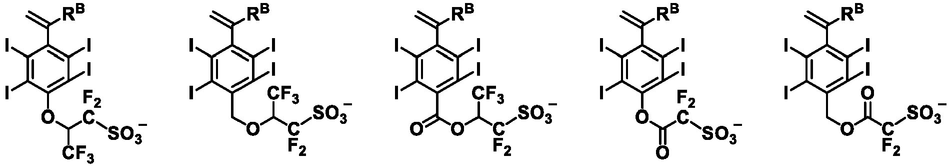

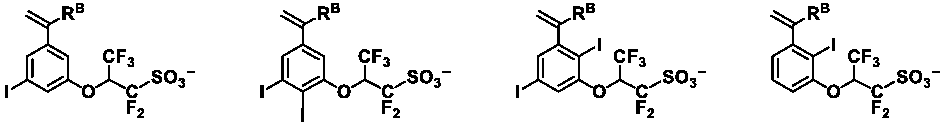

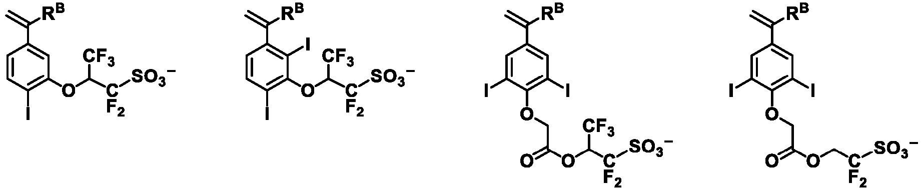

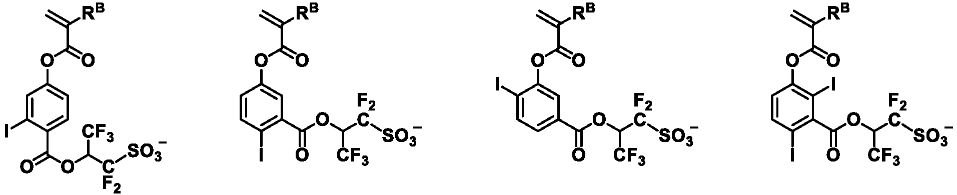

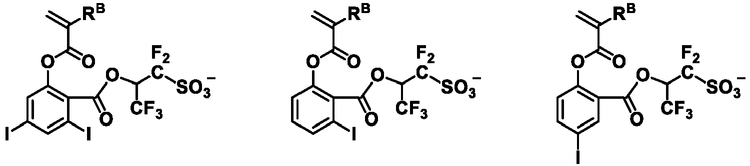

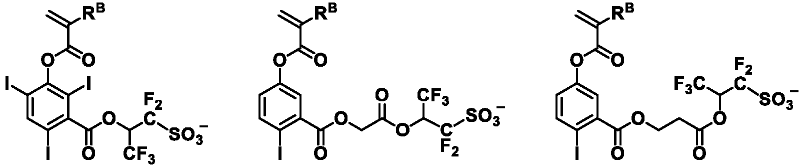

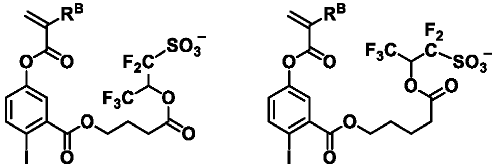

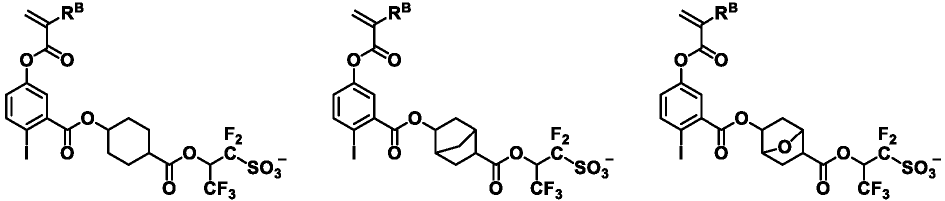

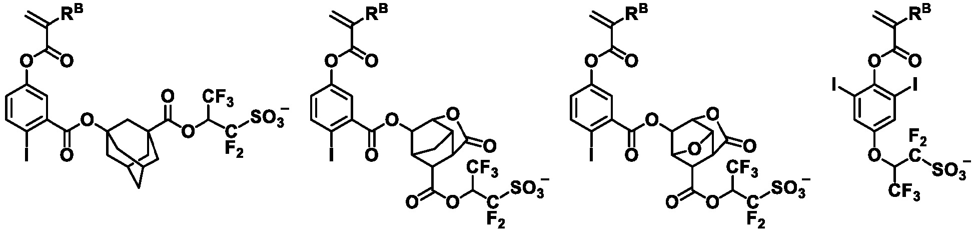

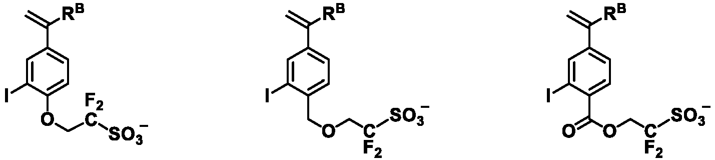

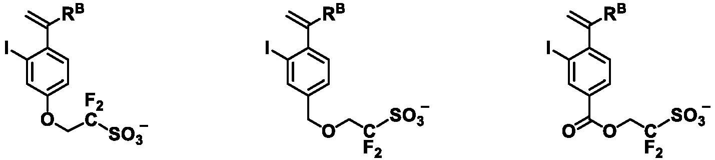

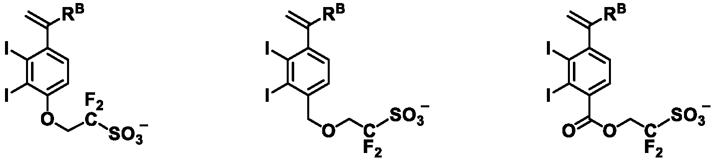

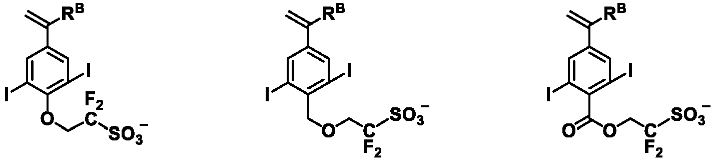

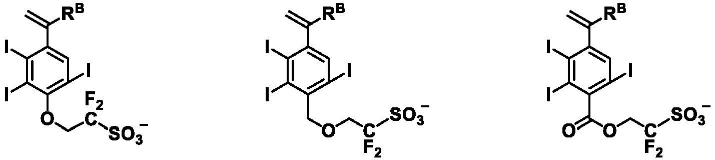

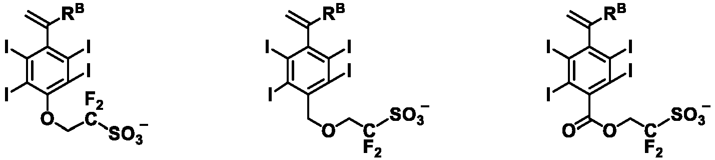

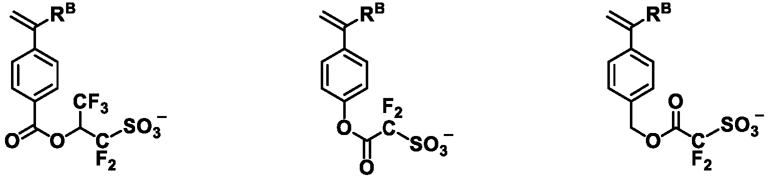

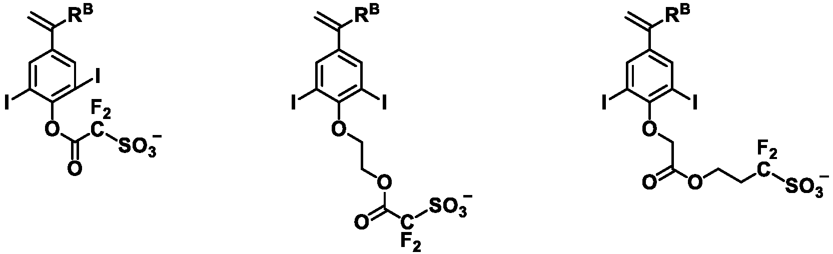

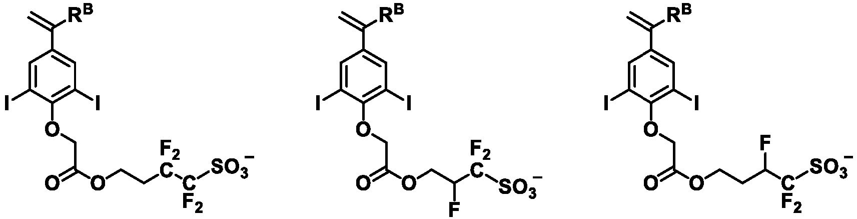

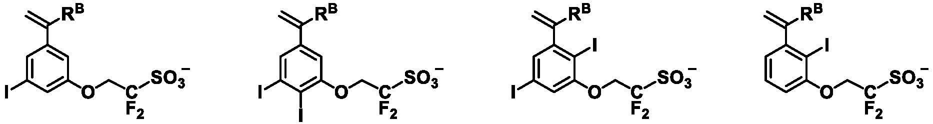

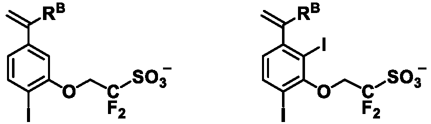

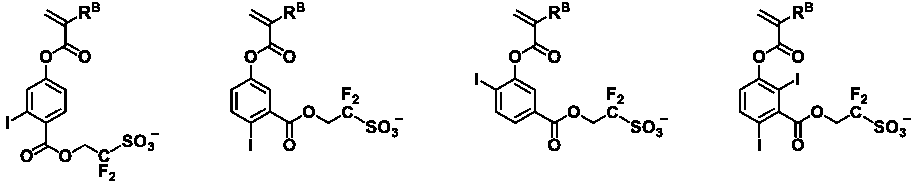

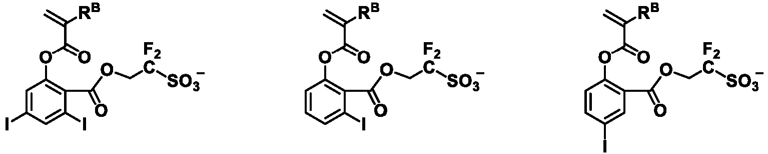

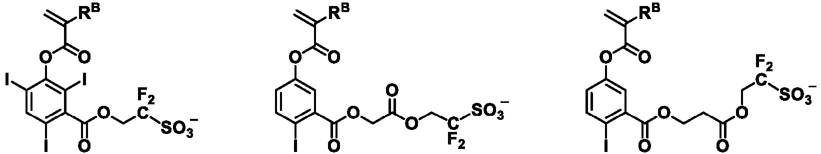

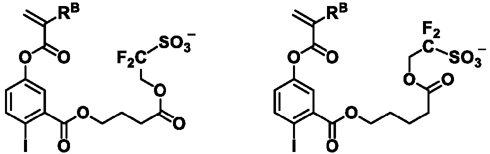

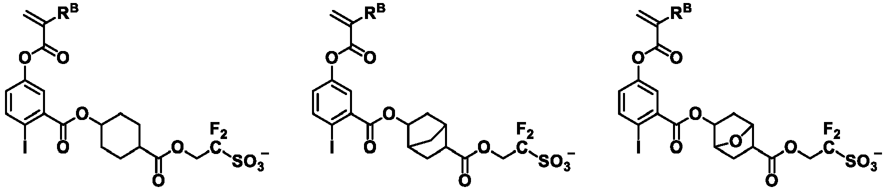

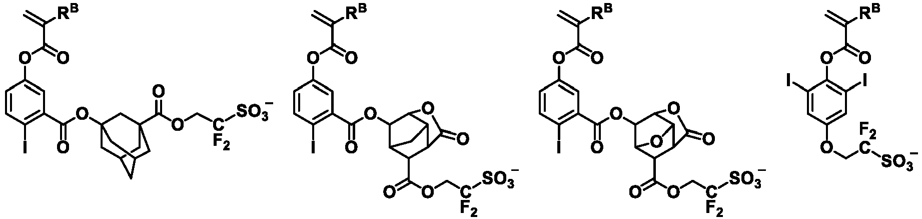

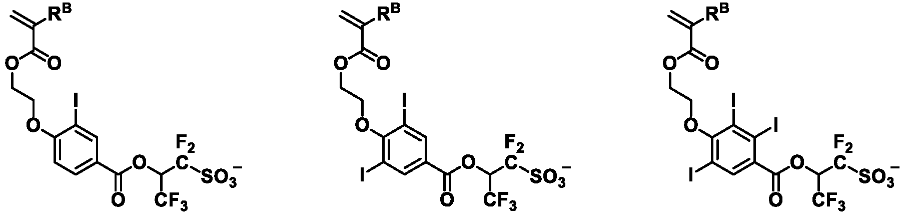

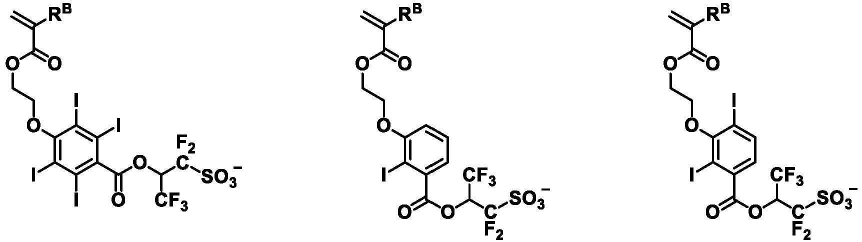

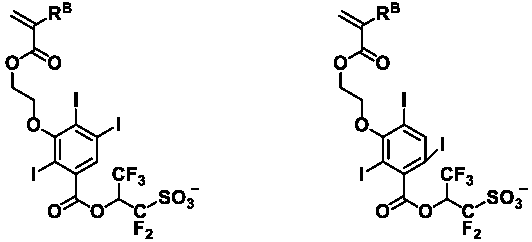

Examples of an anion of a monomer to give the repeating unit-f3 include ones shown below, but are not limited thereto. Note that, in the following formulae, R^{B} is as defined above.

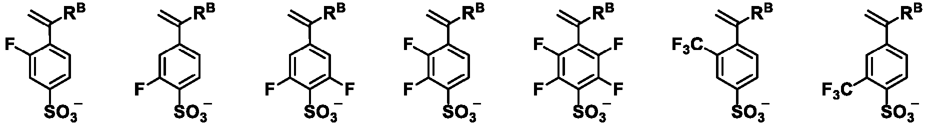

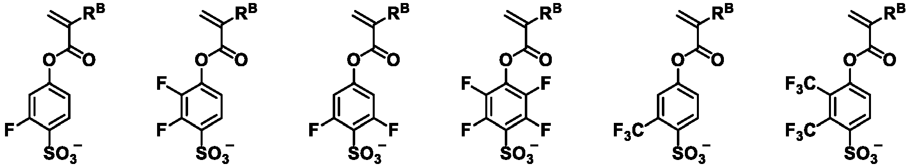

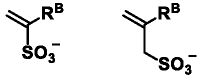

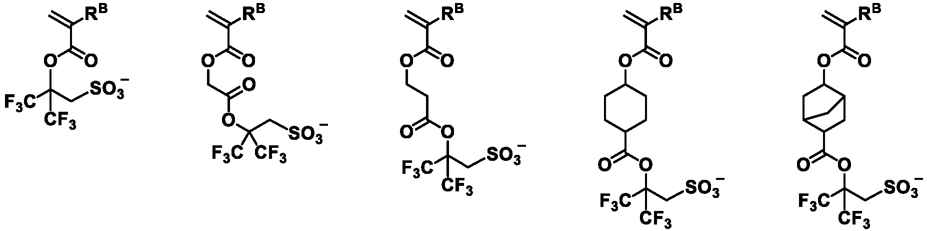

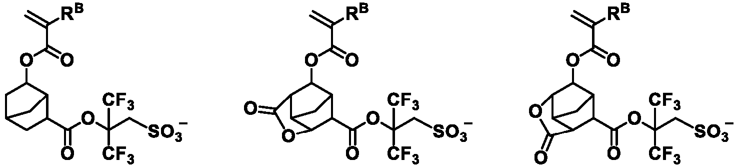

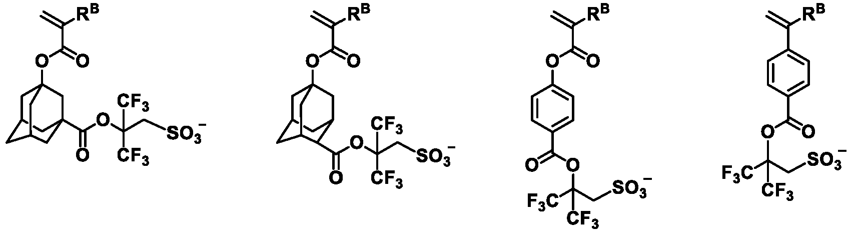

Specific examples of the cation of the repeating unit-f2 or -f3 include the sulfonium salts disclosed in JP 2023-3926 A in paragraphs [0063] to [0080] and the iodonium salts disclosed in paragraph [0082].

The repeating units-f1 to -f3 function as acid generators. Acid diffusion is reduced by the acid generator being bonded to the polymer main chain, and it is possible to prevent the degradation of resolution due to blurring caused by acid diffusion. In addition, LWR and CDU can be improved by the acid generator being dispersed uniformly. Note that, when a base polymer containing a repeating unit-f is used, the blending of the additive-type acid generator described later may be omitted.

In the case of a negative resist material or a non-chemically amplified resist material, the repeating units-a1 and -a2 are not necessarily needed in the base polymer.

As the base polymer, it is possible to use, besides the above-described polymers, a resin such as novolak resin, polyimide, polyamide, polyvinylpyrrolidone, polyvinyl alcohol, polythiophene, and polyaniline.

### [Organic Solvent]

When the inventive composition for forming an organic film is used as a resist material, the composition may contain an organic solvent. This organic solvent is not particularly limited, as long as it is capable of dissolving the above-described components and components described below. Specific examples of the organic solvent include ones disclosed in paragraphs [0144] and [0145] of JP 2008-111103 A: ketones, such as cyclohexanone, cyclopentanone, methyl-2-n-pentyl ketone, and 2-heptanone; alcohols, such as 3-methoxybutanol, 3-methyl-3-methoxybutanol, 1-methoxy-2-propanol, 1-ethoxy-2-propanol, and diacetone alcohol; ethers, such as propylene glycol monomethyl ether, ethylene glycol monomethyl ether, propylene glycol monoethyl ether, ethylene glycol monoethyl ether, propylene glycol dimethyl ether, diethylene glycol dimethyl ether, and anisole; esters, such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; lactones, such as γ-butyrolactone; lactams, such as pyrrolidone, N-methylpyrrolidone, N-ethylpyrrolidone, and N-hydroxyethyl-2-pyrrolidone; water; dimethylsulfoxide; etc.

In the resist material of the present invention, the organic solvent is preferably contained in an amount of 100 to 10000 parts by mass, more preferably 200 to 8000 parts by mass based on 100 parts by mass of the base polymer. One kind of the organic solvent may be used, or two or more kinds thereof may be used in mixture.

The resist material of the present invention may contain an additive-type acid generator. Examples of the additive-type acid generator include sulfonium salts or iodonium salts disclosed in paragraphs [0122] to [0142] of JP 2008-111103 A and paragraphs [0077] to [0148] of JP 2023-62677 A, sulfonium diazomethane, N-sulfonyloxyimide, and oxime-O-sulfonate. When the resist material of the present invention contains the additive-type acid generator, the contained amount is preferably 0.1 to 50 parts by mass, more preferably 1 to 40 parts by mass based on 100 parts by mass of the base polymer. One kind of the additive-type acid generator may be used, or two or more kinds thereof may be used in combination.

### [Quencher]

The resist material of the present invention may contain a quencher. Note that a quencher means a compound that is capable of preventing, by trapping the acid, an acid that is generated from an acid generator in a resist material from diffusing to unexposed portions.

Examples of the quencher include conventional basic compounds. Examples of the conventional basic compounds include primary, secondary, and tertiary aliphatic amines, mixed amines, aromatic amines, heterocyclic amines, nitrogen-containing compounds having a carboxy group, nitrogen-containing compounds having a sulfonyl group, nitrogen-containing compounds having a hydroxy group, nitrogen-containing compounds having a hydroxyphenyl group, alcoholic nitrogen-containing compounds, amides, imides, carbamates, etc. Particularly preferable are primary, secondary, and tertiary amine compounds disclosed in paragraphs [0146] to [0164] of JP 2008-111103 A; especially amine compounds having a hydroxy group, an ether bond, an ester bond, a lactone ring, a cyano group, or a sulfonic acid ester bond; compounds having a carbamate group disclosed in JP 3790649 B2; etc. Adding such a basic compound can, for example, further suppress the acid diffusion rate in the resist film and correct the shape.

Other examples of the quencher include onium salts, such as sulfonium salts, iodonium salts, and ammonium salts of carboxylic acids and sulfonic acids which are not fluorinated at α position as disclosed in JP 2008-158339 A. While α-fluorinated sulfonic acid, imide acid, or methide acid is necessary to deprotect the acid-labile group of carboxylic acid ester, carboxylic acid or sulfonic acid not fluorinated at α position is released by salt exchange with the onium salt not fluorinated at α position. Such a carboxylic acid and sulfonic acid not fluorinated at α position hardly induce a deprotection reaction, and thus function as quenchers.

Further examples of the quencher include polymer-type quenchers disclosed in JP 2008-239918 A. The quenchers enhance the rectangularity of a resist pattern by being oriented on the resist film surface. The polymer-type quencher also has effects of preventing rounding of a pattern top and film thickness loss of a pattern when a top coat for immersion exposure is applied.

When the inventive resist material contains the quencher, the contained amount is preferably 0 to 5 parts by mass, more preferably 0 to 4 parts by mass based on 100 parts by mass of the base polymer. One kind of the quencher may be used, or two or more kinds thereof may be used in combination.

### [Crosslinking Agent]

The resist material of the present invention may contain a crosslinking agent in the case of a negative resist. When a crosslinking agent is contained, the difference between the dissolution rates in exposed portions and unexposed portions can be further increased. Specific examples of the crosslinking agent include those disclosed in paragraphs [0170] to [0177] of JP 2020-027297 A**.** When the resist material of the present invention contains the crosslinking agent, the contained amount is preferably 0 to 30 parts by mass, more preferably 0 to 20 parts by mass based on 100 parts by mass of the base polymer. When the contained amount is in these ranges, there is no risk of the swelling in the developer increasing, and therefore, such ranges are suitable.

The resist material including the inventive composition for forming an organic film may contain a water-repellency enhancer for improving the water-repellency of the resist film surface. The water-repellency enhancer can be employed in immersion lithography with no top coat. The water-repellency enhancer is preferably a polymer containing a fluorinated alkyl group, a polymer containing a 1,1,1,3,3,3-hexafluoro-2-propanol residue with a particular structure, etc., more preferably ones exemplified in JP 2007-297590 A, JP 2008-111103 A, etc. The water-repellency enhancer needs to be dissolved in an alkali developer or an organic solvent developer. The water-repellency enhancer having a particular 1,1,1,3,3,3-hexafluoro-2-propanol residue mentioned above has favorable solubility to developers. A polymer containing a repeating unit with an amino group or amine salt as a water-repellency enhancer exhibits high effects of preventing acid evaporation during post-exposure baking (PEB) and opening failure of a hole pattern after development. When the coating material for photolithography or resist material of the present invention contains the water-repellency enhancer, the contained amount is preferably 0 to 20 parts by mass, more preferably 0.5 to 10 parts by mass based on 100 parts by mass of the base polymer. One kind of the water-repellency enhancer may be used, or two or more kinds thereof may be used in combination.

The surfactant contained when the inventive composition for forming an organic film is used for a resist material can also be used as the water-repellency enhancer. In this case, a surfactant having a high surface active effect can also be used in combination in addition to a surfactant having a high water-repellency enhancing function.

The resist material of the present invention may contain an acetylene alcohol. Specific examples of the acetylene alcohol include ones disclosed in paragraphs [0179] to [0182] of JP 2008-122932 A**.** When the resist material of the present invention contains the acetylene alcohol, the contained amount is preferably 0 to 5 parts by mass based on 100 parts by mass of the base polymer. One kind of the acetylene alcohol may be used, or two or more kinds thereof may be used in combination.

### [Patterning Process]

When the resist material of the present invention is used for manufacturing various integrated circuits, known lithography techniques can be applied. Examples of patterning processes include a method including the steps of:
forming a resist film on a substrate by using the above-described resist material;
exposing the resist film to a high-energy beam; and
developing the exposed resist film by using a developer.

Firstly, the resist material of the present invention is applied onto a substrate (such as Si, SiO₂, SiN, SiON, TiN, WSi, BPSG, SOG, or organic antireflective film) for manufacturing an integrated circuit or a substrate (such as Cr, CrO, CrON, CrN, MoSi₂, SiO₂, Ta, MoSi laminated film, Ru, Ni, Co, W, Mo, V, or an alloy thereof) for manufacturing a mask circuit by an appropriate coating process such as spin coating, roll coating, flow coating, dip coating, spray coating, or doctor coating. The resultant is prebaked on a hot plate preferably at 60 to 150°C for 10 seconds to 30 minutes, more preferably 80 to 150°C for 30 seconds to 20 minutes. Thus, a resist film is formed.

Before the resist material of the present invention is applied, it is possible to treat the substrate by irradiation with light, EB, plasma, etc. or treat the substrate with CVD, ozone, hexamethyldisilazane (HMDS), etc.

The resist film preferably has a film thickness of 1 nm to 200 µm, more preferably 2 nm to 100 µm.

A top coat can also be formed on the resist film. The top coat preferably has a function such as environmental protection, light absorption, antistatic function, resist pattern defect reduction, and resist pattern profile correction.

Subsequently, the resist film is exposed by using a high-energy beam. Specific examples of the high-energy beam include ultraviolet ray, deep ultraviolet ray, EB, EUV having a wavelength of 3 to 15 nm, X-ray, soft X-ray, excimer laser beam, γ-ray, and synchrotron radiation. When ultraviolet ray, deep ultraviolet ray, EUV, X-ray, soft X-ray, excimer laser beam, γ-ray, synchrotron radiation, or the like is employed as the high-energy beam, the irradiation is performed directly or while using a mask for forming a target pattern at an exposure dose of preferably about 1 to 200 mJ/cm², more preferably about 10 to 100 mJ/cm². When an EB is employed as the high-energy beam, the exposure dose is preferably about 0.1 to 1000 µC/cm², more preferably about 0.5 to 900 µC/cm², and the writing is performed directly or while using a mask for forming a target pattern. Note that the inventive resist material is particularly suitable for fine patterning with a g-line, an i-line, a KrF excimer laser beam, an ArF excimer laser beam, an EB, EUV, X-ray, soft X-ray, γ-ray, or synchrotron radiation, among the high-energy beams.

The exposure may be followed by PEB on a hot plate preferably at 40 to 150°C for 10 seconds to 30 minutes, more preferably 50 to 120°C for 30 seconds to 20 minutes.

After the exposure or after the PEB, development is performed using a developer for 3 seconds to 3 minutes, preferably 5 seconds to 2 minutes according to the usual method, such as a dip method, a puddle method, or a spray method. Thus, the target pattern is formed on the substrate.

The developer may be an organic solvent developer or may be an aqueous alkaline solution developer. Examples of the organic solvent developer include 2-octanone, 2-nonanone, 2-heptanone, 3-heptanone, 4-heptanone, 2-hexanone, 3-hexanone, diethyl ketone, methyl ethyl ketone, methyl isobutyl ketone, cyclopentanone, cyclohexanone, diisobutyl ketone, methylcyclohexanone, acetophenone, methylacetophenone, propyl acetate, butyl acetate, isobutyl acetate, pentyl acetate, butenyl acetate, isopentyl acetate, propyl formate, butyl formate, isobutyl formate, pentyl formate, isopentyl formate, methyl valerate, methyl pentenoate, methyl crotonate, ethyl crotonate, methyl propionate, ethyl propionate, ethyl 3-ethoxypropionate, methyl lactate, ethyl lactate, propyl lactate, butyl lactate, isobutyl lactate, pentyl lactate, isopentyl lactate, methyl 2-hydroxyisobutyrate, ethyl 2-hydroxyisobutyrate, methyl benzoate, ethyl benzoate, phenyl acetate, benzyl acetate, phenylmethyl acetate, benzyl formate, phenylethyl formate, methyl 3-phenylpropionate, benzyl propionate, phenylethyl acetate, 2-phenylethyl acetate, diethylene glycol dimethyl ether, ethylene glycol dimethyl ether, xylene, toluene, anisole, methanol, ethanol, isopropanol, n-butyl alcohol, isobutyl alcohol, acetic anhydride, and acetic acid. Specific examples of the aqueous alkaline solution developer include aqueous solutions of potassium hydroxide, sodium hydroxide, etc. and aqueous alkaline solutions of tetramethylammonium hydroxide, tetraethylammonium hydroxide, tetrapropylammonium hydroxide, tetrabutylammonium hydroxide, choline hydroxide, etc. One kind of the developer may be used, or two or more kinds thereof may be used in mixture.

When the development is completed, rinsing can be performed. The rinsing liquid is preferably a solvent that is miscible with the developer but does not dissolve the resist film. As such a solvent, it is preferable to use an alcohol having 3 to 10 carbon atoms, an ether compound having 8 to 12 carbon atoms, an alkane, alkene, alkyne, and aromatic solvent, each having 6 to 12 carbon atoms, or pure water.

Specific examples of the alcohol having 3 to 10 carbon atoms include n-propyl alcohol, isopropyl alcohol, 1-butyl alcohol, 2-butyl alcohol, isobutyl alcohol, tert-butyl alcohol, 1-pentanol, 2-pentanol, 3-pentanol, tert-pentyl alcohol, neopentyl alcohol, 2-methyl-1-butanol, 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol, cyclohexanol, 1-octanol, etc.

Specific examples of the ether compound having 8 to 12 carbon atoms include di-n-butyl ether, diisobutyl ether, di-sec-butyl ether, di-n-pentyl ether, diisopentyl ether, di-sec-pentyl ether, di-tert-pentyl ether, di-n-hexyl ether, etc.

Specific examples of the alkane having 6 to 12 carbon atoms include hexane, heptane, octane, nonane, decane, undecane, dodecane, methylcyclopentane, dimethylcyclopentane, cyclohexane, methylcyclohexane, dimethylcyclohexane, cycloheptane, cyclooctane, cyclononane, etc. Specific examples of the alkene having 6 to 12 carbon atoms include hexene, heptene, octene, cyclohexene, methylcyclohexene, dimethylcyclohexene, cycloheptene, cyclooctene, etc. Specific examples of the alkyne having 6 to 12 carbon atoms include hexyne, heptyne, octyne, etc.

Examples of the aromatic solvent include toluene, xylene, ethylbenzene, isopropylbenzene, tert-butylbenzene, mesitylene, etc.

The rinsing can reduce resist pattern collapse and defect formation. Meanwhile, the rinsing is not necessarily essential, and the amount of the solvent used can be reduced by not performing the rinsing.

After the development, a hole pattern or trench pattern can be shrunk by thermal flow, RELACS process, or DSA process. A shrink agent is applied onto the hole pattern, and the shrink agent undergoes crosslinking on the resist film surface by diffusion of the acid catalyst from the resist film during baking, so that the shrink agent is attached to sidewalls of the hole pattern. The baking temperature is preferably 70 to 180°C, more preferably 80 to 170°C. The baking time is preferably 10 to 300 seconds. The extra shrink agent is removed, and thus the hole pattern is shrunk.

### EXAMPLES

Hereinafter, the present invention will be further specifically described with reference to Synthesis Examples, Examples, and Comparative Examples. However, the present invention is not limited thereto. Note that, specifically, the molecular weight was measured in the following manner. Weight-average molecular weight (Mw) and number-average molecular weight (Mn) on polystyrene basis were measured by gel permeation chromatography (GPC) using tetrahydrofuran as an eluent (solvent), and dispersity (Mw/Mn) was calculated therefrom.

### [Synthesis of Benzenesulfonic Acid Salt (BSA-1)]

20 g of a compound (A-1) was dissolved in a mixed solvent of 50 ml of water and 50 ml of dioxane. This solution was aged at 100°C for 24 hours, and then the solvent was distilled off under reduced pressure. The product was dried under reduced pressure at 50°C to obtain 21 g of a compound (A'-1) as a colorless transparent liquid. The 21 g of the compound (A'-1) was dissolved in 40 ml of water, and 8 g of triethylamine was added dropwise thereto at room temperature. This solution was aged at room temperature for 24 hours, and then the solvent was distilled off under reduced pressure. The product was dried under reduced pressure at 50°C to obtain 25 g of a benzenesulfonic acid salt (BSA-1) as a white solid.
Molecular formula: C₁₂H₁₆NO₃SF₅
Molecular weight: 349.32

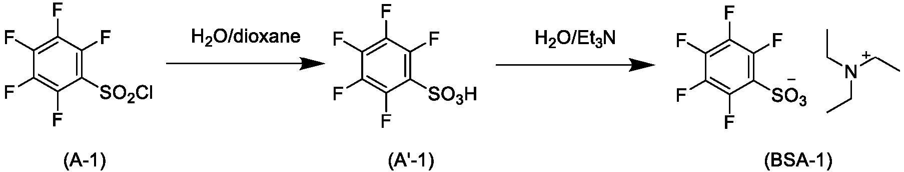

### [Synthesis of Benzenesulfonic Acid Salt (BSA-2)]

20 g of a compound (A-2) was dissolved in a mixed solvent of 50 ml of water and 50 ml of dioxane. This solution was aged at 100°C for 24 hours, and then the solvent was distilled off under reduced pressure. The product was dried under reduced pressure at 50°C to obtain 19 g of a compound (A'-2) as a white solid. The 19 g of the compound (A'-2) was dissolved in 180 ml of water, and 7 g of triethylamine was added dropwise thereto at room temperature. This solution was aged at room temperature for 24 hours, and then the solvent was distilled off under reduced pressure. The product was dried under reduced pressure at 50°C to obtain 23 g of a benzenesulfonic acid salt (BSA-2) as a white solid.
Molecular formula: C₂₁H₃₉NO₃S
Molecular weight: 385.61

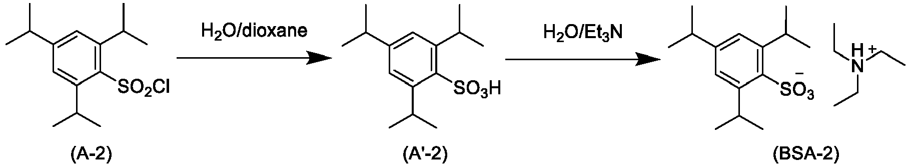

### [Synthesis of Benzenesulfonic Acid Salt (BSA-3)]

20 g of a compound (A-3) was dissolved in a mixed solvent of 140 ml of water and 1000 ml of ethanol. To this solution, 21 g of potassium hydroxide was added, the mixture was aged at 90°C for 24 hours, then hydrochloric acid was added thereto, and the solvent was distilled off under reduced pressure. 150 ml of dichloromethane and 150 ml of water were added thereto, the organic layer was collected, and then the solvent was distilled off under reduced pressure. The obtained solid was dried under reduced pressure at 50°C to obtain 20 g of a compound (A'-3) as a white solid. 19 g of the compound (A'-3) was dissolved in 150 ml of water, and 7 g of tripropylamine was added dropwise thereto at room temperature. This solution was aged at room temperature for 24 hours, and then the solvent was distilled off under reduced pressure. The product was dried under reduced pressure at 50°C to obtain 22 g of a benzenesulfonic acid salt (BSA-3) as a white solid.
Molecular formula: C₃₃H₅₇NO₃S
Molecular weight: 547.88

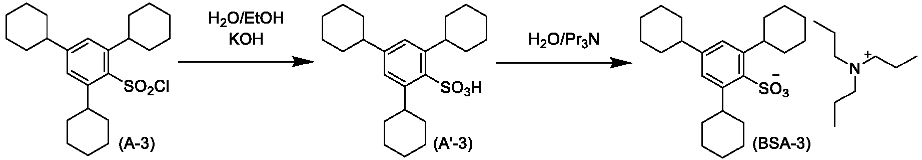

### [Synthesis of Benzenesulfonic Acid Salt (BSA-4)]

20 g of a compound (A-4) was dissolved in 200 ml of water, and 6 g of pyridine was added dropwise thereto at room temperature. This solution was aged at room temperature for 24 hours, and then the solvent was distilled off under reduced pressure. The product was dried under reduced pressure at 50°C to obtain 24 g of a benzenesulfonic acid salt (BSA-4) as a white solid.
Molecular formula: C₁₁H₉NO₃SCl₂
Molecular weight: 306.16

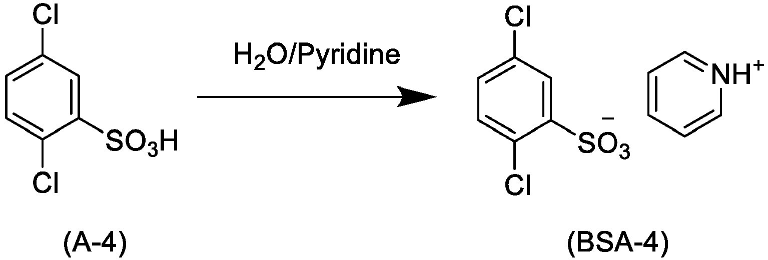

### [Synthesis of Benzenesulfonic Acid Salt (BSA-5)]

20 g of a compound (A-5) was dissolved in 200 ml of water, and 8 g of triethylamine was added dropwise thereto at room temperature. This solution was aged at room temperature for 24 hours, and then the solvent was distilled off under reduced pressure. The product was dried under reduced pressure at 50°C to obtain 25 g of a benzenesulfonic acid salt (BSA-5) as a white solid.
Molecular formula: C₁₂H₁₈NO₃SCl₃
Molecular weight: 362.69

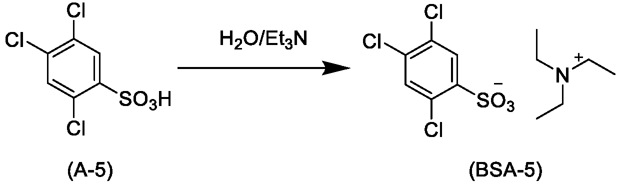

### [Synthesis of Benzenesulfonic Acid Salt (BSA-6)]

20 g of a compound (A-6) was dissolved in 200 ml of water, and 8 g of tripropylamine was added dropwise thereto at room temperature. This solution was aged at room temperature for 24 hours, and then the reaction solution was filtered to obtain a white solid. The solid was dried under reduced pressure at 50°C to obtain 17 g of a benzenesulfonic acid salt (BSA-6) as a white solid.
Molecular formula: C₂₇H₃₆NO₃SP
Molecular weight: 485.62

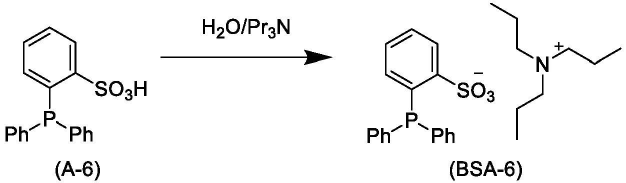

### [Synthesis of Benzenesulfonic Acid Salt (R-1)]

20 g of a compound (S-1) was dissolved in 200 ml of water, and 8 g of pyridine was added dropwise thereto at room temperature. This solution was aged at room temperature for 24 hours, and then the solvent was distilled off under reduced pressure. The product was dried under reduced pressure at 50°C to obtain 26 g of a benzenesulfonic acid salt (R-1) as a white solid.
Molecular formula: C₁₂H₁₃NO₃S
Molecular weight: 251.30

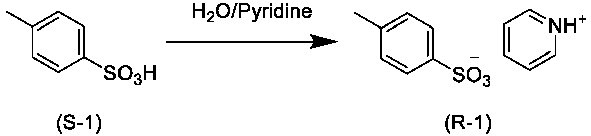

### [Synthesis of Benzenesulfonic Acid Salt (R-2)]

20 g of a compound (S-1) was dissolved in 200 ml of water, and 11 g of triethylamine was added dropwise thereto at room temperature. This solution was aged at room temperature for 24 hours, and then the solvent was distilled off under reduced pressure. The product was dried under reduced pressure at 50°C to obtain 29 g of a benzenesulfonic acid salt (R-2) as a white solid.
Molecular formula: C₁₃H₂₃NO₃S
Molecular weight: 273.39

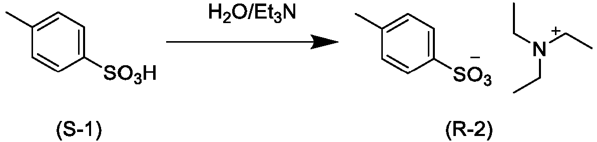

### [Resin and/or Compound for Forming Organic Film]

C1: a resin represented by the following formula (C1)
C2: a resin represented by the following formula (C2)
C3: a resin represented by the following formula (C3)
C4: a resin represented by the following formula (C4)
C5: a resin represented by the following formula (C5)
C6: a resin represented by the following formula (C6)
C7: a resin represented by the following formula (C7)
C8: a resin represented by the following formula (C8)
C9: a resin represented by the following formula (C9)
C10: a resin represented by the following formula (C10)
C11: a resin represented by the following formula (C11)

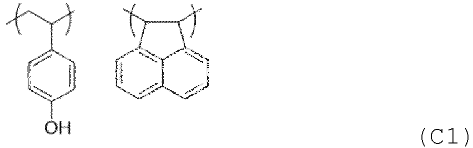

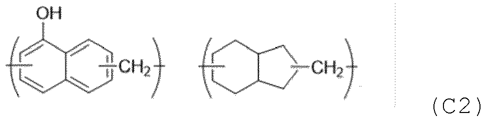

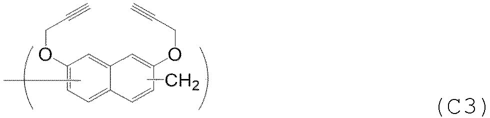

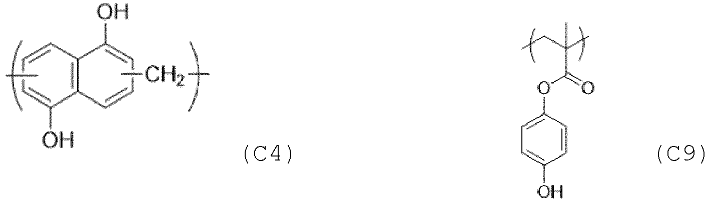

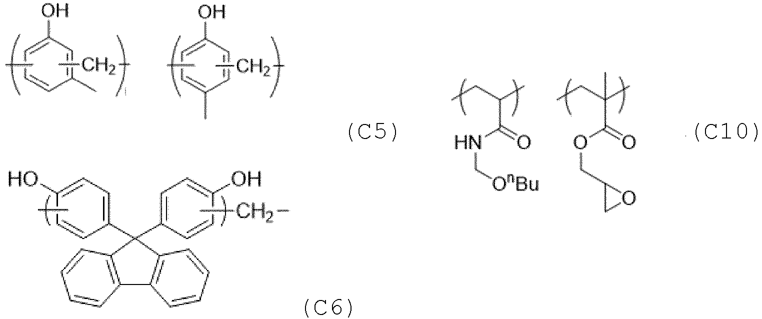

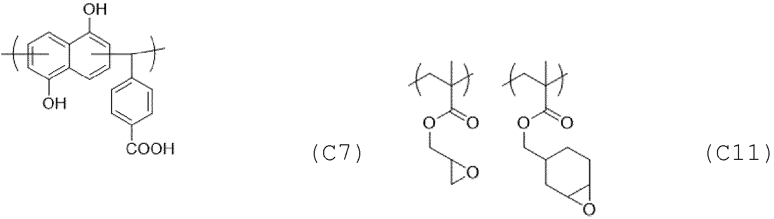

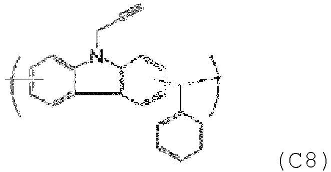

### [Solvent]

(D1): propylene glycol monomethyl ether acetate
(D2): propylene glycol monoethyl ether
(D3): cyclohexanone

### [Preparation of Compositions (Sol. 1 to 24 and Comparative Sol. 1 to 5) for Forming Organic Film]

The compounds (B) (acid generators) ((BSA-1) to (BSA-6), (R1), and (R2)), the resins (C1) to (C11) for forming an organic film, and the solvents (D1) to (D3) were dissolved in the proportions shown in Tables 1 and 2, and the solution was filtered through a 0.1-µm filter made of a fluorinated resin to prepare each of organic film materials (resist middle layer film materials: Sol. 1 to 24 and comparative Sol. 1 to 5).

**[Table 1]**

| No. | Resin (parts by mass) | Crosslinking agent (parts by mass) | Acid generator (parts by mass) | Solvent 1 (parts by mass) | Solvent 2 (parts by mass) |
|---|---|---|---|---|---|
| Sol. 1 | Compound C1 (100) | XL-1 (10) | BSA-1 (2) | D1 (1000) | None |
| Sol. 2 | Compound C1 (100) | XL-2 (10) | BSA-2 (2) | D1 (1000) | None |
| Sol. 3 | Compound C2 (100) | XL-1 (10) | BSA-3 (2) | D1 (1000) | None |
| Sol. 4 | Compound C2 (100) | XL-2 (10) | BSA-4 (2) | D1 (1000) | None |
| Sol. 5 | Compound C3 (100) | XL-1 (10) | BSA-5 (2) | D1 (700) | D2 (300) |
| Sol. 6 | Compound C3 (100) | XL-2 (10) | BSA-6 (2) | D1 (1000) | None |
| Sol. 7 | Compound C4 (100) | XL-1 (10) | BSA-1 (2) | D1 (1000) | None |
| Sol. 8 | Compound C4 (100) | XL-2 (10) | BSA-2 (2) | D1 (700) | D3 (300) |
| Sol. 9 | Compound C5 (100) | XL-1 (10) | BSA-3 (2) | D1 (1000) | None |
| Sol. 10 | Compound C5 (100) | XL-3 (10) | BSA-1 (2) | D1 (1000) | None |
| Sol. 11 | Compound C6 (100) | XL-1 (10) | BSA-2 (2) | D1 (700) | D2 (300) |
| Sol. 12 | Compound C6 (100) | XL-2 (10) | BSA-3 (2) | D1 (1000) | None |
| Sol. 13 | Compound C7 (100) | XL-1 (10) | BSA-1 (2) | D1 (1000) | None |
| Sol. 14 | Compound C7 (100) | None | BSA-2 (2) | D1 (1000) | None |
| Sol. 15 | Compound C8 (100) | XL-1 (10) | BSA-3 (2) | D1 (700) | D3 (300) |
| Sol. 16 | Compound C8 (100) | XL-2 (10) | BSA-4 (2) | D1 (1000) | None |
| Sol. 17 | Compound C9 (100) | XL-3 (10) | BSA-5 (2) | D1 (1000) | None |
| Sol. 18 | Compound C9 (100) | None | BSA-6 (2) | D1 (1000) | None |
| Sol. 19 | Compound C10 (100) | XL-2 (10) | BSA-1 (2) | D1 (700) | D2 (300) |

**[Table 2]**

| | | | | | |
|---|---|---|---|---|---|
| Sol. 20 | Compound C10 (100) | None | BSA-2 (2) | D1 (1000) | None |
| Sol. 21 | Compound C11 (100) | XL-1 (10) | BSA-3 (2) | D1 (1000) | None |
| Sol. 22 | Compound C11 (100) | None | BSA-5 (2) | D1 (1000) | None |
| Sol. 23 | Compound C1 (50) | XL-1 (10) | BSA-1 (2) | D1 (1000) | None |
| | Compound C2 (50) | | | | |
| Sol. 24 | Compound C1 (50) | XL-2 (10) | BSA-2 (2) | D1 (700) | D3 (300) |
| | Compound C2 (50) | | | | |
| Comparative Sol. 1 | Compound C3 (100) | XL-1 (10) | R-1 (2) | D1 (1000) | None |
| Comparative Sol. 2 | Compound C4 (100) | XL-2 (10) | R-2 (2) | D1 (1000) | None |
| Comparative Sol. 3 | Compound C5 (100) | XL-3 (10) | R-1 (2) | D1 (1000) | None |
| Comparative Sol. 4 | Compound C6 (100) | XL-1 (10) | R-2 (2) | D1 (1000) | None |
| Comparative Sol. 5 | Compound C7 (100) | XL-2 (10) | R-1 (2) | D1 (1000) | None |

### [Preparation of Silicon Wafers Having Cured Organic Film Formed Thereon By Using Compositions (Sol. 1 to 24 and Comparative Sol. 1 to 5) for Forming Organic Film]

Using a coater/developer "CLEAN TRACK LITHIUS Pro AP" of Tokyo Electron Ltd., each of the compositions (Sol. 1 to 24 and comparative Sol. 1 to 5) for forming an organic film prepared above was respectively discharged in an amount of 2 ml onto a 300-mm silicon wafer at the center. Then, the wafer was rotated at a rotational rate to achieve, after baking, an average film thickness of 200 nm to spread the composition. Thus, a film was formed. While rotating the silicon wafer at a rate of 1000 rpm, the remover-discharging nozzle was moved at a speed of 5 mm/s from the periphery of the silicon wafer to a position 3 mm from the center while discharging a remover (mixed solution of propylene glycol monomethyl ether acetate and propylene glycol monomethyl ether (30:70, mass ratio)) at a discharge rate of 2 ml/s, and the remover was further discharged at that position for 5 seconds at a discharge rate of 2 ml/s. After that, the discharging of the discharged liquid was terminated, and the silicon wafer was further rotated at a rate of 1000 rpm for 30 seconds. Next, the silicon wafer, having the film of the composition for forming an organic film formed thereon, was heated at 350°C for 60 seconds. Thus, silicon wafers having a cured organic film formed were obtained.

### [Solvent Resistance Evaluation: Examples 1-1 to 1-24 and Comparative Examples 1-1 to 1-5]

A film of each of the compositions (Sol. 1 to 24 and comparative Sol. 1 to 5) for forming an organic film was formed on a silicon wafer according to the above-described method to obtain Films 1 to 29. The film thickness of the peripheral portion was measured. Subsequently, a PGMEA (propylene glycol monomethyl ether acetate) solvent was dispensed thereon, left to stand for 30 seconds, spin-dried, and baked at 100°C for 60 seconds to evaporate the PGMEA, and then the film thickness of the peripheral portion was measured. The absolute value of the value determined by (X₁-X)/X × 100 was obtained as a film thickness change rate (%), where X is the film thickness before dispensing the PGMEA solvent, and X₁ is the film thickness after the PGMEA solvent was dispensed. When the film thickness change rate was lower than 0.5%, the composition was evaluated as "Good", and when 0.5% or higher, "Poor".

### [In-Plane Uniformity Evaluation: Examples 1-1 to 1-24 and Comparative Examples 1-1 to 1-5]

Regarding each of Films 1 to 29 formed on a silicon wafer according to the above-described method, the film thickness within a radius of 145 mm from the center of the cured organic film was measured. The value determined by (Xₘₐₓ-Xₘᵢₙ)/X_{average} was obtained as in-plane uniformity (%), where Xₘₐₓ is the maximum value of the film thickness, Xₘᵢₙ is the minimum value of the film thickness, and X_{average} is the average film thickness. When the in-plane uniformity was less than 3%, the composition was evaluated as "Good", and when 3% or more, "Poor".

### [Silicon-Containing Resist Middle Layer Film Coating Property Evaluation: Examples 1-1 to 1-24 and Comparative Examples 1-1 to 1-5]

A film of each of the compositions (Sol. 1 to 24 and comparative Sol. 1 to 5) for forming an organic film was formed respectively on a silicon wafer according to the above-described method to obtain Films 1 to 29. The silicon-containing resist middle layer film material (SOG1) described below was applied thereto, and baking was performed at 200°C for 60 seconds to form a silicon-containing resist middle layer film. Then, the condition of the coating film of the silicon-containing resist middle layer film was visually observed and evaluated.

When the condition of the coating film was good, the composition was evaluated as "Good", and when dewetting had occurred, as "Poor".

Note that, in this evaluation, to evaluate whether the coatability of the silicon-containing resist middle layer film is excellent or not, the thickness of the silicon-containing resist middle layer film was set to 5 nm, and this is a special, severe evaluation condition.

The silicon-containing resist middle layer film material (SOG1) was prepared by dissolving a polymer (SP1), a crosslinking catalyst, and an acid in an organic solvent and water in the proportions of Table 3, and filtering the mixture through a 0.1-µm filter made of a fluororesin.

**[Table 3]**

| | Polymer (parts by mass) | Crosslinking catalyst (parts by mass) | Acid (parts by mass) | Organic solvent (parts by mass) | Water (parts by mass) |
|---|---|---|---|---|---|
| SOG1 | SP1 (100) | TMPANO₃ (1) | Maleic acid (1) | PGEE (44100) | Water (4900) |

The polymer (SP1) is shown below. 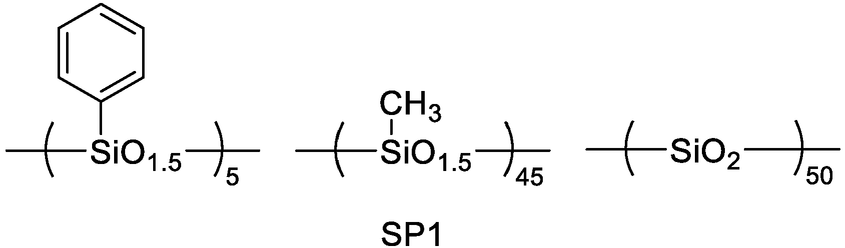
TMPANO₃: trimethylphenylammonium nitrate
PGEE: propylene glycol ethyl ether

**[Table 4]**

| | Composition for forming organic film | Organic film | Solvent resistance | In-plane uniformity | Silicon-containing resist middle layer film coating property evaluation |
|---|---|---|---|---|---|
| Example 1-1 | Sol. 1 | Film 1 | Good | Good | Good |
| Example 1-2 | Sol. 2 | Film 2 | Good | Good | Good |
| Example 1-3 | Sol. 3 | Film 3 | Good | Good | Good |
| Example 1-4 | Sol. 4 | Film 4 | Good | Good | Good |
| Example 1-5 | Sol. 5 | Film 5 | Good | Good | Good |
| Example 1-6 | Sol. 6 | Film 6 | Good | Good | Good |
| Example 1-7 | Sol. 7 | Film 7 | Good | Good | Good |
| Example 1-8 | Sol. 8 | Film 8 | Good | Good | Good |
| Example 1-9 | Sol. 9 | Film 9 | Good | Good | Good |
| Example 1-10 | Sol. 10 | Film 10 | Good | Good | Good |
| Example 1-11 | Sol. 11 | Film 11 | Good | Good | Good |
| Example 1-12 | Sol. 12 | Film 12 | Good | Good | Good |
| Example 1-13 | Sol. 13 | Film 13 | Good | Good | Good |
| Example 1-14 | Sol. 14 | Film 14 | Good | Good | Good |
| Example 1-15 | Sol. 15 | Film 15 | Good | Good | Good |
| Example 1-16 | Sol. 16 | Film 16 | Good | Good | Good |
| Example 1-17 | Sol. 17 | Film 17 | Good | Good | Good |
| Example 1-18 | Sol. 18 | Film 18 | Good | Good | Good |
| Example 1-19 | Sol. 19 | Film 19 | Good | Good | Good |
| Example 1-20 | Sol. 20 | Film 20 | Good | Good | Good |
| Example 1-21 | Sol. 21 | Film 21 | Good | Good | Good |
| Example 1-22 | Sol. 22 | Film 22 | Good | Good | Good |
| Example 1-23 | Sol. 23 | Film 23 | Good | Good | Good |
| Example 1-24 | Sol. 24 | Film 24 | Good | Good | Good |
| Comparative Example 1-1 | Comparative Sol. 1 | Film 25 | Poor | Poor | Poor |
| Comparative Example 1-2 | Comparative Sol. 2 | Film 26 | Poor | Poor | Poor |
| Comparative Example 1-3 | Comparative Sol. 3 | Film 27 | Poor | Poor | Poor |
| Comparative Example 1-4 | Comparative Sol. 4 | Film 28 | Poor | Poor | Poor |
| Comparative Example 1-5 | Comparative Sol. 5 | Film 29 | Poor | Poor | Poor |

As shown in Table 4, it was confirmed that the inventive compositions (Sol. 1 to 24) for forming an organic film, which contained the thermal acid generators (BSA-1 to BSA-6) having a high molecular weight, were excellent in solvent resistance, in-plane uniformity, and the coating property of the silicon-containing resist middle layer film. At the same time, good in-plane uniformity was achieved.

The acid generators used in the Comparative Examples had a low molecular weight, and therefore, easily sublimed. Particularly in the peripheral portion of the film, poor solvent resistance, lack of film thickness uniformity, and coating failure of the silicon-containing resist middle layer film were observed.

### [Patterning Test: Examples 2-1 to 2-24]

A cured organic film was formed respectively on an SiO₂ wafer substrate by using each of the compositions (Sol. 1 to 24) for forming an organic film according to the above-described method. The silicon-containing resist middle layer film material (SOG2) described below was applied thereto, and baking was performed at 200°C for 60 seconds to form a 35-nm thick silicon-containing resist middle layer film. The monolayer resist for ArF described below was applied thereto as a resist upper layer film material and baked at 105°C for 60 seconds to form a 100-nm thick photoresist film. Subsequently, the liquid immersion top coat composition (TC-1) described below was applied to the photoresist film and baked at 90°C for 60 seconds to form a 50-nm thick top coat.

The silicon-containing resist middle layer film material (SOG2) was prepared by dissolving a polymer (SP1), a crosslinking catalyst, and an acid in an organic solvent and water in the proportions of Table 5, and filtering the mixture through a 0.1-µm filter made of a fluororesin.

**[Table 5]**

| | Polymer (parts by mass) | Crosslinking catalyst (parts by mass) | Acid (parts by mass) | Organic solvent (parts by mass) | Water (parts by mass) |
|---|---|---|---|---|---|
| SOG2 | SP1 (100) | TMPANO₃ (1) | Maleic acid (1) | PGEE (4410) | Water (490) |

The resist upper layer film material (monolayer resist for ArF) was prepared by dissolving a polymer (RP1), an acid generator (PAG1), and a basic compound (Amine1), each in the proportion shown in Table 6, in a PGMEA (propylene glycol monomethyl ether acetate) solvent containing 0.1% by mass of FC-430 (manufactured by Sumitomo 3M Limited), and filtering the mixture through a 0.1-µm filter made of a fluororesin.

**[Table 6]**

| | Polymer (parts by mass) | Acid generator (parts by mass) | Basic compound (parts by mass) | Solvent (parts by mass) |
|---|---|---|---|---|
| Monolayer resist for ArF | RP1 (100) | PAG1 (6.6) | Amine1 (0.8) | PGMEA (2500) |

The polymer (RP1), the acid generator (PAG1), and the basic compound (Amine1) are shown below.

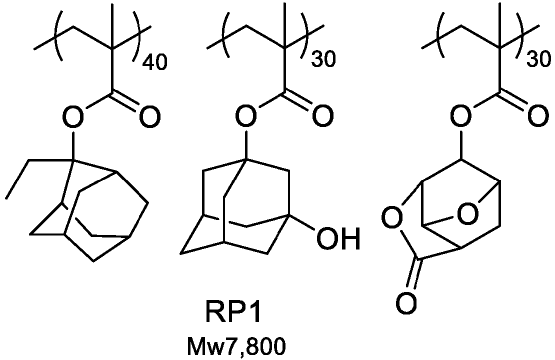

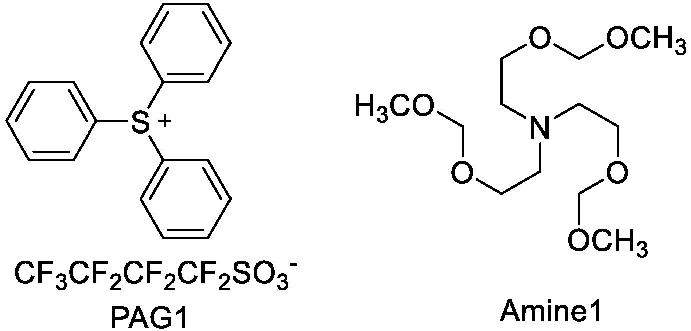

The liquid immersion top coat composition (TC-1) was prepared by dissolving a polymer (PP1) in an organic solvent at the proportion shown in Table 7, and filtering the mixture through a 0.1-µm filter made of a fluororesin.

**[Table 7]**

| | Polymer (parts by mass) | Organic solvent (parts by mass) |
|---|---|---|
| TC-1 | PP1 (100) | Diisoamyl ether (2700) |
| | | 2-methyl-1-butanol (270) |

The polymer (PP1) is shown below.

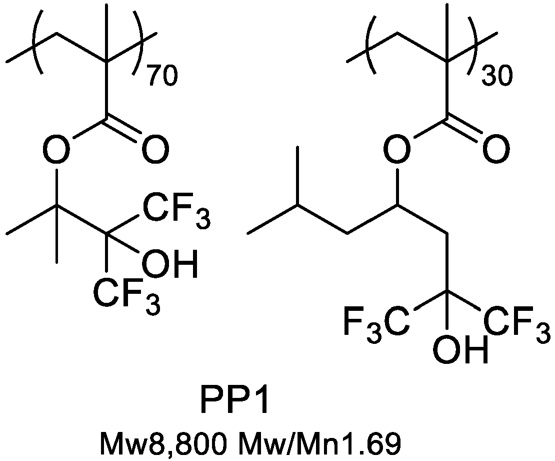

Then, the substrate was exposed to light with an ArF liquid immersion exposure apparatus (NSR-S610C manufactured by Nikon Corporation, NA: 1.30, σ: 0.98/0.65, 35° s-polarized dipole illumination, 6% halftone phase shift mask), baked at 100°C for 60 seconds (PEB), and developed with a 2.38% by mass aqueous solution of tetramethylammonium hydroxide (TMAH) for 30 seconds, thereby obtaining a 55 nm 1:1 positive line-and-space pattern (a resist upper layer film pattern).

Subsequently, using an etching apparatus Telius manufactured by Tokyo Electron Ltd., the silicon-containing resist middle layer film was dry-etched (the pattern was transferred) while using the resist upper layer film pattern as a mask; the organic film was dry-etched (the pattern was transferred) while using the obtained silicon-containing resist middle layer film pattern as a mask; and the SiO₂ wafer substrate (SiO₂ film) was dry-etched (the pattern was transferred) while using the obtained organic film pattern as a mask. The etching conditions were as follows.

### Conditions in transferring resist upper layer film pattern to silicon-containing resist middle layer film

Chamber pressure: 10.0 Pa
RF-power: 1,500 W
CF₄ gas flow rate: 75 ml/min
O₂ gas flow rate: 15 ml/min
Time: 15 sec

### Conditions in transferring silicon-containing resist middle layer film pattern to organic film

Chamber pressure: 2.0 Pa
RF-power: 500 W
Ar gas flow rate: 75 ml/min
O₂ gas flow rate: 45 ml/min
Time: 120 sec

### Conditions in transferring organic film pattern to SiO₂ wafer substrate

Chamber pressure: 2.0 Pa
RF-power: 2,200 W
C₅F₁₂ gas flow rate: 20 ml/min
C₂F₆ gas flow rate: 10 ml/min
Ar gas flow rate: 300 ml/min
O₂ gas flow rate: 60 ml/min
Time: 90 sec

The cross section of the obtained pattern was observed with an electron microscope (S-4700) manufactured by Hitachi, Ltd. Table 8 shows the results.

**[Table 8]**

| | Composition for forming organic film | Profile after etching for transferring to substrate |
|---|---|---|
| Example 2-1 | Sol. 1 | Good |
| Example 2-2 | Sol. 2 | Good |
| Example 2-3 | Sol. 3 | Good |
| Example 2-4 | Sol. 4 | Good |
| Example 2-5 | Sol. 5 | Good |
| Example 2-6 | Sol. 6 | Good |
| Example 2-7 | Sol. 7 | Good |
| Example 2-8 | Sol. 8 | Good |
| Example 2-9 | Sol. 9 | Good |
| Example 2-10 | Sol. 10 | Good |
| Example 2-11 | Sol. 11 | Good |
| Example 2-12 | Sol. 12 | Good |
| Example 2-13 | Sol. 13 | Good |
| Example 2-14 | Sol. 14 | Good |
| Example 2-15 | Sol. 15 | Good |
| Example 2-16 | Sol. 16 | Good |
| Example 2-17 | Sol. 17 | Good |
| Example 2-18 | Sol. 18 | Good |
| Example 2-19 | Sol. 19 | Good |
| Example 2-20 | Sol. 20 | Good |
| Example 2-21 | Sol. 21 | Good |
| Example 2-22 | Sol. 22 | Good |
| Example 2-23 | Sol. 23 | Good |
| Example 2-24 | Sol. 24 | Good |

As shown in Table 8, in Examples 2-1 to 2-24, where the inventive compositions (Sol. 1 to 24) for forming an organic film were used, the resist upper layer film pattern was successfully transferred to the SiO₂ wafer substrate in the end in every case. Thus, it was confirmed that the inventive compositions for forming an organic film can be used suitably for fine processing using a multilayer resist method.

From the above, the inventive composition for forming an organic film has excellent film-formability and excellent in-plane uniformity, and is excellent in the coating property of silicon-containing resist middle layer films, and therefore, is extremely useful as an organic film material used in a multilayer resist process. Furthermore, the inventive composition makes it possible to form a fine pattern with high precision and form a hump-suppressed organic film, and therefore, makes it possible to manufacture semiconductor devices and the like efficiently.

The present description includes the following inventions.
[1]: A composition for forming an organic film, comprising: a resin and/or compound (A) for forming an organic film; a benzenesulfonic acid salt compound (B) represented by the following formula (1), an anion moiety in the formula (1) having a molecular weight of 200 or more, and the compound (B) not containing a perfluoroalkyl group; and a solvent (C), 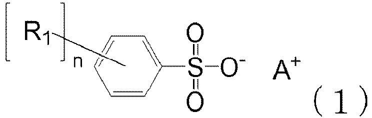 wherein R₁ represents a linear, cyclic, or branched, alkyl group, alkenyl group, oxoalkyl group, aryl group, or aralkyl group having 1 to 20 carbon atoms and optionally having a substituent not containing a perfluoroalkyl group, represents -P(R)₂, R being an alkyl group or aryl group having 1 to 20 carbon atoms, or represents a halogen atom or a nitro group, "n" represents an integer of 1 to 5, and A⁺ represents an ammonium cation, a pyridinium cation, a sulfonium cation, a phosphonium cation, an imidazolium cation, a piperidinium cation, or a pyrrolidinium cation.
[2]: The composition for forming an organic film according to [1], wherein the benzenesulfonic acid salt compound (B) is represented by the following general formula (2-1), (2-2), or (2-3), 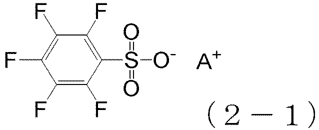
   wherein A⁺ is identical to the A⁺ in the formula (1), 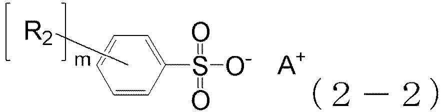
   wherein R₂ represents a chlorine atom or an iodine atom; "m" represents an integer of 1 to 5; and A⁺ is identical to the A⁺ in the formula (1), 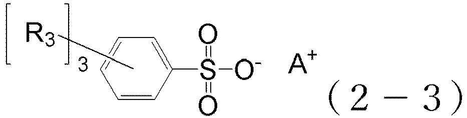
   wherein R₃ represents an alkyl group having 1 to 20 carbon atoms and optionally having a substituent not containing a perfluoroalkyl group; and A⁺ is identical to the A⁺ in the formula (1).
[3]: The composition for forming an organic film according to [2], wherein the anion moiety in the general formula (2-1), (2-2), or (2-3) of the benzenesulfonic acid salt compound (B) has a molecular weight of 245 or more.
[4]: The composition for forming an organic film according to [2] or [3], wherein the benzenesulfonic acid salt compound (B) is represented by the general formula (2-3), the R₃ in the formula does not contain a perfluoroalkyl group and represents a branched or cyclic alkyl group having 3 to 20 carbon atoms, and A⁺ represents a triethylammonium cation or a tributylammonium cation.
[5]: The composition for forming an organic film according to any one of [1] to [4], wherein the resin or compound (A) for forming an organic film has any of a methylol group, an epoxy group, or a phenolic hydroxy group.
[6]: A method for forming an organic film to be used in a manufacturing process of a semiconductor device, the method comprising:
   spin-coating a substrate to be processed with the composition for forming an organic film according to any one of [1] to [5]; and
   forming a cured film by heating the substrate coated with the composition for forming an organic film at a temperature of 100°C or higher and 600°C or lower for 10 to 600 seconds.
[7]: A patterning process comprising:
   forming an organic film on a body to be processed by using the composition for forming an organic film according to any one of [1] to [5];
   forming a resist middle layer film on the organic film by using a resist middle layer film material containing a silicon atom;
   forming a resist upper layer film on the resist middle layer film by using a resist upper layer film material comprising a photoresist composition;
   forming a circuit pattern in the resist upper layer film;
   transferring the pattern to the resist middle layer film by etching while using the resist upper layer film having the formed circuit pattern as a mask;
   transferring the pattern to the organic film by etching while using the resist middle layer film having the transferred pattern as a mask; and
   further transferring the pattern to the body to be processed by etching while using the organic film having the transferred pattern as a mask.
[8]: A patterning process comprising:
   forming an organic film on a body to be processed by using the composition for forming an organic film according to any one of [1] to [5];
   forming a resist middle layer film on the organic film by using a resist middle layer film material containing a silicon atom;
   forming an organic antireflective film or an adhesive film on the resist middle layer film;
   forming a resist upper layer film on the organic antireflective film or the adhesive film by using a resist upper layer film material comprising a photoresist composition;
   forming a circuit pattern in the resist upper layer film;
   transferring the pattern to the organic antireflective film or the adhesive film and to the resist middle layer film by etching while using the resist upper layer film having the formed circuit pattern as a mask;
   transferring the pattern to the organic film by etching while using the resist middle layer film having the transferred pattern as a mask; and
   further transferring the pattern to the body to be processed by etching while using the organic film having the transferred pattern as a mask.
[9]: A patterning process comprising:
   forming an organic film on a body to be processed by using the composition for forming an organic film according to any one of [1] to [5];
   forming an inorganic hard mask selected from the group consisting of a silicon oxide film, a silicon nitride film, and a silicon oxynitride film on the organic film;
   forming a resist upper layer film on the inorganic hard mask by using a resist upper layer film material comprising a photoresist composition;
   forming a circuit pattern in the resist upper layer film;
   transferring the pattern to the inorganic hard mask by etching while using the resist upper layer film having the formed circuit pattern as a mask;
   transferring the pattern to the organic film by etching while using the inorganic hard mask having the transferred pattern as a mask; and
   further transferring the pattern to the body to be processed by etching while using the organic film having the transferred pattern as a mask.
[10]: A patterning process comprising:
   forming an organic film on a body to be processed by using the composition for forming an organic film according to any one of [1] to [5];
   forming an inorganic hard mask selected from the group consisting of a silicon oxide film, a silicon nitride film, and a silicon oxynitride film on the organic film;
   forming an organic antireflective film or an adhesive film on the inorganic hard mask;
   forming a resist upper layer film on the organic antireflective film or the adhesive film by using a resist upper layer film material comprising a photoresist composition;
   forming a circuit pattern in the resist upper layer film;
   transferring the pattern to the organic antireflective film or the adhesive film and to the inorganic hard mask by etching while using the resist upper layer film having the formed circuit pattern as a mask;
   transferring the pattern to the organic film by etching while using the inorganic hard mask having the transferred pattern as a mask; and
   further transferring the pattern to the body to be processed by etching while using the organic film having the transferred pattern as a mask.
[11]: The patterning process according to [9] or [10], wherein the inorganic hard mask is formed by a CVD method or an ALD method.
[12]: The patterning process according to any one of [7] to [11], wherein the circuit pattern is formed by a lithography using light having a wavelength of 10 nm or more and 300 nm or less, a direct writing with electron beam, nanoimprinting, or a combination thereof.
[13]: The patterning process according to any one of [7] to [12], wherein the circuit pattern is developed with an alkaline development or an organic solvent.
[14]: The patterning process according to any one of [7] to [13], wherein the body to be processed is a semiconductor device substrate or the semiconductor device substrate coated with any of a metal film, a metal carbide film, a metal oxide film, a metal nitride film, a metal oxycarbide film, and a metal oxynitride film.
[15]: The patterning process according to [14], wherein the metal constituting the body to be processed is silicon, titanium, tungsten, hafnium, zirconium, chromium, germanium, copper, silver, gold, aluminum, indium, gallium, arsenic, palladium, iron, tantalum, iridium, molybdenum, or an alloy thereof.
[16]: A benzenesulfonic acid salt compound for forming an organic film, being represented by the following formula (1), an anion moiety in the formula (1) having a molecular weight of 200 or more, and the compound not containing a perfluoroalkyl group, 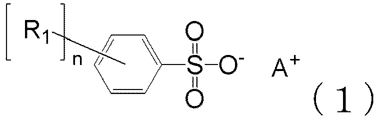 wherein R₁ represents a linear, cyclic, or branched, alkyl group, alkenyl group, oxoalkyl group, aryl group, or aralkyl group having 1 to 20 carbon atoms and optionally having a substituent not containing a perfluoroalkyl group, represents -P(R)₂, R being an alkyl group or aryl group having 1 to 20 carbon atoms, or represents a halogen atom or a nitro group, "n" represents an integer of 1 to 5, and A⁺ represents an ammonium cation, a pyridinium cation, a sulfonium cation, a phosphonium cation, an imidazolium cation, a piperidinium cation, or a pyrrolidinium cation.
[17]: The benzenesulfonic acid salt compound according to [16], being represented by the following general formula (2-1), (2-2), or (2-3), 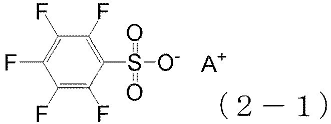
   wherein A⁺ is identical to the A⁺ in the formula (1), 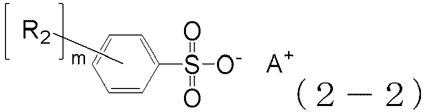
   wherein R₂ represents a chlorine atom or an iodine atom; "m" represents an integer of 1 to 5; and A⁺ is identical to the A⁺ in the formula (1), 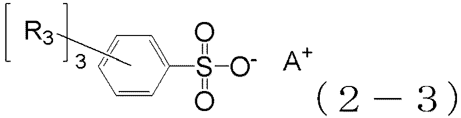
   wherein R₃ represents an alkyl group having 1 to 20 carbon atoms and optionally having a substituent not containing a perfluoroalkyl group; and A⁺ is identical to the A⁺ in the formula (1).
[18]: The benzenesulfonic acid salt compound according to [17], wherein the anion moiety in the general formula (2-1), (2-2), or (2-3) has a molecular weight of 245 or more.
[19]: The benzenesulfonic acid salt compound according to [18], being represented by the general formula (2-3), the R₃ in the formula not containing a perfluoroalkyl group, the compound being a branched or cyclic alkyl group having 3 to 20 carbon atoms, and A⁺ representing a triethylammonium cation or a tributylammonium cation.
[20]: A patterning process comprising:
   forming a resist film on a body to be processed by using a resist material comprising the composition for forming an organic film according to any one of [1] to [5];
   exposing the resist film to a high-energy beam; and
   developing the exposed resist film by using a developer.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A composition for forming an organic film, comprising: a resin and/or compound (A) for forming an organic film; a benzenesulfonic acid salt compound (B) represented by the following formula (1), an anion moiety in the formula (1) having a molecular weight of 200 or more, and the compound (B) not containing a perfluoroalkyl group; and a solvent (C), 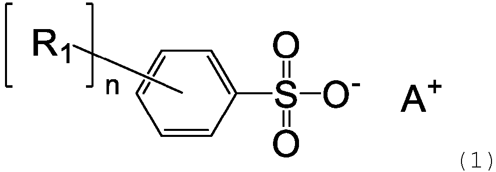 wherein R₁ represents a linear, cyclic, or branched, alkyl group, alkenyl group, oxoalkyl group, aryl group, or aralkyl group having 1 to 20 carbon atoms and optionally having a substituent not containing a perfluoroalkyl group, represents -P(R)₂, R being an alkyl group or aryl group having 1 to 20 carbon atoms, or represents a halogen atom or a nitro group, "n" represents an integer of 1 to 5, and A⁺ represents an ammonium cation, a pyridinium cation, a sulfonium cation, a phosphonium cation, an imidazolium cation, a piperidinium cation, or a pyrrolidinium cation.

2. The composition for forming an organic film according to claim 1, wherein the benzenesulfonic acid salt compound (B) is represented by the following general formula (2-1), (2-2), or (2-3), 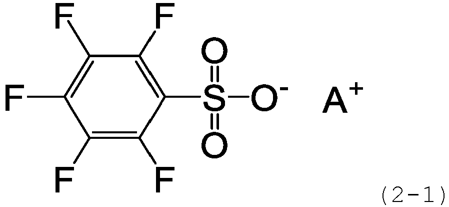
wherein A⁺ is identical to the A⁺ in the formula (1), 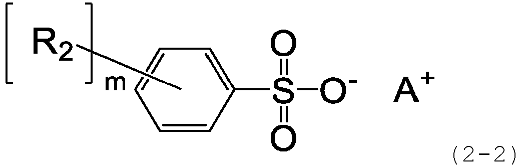
wherein R₂ represents a chlorine atom or an iodine atom; "m" represents an integer of 1 to 5; and A⁺ is identical to the A⁺ in the formula (1), 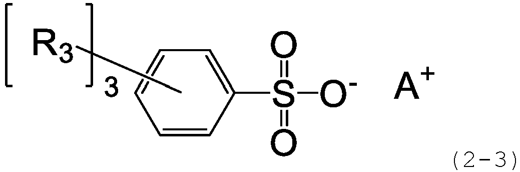
wherein R₃ represents an alkyl group having 1 to 20 carbon atoms and optionally having a substituent not containing a perfluoroalkyl group; and A⁺ is identical to the A⁺ in the formula (1).

3. The composition for forming an organic film according to claim 2, wherein the anion moiety in the general formula (2-1), (2-2), or (2-3) of the benzenesulfonic acid salt compound (B) has a molecular weight of 245 or more.

4. The composition for forming an organic film according to claim 2, wherein the benzenesulfonic acid salt compound (B) is represented by the general formula (2-3), the R₃ in the formula does not contain a perfluoroalkyl group and represents a branched or cyclic alkyl group having 3 to 20 carbon atoms, and A⁺ represents a triethylammonium cation or a tributylammonium cation.

5. The composition for forming an organic film according to claims 1 to 4, wherein the resin or compound (A) for forming an organic film has any of a methylol group, an epoxy group, or a phenolic hydroxy group.

6. A method for forming an organic film to be used in a manufacturing process of a semiconductor device, the method comprising:
spin-coating a substrate to be processed with the composition for forming an organic film according to any one of claims 1 to 5; and
forming a cured film by heating the substrate coated with the composition for forming an organic film at a temperature of 100°C or higher and 600°C or lower for 10 to 600 seconds.

7. A patterning process comprising:
forming an organic film on a body to be processed by using the composition for forming an organic film according to any one of claims 1 to 5;
forming a resist middle layer film on the organic film by using a resist middle layer film material containing a silicon atom;
forming a resist upper layer film on the resist middle layer film by using a resist upper layer film material comprising a photoresist composition;
forming a circuit pattern in the resist upper layer film;
transferring the pattern to the resist middle layer film by etching while using the resist upper layer film having the formed circuit pattern as a mask;
transferring the pattern to the organic film by etching while using the resist middle layer film having the transferred pattern as a mask; and
further transferring the pattern to the body to be processed by etching while using the organic film having the transferred pattern as a mask, or
a patterning process comprising:
forming an organic film on a body to be processed by using the composition for forming an organic film according to any one of claim 1;
forming a resist middle layer film on the organic film by using a resist middle layer film material containing a silicon atom;
forming an organic antireflective film or an adhesive film on the resist middle layer film;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the resist middle layer film by etching while using the resist upper layer film having the formed circuit pattern as a mask;
transferring the pattern to the organic film by etching while using the resist middle layer film having the transferred pattern as a mask; and
further transferring the pattern to the body to be processed by etching while using the organic film having the transferred pattern as a mask, or
a patterning process comprising:
forming an organic film on a body to be processed by using the composition for forming an organic film according to any one of claim 1;
forming an inorganic hard mask selected from the group consisting of a silicon oxide film, a silicon nitride film, and a silicon oxynitride film on the organic film;
forming a resist upper layer film on the inorganic hard mask by using a resist upper layer film material comprising a photoresist composition;
forming a circuit pattern in the resist upper layer film;
transferring the pattern to the inorganic hard mask by etching while using the resist upper layer film having the formed circuit pattern as a mask;
transferring the pattern to the organic film by etching while using the inorganic hard mask having the transferred pattern as a mask; and
further transferring the pattern to the body to be processed by etching while using the organic film having the transferred pattern as a mask, or
a patterning process comprising:
forming an organic film on a body to be processed by using the composition for forming an organic film according to any one of claim 1;
forming an inorganic hard mask selected from the group consisting of a silicon oxide film, a silicon nitride film, and a silicon oxynitride film on the organic film;
forming an organic antireflective film or an adhesive film on the inorganic hard mask;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the inorganic hard mask by etching while using the resist upper layer film having the formed circuit pattern as a mask;
transferring the pattern to the organic film by etching while using the inorganic hard mask having the transferred pattern as a mask; and
further transferring the pattern to the body to be processed by etching while using the organic film having the transferred pattern as a mask.

8. The patterning process according to claim 7, wherein the inorganic hard mask is formed by a CVD method or an ALD method.

9. The patterning process according to claim 7, wherein the circuit pattern is formed by a lithography using light having a wavelength of 10 nm or more and 300 nm or less, a direct writing with electron beam, nanoimprinting, or a combination thereof.

10. The patterning process according to claim 7, wherein the circuit pattern is developed with an alkaline development or an organic solvent.

11. The patterning process according to claim 7, wherein the body to be processed is a semiconductor device substrate or the semiconductor device substrate coated with any of a metal film, a metal carbide film, a metal oxide film, a metal nitride film, a metal oxycarbide film, and a metal oxynitride film.

12. The patterning process according to claim 11, wherein the metal constituting the body to be processed is silicon, titanium, tungsten, hafnium, zirconium, chromium, germanium, copper, silver, gold, aluminum, indium, gallium, arsenic, palladium, iron, tantalum, iridium, molybdenum, or an alloy thereof.

13. A benzenesulfonic acid salt compound for forming an organic film, being represented by the following formula (1), an anion moiety in the formula (1) having a molecular weight of 200 or more, and the compound not containing a perfluoroalkyl group, 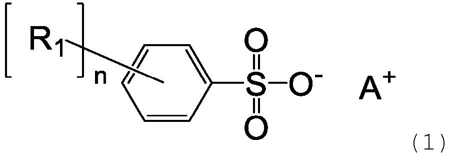 wherein R₁ represents a linear, cyclic, or branched, alkyl group, alkenyl group, oxoalkyl group, aryl group, or aralkyl group having 1 to 20 carbon atoms and optionally having a substituent not containing a perfluoroalkyl group, represents -P(R)₂, R being an alkyl group or aryl group having 1 to 20 carbon atoms, or represents a halogen atom or a nitro group, "n" represents an integer of 1 to 5, and A⁺ represents an ammonium cation, a pyridinium cation, a sulfonium cation, a phosphonium cation, an imidazolium cation, a piperidinium cation, or a pyrrolidinium cation.

14. The benzenesulfonic acid salt compound according to claim 13, being represented by the following general formula (2-1), (2-2), or (2-3), 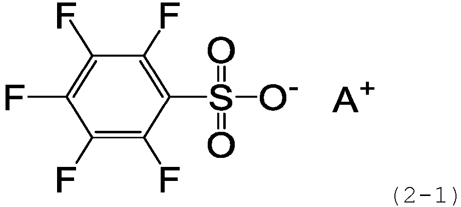
wherein A⁺ is identical to the A⁺ in the formula (1), 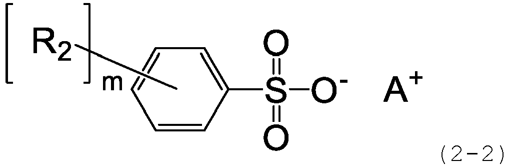
wherein R₂ represents a chlorine atom or an iodine atom; "m" represents an integer of 1 to 5; and A⁺ is identical to the A⁺ in the formula (1), 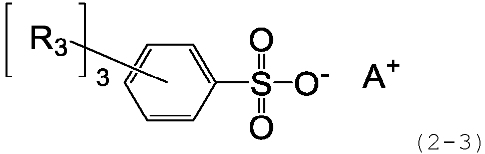
wherein R₃ represents an alkyl group having 1 to 20 carbon atoms and optionally having a substituent not containing a perfluoroalkyl group; and A⁺ is identical to the A⁺ in the formula (1), preferably,
wherein the benzenesulfonic acid salt compound, wherein the anion moiety in the general formula (2-1), (2-2), or (2-3) has a molecular weight of 245 or more, further preferably,
wherein the benzenesulfonic acid salt compound, being represented by the general formula (2-3), the R₃ in the formula not containing a perfluoroalkyl group, the compound being a branched or cyclic alkyl group having 3 to 20 carbon atoms, and A⁺ representing a triethylammonium cation or a tributylammonium cation.

15. A patterning process comprising:
forming a resist film on a body to be processed by using a resist material comprising the composition for forming an organic film according to any one of claims 1 to 5;
exposing the resist film to a high-energy beam; and
developing the exposed resist film by using a developer.
